# EUROPEAN PATENT APPLICATION

(11) **EP 4 706 681 A2**
(43) Date of publication of application: **11.03.2026**
(21) Application number: 25204778.2
(22) Date of filing: 07.03.2022
(51) Int. Cl.: A61K 45/06

(54) **CHAPERONIN-CONTAINING TCP-1 INHIBITORS FOR THE TREATMENT OF CANCER**

(30) Priority: 05.03.2021 US 202163157051 P
(62) Divisional of application: 22764221.2
(71) Applicant: University of Central Florida Research Foundation, Inc., Orlando, Florida 32816 (US)
(72) Inventor: KHALED, Annette, Orlando, 32816 (US)
(74) Representative: Dehns

(57) **Abstract**

Disclosed herein are compositions and uses thereof for treating cancers.

## Description

### CROSS REFERENCE TO REALTED APPLICATIONS

1. This application claims the benefit of priority to U.S. Provisional Application No. 63/157,051, filed March 5, 2021, which is incorporated by reference herein in its entirety.

### BACKGROUND

2. Breast cancer is the most common cancer among women and a leading cause of death. Worldwide estimates of age-standardized incidence rate and mortality rate for breast cancer is 46.3 and 13.0 per 100,000, respectively. Breast cancer is typically classified based on the expression of estrogen receptor (ER), progesterone receptor (PR), and human epidermal growth factor receptor 2 (HER2). The main molecular subtypes of breast cancer are: luminal A, which is hormone-receptor positive (ER+PR+HER2-, Ki67 low), low grade, grows slowly and has the best prognosis; luminal B, which is also hormone-receptor positive (ER+PR+ HER2+/-, Ki67 high), grows faster than luminal A and has a worse prognosis; HER2 positive or enriched, which is hormone receptor negative but HER2 positive (ER-PR-HER2+), grows faster than luminal cancers, and has a worse prognosis; and triple negative (TNBC) or basal, which is hormone receptor negative (ER-PR-HER2-), is more invasive, is common in women with BRCA1 mutations and cannot be treated with endocrine therapies or HER2 inhibitors as with the other subtypes. These subtypes of breast cancer help stratify patients and impact prognostic predictions and therapeutic decision making. However, breast cancer is a heterogenous disease with complexities that go beyond these subtypes. Loss of tumor suppressors and amplification of oncogenes are common in breast tumors. Gene amplification is the most frequent genetic alteration in breast cancer with MYC, CCND1, epidermal growth factor receptor (EGFR), fibroblast growth factor receptor (FGFR), CDK4, and MDM2 being the genes most frequently amplified in primary and recurrent breast tumors. Genetic alterations in these genes correlate with patient prognosis and clinicopathological features and their therapeutic targeting remains the goal of precision medicine. What is needed are compositions and methods to diagnosing and treating cancers.

### SUMMARY

3. Disclosed herein are methods for treating, preventing, reducing, and/or inhibiting a cancer and/or metastasis in a subject. Also disclosed herein are methods for diagnosing cancers.

4. The details of one or more embodiments of the invention are set forth in the accompanying drawings and the description below. Other features, objects, and advantages of the invention will be apparent from the description and drawings, and from the claims.

5. In some aspects, disclosed herein are methods of treating, inhibiting, decreasing, reducing, ameliorating, and/or preventing a cancer and/or metastasis in a subject in need thereof, comprising administering to the subject a therapeutically effective amount of a chaperonin-containing TCP1 (CCT) inhibitor. In some embodiments, the CCT inhibitor described herein is a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor. In some embodiments, the CCT inhibitor is a CCT2 inhibitor.

6. In some embodiments, the CCT inhibitor comprises a small molecule, an antibody, a peptide, a polypeptide, a small interfering RNA (siRNA), or a short hairpin RNA.

7. In some examples, the method disclosed herein further comprises administering to the subject a therapeutically effective amount of a cell cycle inhibitor. In some embodiments, the cell cycle inhibitor comprises a CCND1 inhibitor, a CDK2 inhibitor, or a CDK4 inhibitor (for example, palbociclib, ribociclib, or abemaciclib).

8. In some embodiments, the cancer is a metastatic cancer. In some embodiments, the cancer is sarcoma, glioma, melanoma, lymphoma, or a breast cancer. In some embodiments, the breast cancer comprises luminal A breast cancer, luminal B breast cancer, estrogen receptor(ER)- progesterone receptor(PR)- HER2+ breast cancer, or triple negative breast cancer.

9. In some embodiments, the cancer is a pediatric cancer. In some embodiments, the pediatric cancer is neuroblastoma, clear cell sarcoma of the kidney (CCSK), Wilms tumor, Rhabdoid tumor of the kidney (RTK), rhabdomyosarcoma, or Choroid plexus carcinoma.

10. In some embodiments, cancer cells obtained from the subject have an increased level of one or more tumor biomarkers selected from the group consisting of MYC, MYCN, CDK2, CDK4, CCNE1, CCND1, YAP1, and RB1 relative to a reference control.

11. Also disclosed herein are methods of treating, inhibiting, decreasing, reducing, ameliorating, and/or preventing a drug-resistant cancer and/or metastasis in a subject in need thereof, comprising administering to the subject a therapeutically effective amount of a chaperonin-containing TCP1 (CCT) inhibitor. In one example, the drug-resistant cancer and/or metastasis is resistant to a cell cycle inhibitor. In one example, the drug-resistant cancer and/or metastasis is resistant to a CDK4 inhibitor.

12. Also disclosed herein is a method of diagnosing a subject as having a cancer, comprising
a) quantifying a level of a chaperonin-containing TCP1 (CCT) relative to a reference control;
b) determining the subject as having the cancer when the level of CCT is higher than the reference control; and c) determining the subject as not having the cancer when the level of CCT is lower than the reference control.

13. In some embodiments, the method further comprises administering to the subject a therapeutically effective amount of the CCT inhibitor disclosed herein.

### DESCRIPTION OF DRAWINGS

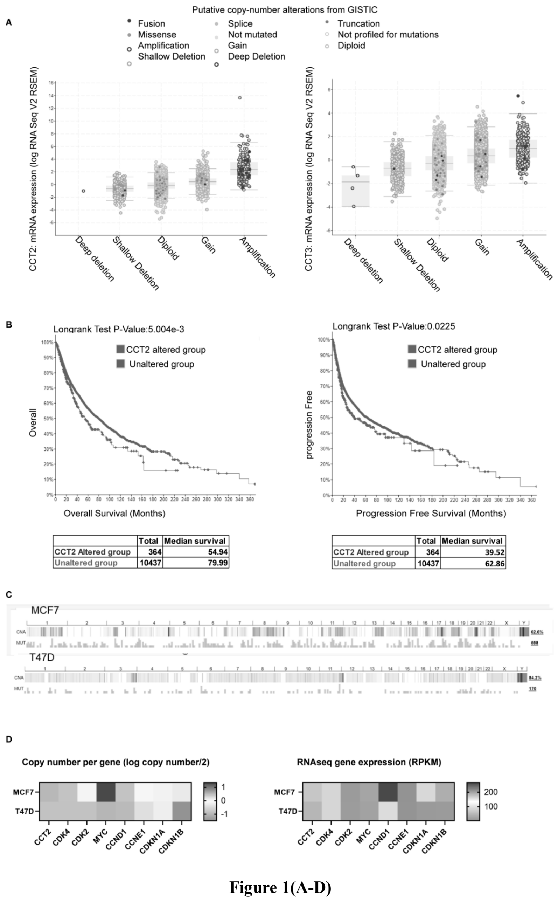

14. Figure 1(A-D). Co-occurrence of CCT2 genetic alterations with cell cycle gene alterations is suggestive of functional relationships. (Figure 1A and 1B) The TCGA PanCancer database was evaluated using cBioPortal for CCT2 and CCT3 genetic alterations (Figure 1A) and overall and progression free survival of patients (Figure 1B) in CCT2 altered and unaltered groups. (Figure 1C) Copy number alterations and mutation profiles for MCF7 and T47D cell lines based on CCLE data exported using cBioPortal are shown. (Figure 1D) CCT mutation and expression in T47D and MCF7 cells was obtained from the COSMIC database.

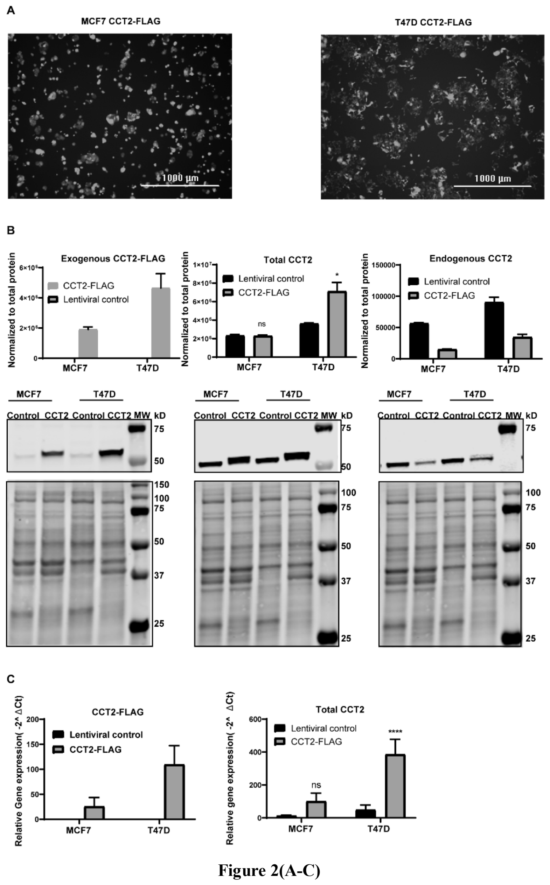

15. Figure 2(A-C). CCT2-FLAG is overexpressed inT47D and MCF7 breast cancer cell line. (Figure 2A) Expression of GFP by cells transduced with lentiviral CCT2-FLAG and control vectors is shown. (Figure 2B) Western blot for exogenous CCT2-FLAG (anti-FLAG antibody), total CCT2 (N-terminal specific anti-CCT2 antibody), and endogenous CCT2 (C-terminal specific anti-CCT2 antibody) proteins. Data were normalized to total protein. Representative blots are shown, and data replicates summarized in the graph. (Figure 2C) Relative mRNA expression for total CCT2 and exogenous CCT2-FLAG was determined by RT-qPCR. GAPDH was used as a reference gene. Calculations were based on using the equation 2-ΔΔ Ct equation. ****p-value <0.00005.

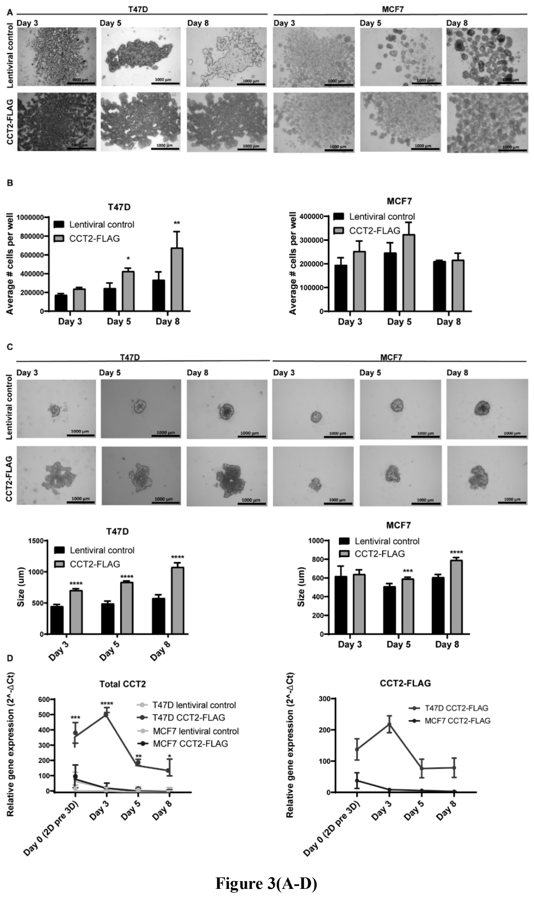

16. Figure 3(A-D). CCT2 enhances the formation of spheroids by breast cancer cells. (Figure 3A and 3B) Merged brightfield and GFP images from T47D and MCF7 spheroids grown on 24-well ULA flat bottom plates at days 3, 5, and 8 of 3D culture. Magnification was 2.5X. (Figure 3B) Total spheroid cell count per well for T47D and MCF7 cells is shown. Cells were dissociated from spheroids and counted using flow cytometry. (Figure 3C) Merged brightfield and GFP images of T47D and MCF7 spheroids grown on 96-well ULA round bottom plates at days 3, 5, and 8 of spheroid growth. Magnification was 2.5X. Spheroids from 96-well ULA plates were used for perimeter measurements. (Figure 3D) Relative mRNA expression for total CCT2 and exogenous CCT2-FLAG was determined by RT-qPCR. GAPDH was used as reference gene. **p-value <0.005, *** p-value < 0.0005, **** p-value <0.00005.

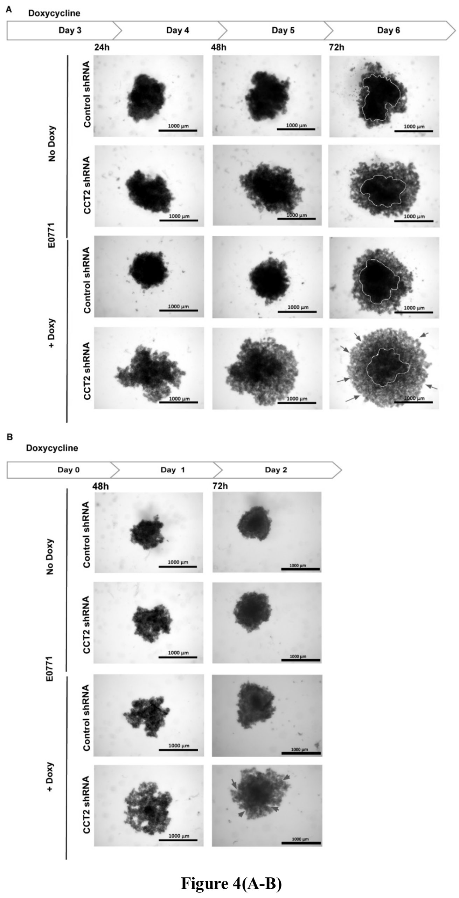

17. Figure 4(A-B). CCT2 depletion impairs breast cancer spheroid formation. (Figure 4A) Brightfield images for E0771 cells at 24, 48, 72 hours after CCT2 depletion using doxycycline to induce CCT2 or control shRNA expression. CCT2 depletion was induced at day 3 spheroid of spheroid growth. (Figure 4B) CCT2 depletion in E0771 cells was induced at day 0 (start of spheroid cultures) and imaged at 48 hours and 72 hours after doxycycline was used to induce expression of CCT2 or control shRNA. Circles delineate the compact spheroid core, while arrows indicate the formation of loose cell aggregates.

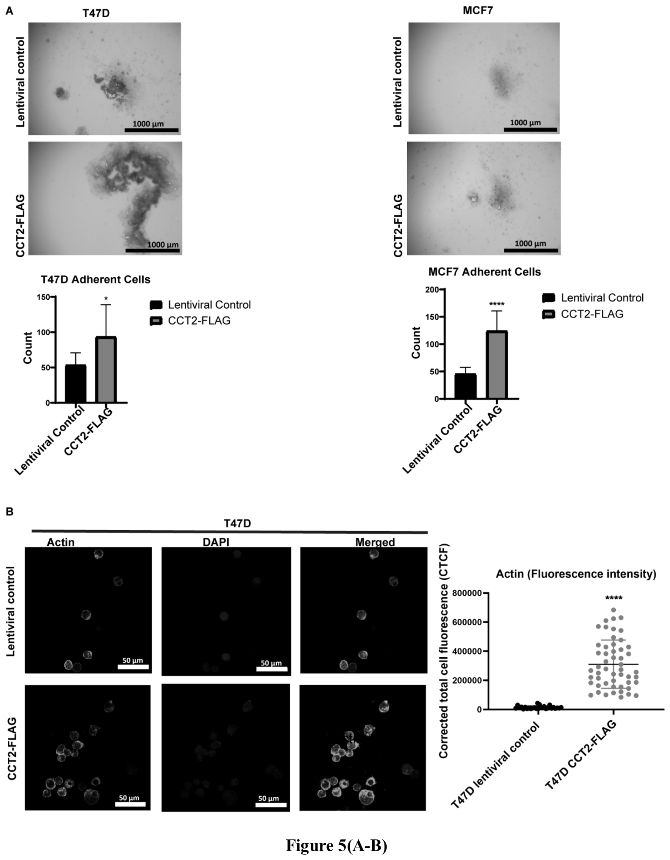

18. Figure 5(A-B). CCT2 overexpression promotes adherence of breast cancer cells in post-3D spheroid cultures and increases intracellular actin. (Figure 5A) Brightfield and GFP overlay images of day 8 spheroids from T47D and MCF7, CCT2-FLAG overexpressing and lentiviral control, cells plated onto standard tissue culture plates are shown. Non-adherent cells were washed off and images were taken of adherent cells. Adherent cells were then dissociated and counted by flow cytometry. Magnification was 2.5X. (Figure 5B) Confocal microscopy images of F-actin (stained with green fluorescent phalloidin), DAPI and overlays of the two signals are shown for T47D, CCT2-FLAG overexpressing and lentiviral control, cells. Magnification was 40X. Fluorescent cells were quantified using ImageJ. * p-value <0.05, *** p-value <0.0005,**** p-value <0.00005.

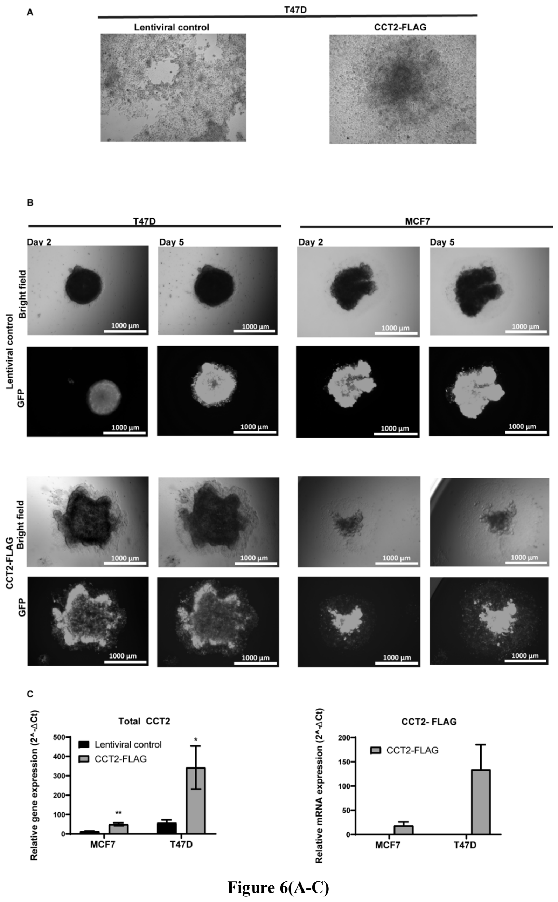

19. Figure 6(A-C). CCT2 supports transition of breast cancer cells from 3D to 2D monolayer culture. (Figure 6A) Brightfield microscopic images are shown of established 2D monolayer cultures for spheroid derived T47D cells, CCT2-FLAG overexpressing and lentiviral control. Magnification was 20X. (Figure 6B) Brightfield and GFP microscopic images showing results of spheroid transfer to standard tissue culture plates at days 2 and 5 post-transfer. Magnification was 2.5X. (Figure 6C) Total CCT2 and CCT2-FLAG mRNA expression was evaluated from T47D and MCF7, CCT2-FLAG overexpressing and lentiviral control, cells grown in 2D post-3D monolayer cultures. The equation used was 2^{-ΔΔ Ct} and GAPDH was the reference gene as previously described. * p-value <0.05, ** p-value <0.005.

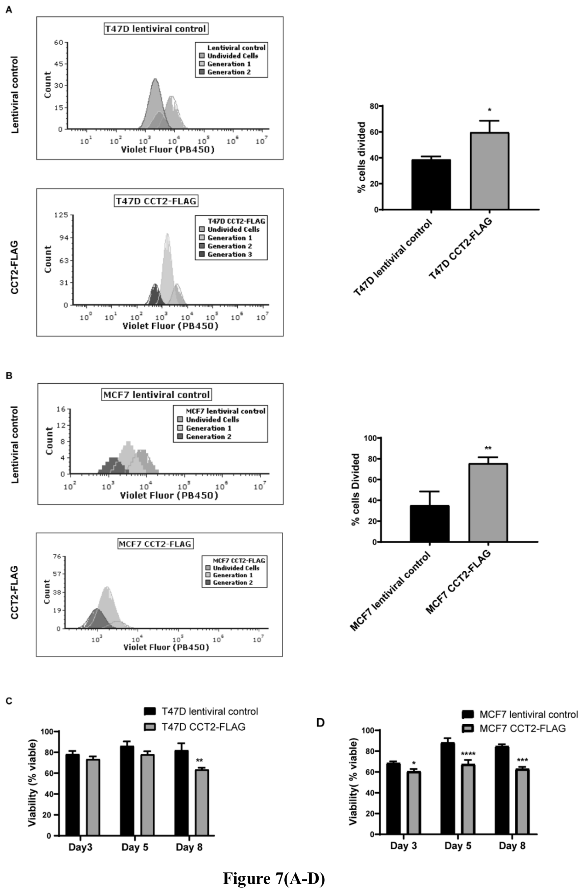

20. Figure 7(A-D). CCT2 overexpression increases cell division of breast cancer cells in 3D cultures. (Figure 7A and 7B) The ViaFluor^{®} dye was used to assess cell division over time for T47D cells (Figure 7A) and MCF7 cells (Figure 7B), CCT2-FLAG overexpressing and lentiviral controls, from days 3-5 of spheroid 3D cultures. Cell division was assessed by flow cytometry, and the generation time (histograms) and percent cells divided (graph) was determined. (Figure 7C and 7D) PI exclusion assay was used to assess the viability of cells from spheroid cultures at days 3, 5, and 8 of 3D growth for T47D cells (Figure 7C) and MCF7 cells (Figure 7D). Data was acquired using a CytoFlex S flow cytometer and analyzed using FCS Express software. * p-value <0.05, **p- value <0.005, *** p-value <0.0005, **** p-value < 0.00005.

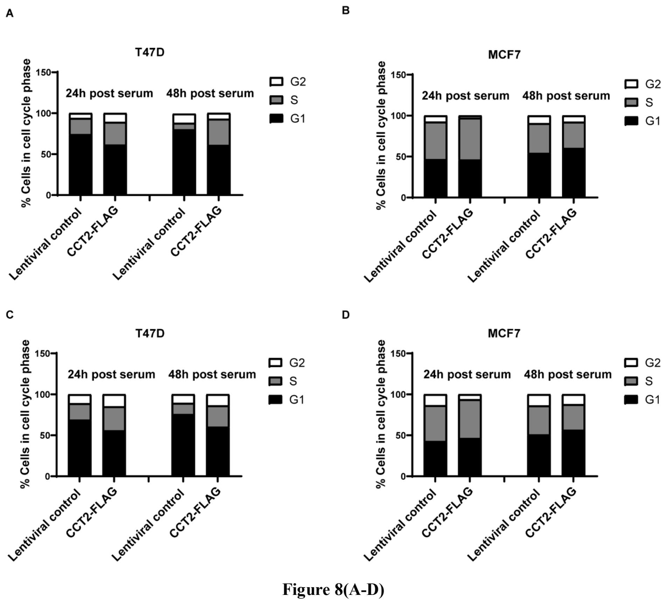

21. Figure 8(A-D). CCT2 overexpression promotes progression of breast cancer cells through the G1/S transtion. Proliferation of T47D and MCF7, CCT2-FLAG overexpressing and lentiviral control, cells in 2D cultures was synchronized after serum deprivation and cell cycle distribution was analyzed by PI staining of cells from 2D monolayer cultures (Figure 8A and 8B) and 2D post 3D cultures (Figure 8C and 8D). Data was acquired using the CytoFlex S flow cytometer and analyzed with FCS Express software.

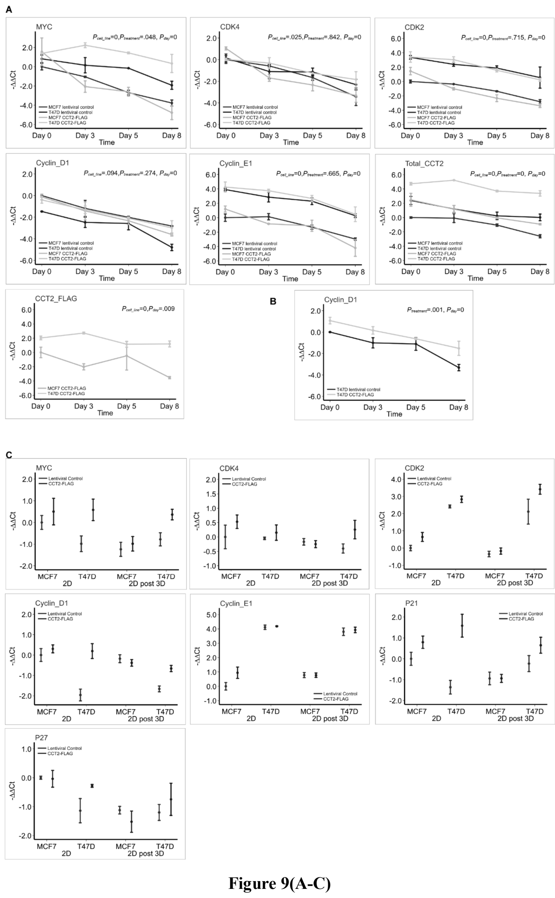

22. Figure 9(A-C). CCT2 upregulates expression of MYC and cell cycle genes in breast cancer cells. (Figure 9A) Graphs show results of relative gene expression measured as (-ΔΔCt) for each gene in response to CCT2-FLAG overexpression in T47D and MCF7 cells at days 0 (pre-spheroid), 3, 5, and 8 of 3D spheroid cultures. The MCF7 lentiviral control was used as a reference sample. This data was used to determine the multiple linear mixed effect model shown in Table 4. (Figure 9B) Cyclin D1 expression was evaluated in reference to T47D lentiviral control and statistical analysis shown in Table 4. (Figure 9C) Comparison of gene expression was measured (-ΔΔCt) for each gene in response to CCT-FLAG overexpression in T47D and MCF7 cells grown in 2D (pre-spheroid) and 2D post 3D/spheroid culture separately. This data was used to perform that multiple factor ANOVA analysis shown in Table 5.

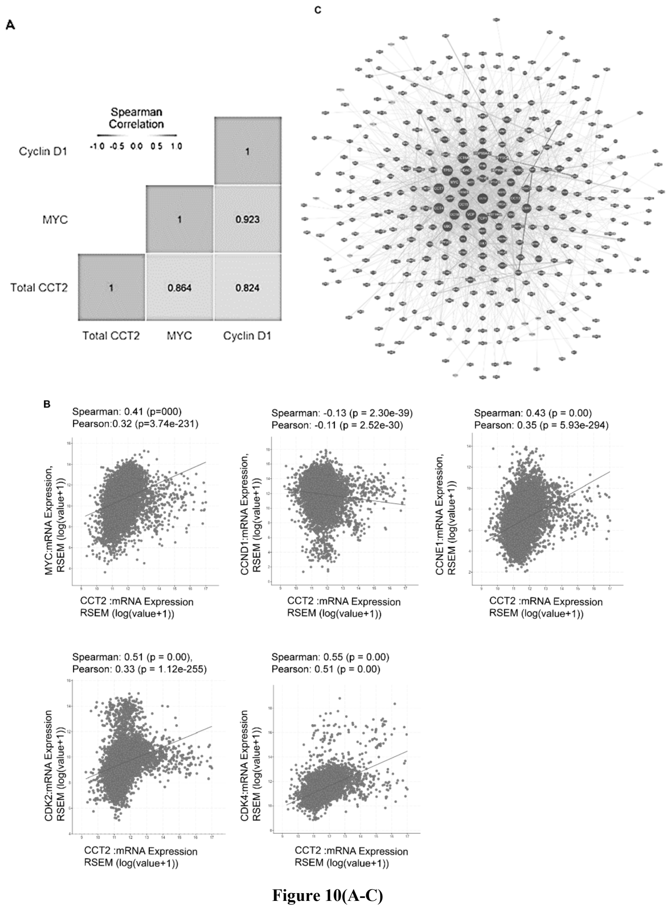

23. Figure 10(A-C). CCT2 as a possible oncogene. (Figure 10A) Spearman correlation coefficients were determined to demonstrate gene interactions among MYC, CCND1 (Cyclin D1), and total CCT2 in MCF7 and T47D cells, CCT2 overexpressing and lentiviral controls. Results from the combined dataset are shown. Gene expression was measured as -ΔΔCt using the MCF7 lentiviral control as reference. The red colored squares indicate that correlations were significant with p-values <0.05. P-values were adjusted based on the Holm multiple test adjustment. (Figure 10B) The TCGA PanCancer dataset was analyzed for co-expression of CCT2 mRNA with MYC, CCND1, CCNE1, CDK2, and CDK4 mRNA. Spearman and Pearson correlation p-values are shown. (Figure 10C) The CCT2 interaction network with physical and genetic integrators is shown (data from BioGRID). Greater node size represents increased connectivity and thicker edge sizes represent increased evidence supporting the association. The list of CCT2 interactors is included in Table 6.

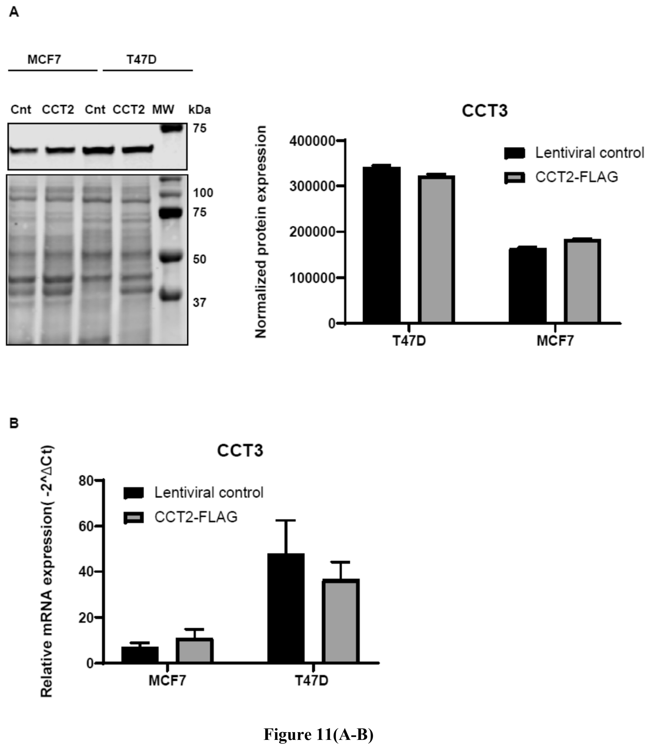

24. Figure 11(A-B). CCT3 expression in breast cancer CCT2-FLAG overexpressing cells. (Figure 11A) Representative immunoblot of CCT3 protein expression in T47D and MCF7, CCT2-FLAG overexpressing and lentiviral control, cells. Graph summarizes data from blots that was normalized to total protein. (Figure 11B) CCT3 relative mRNA expression was assessed using RT-qPCR. GAPDH was used as the reference gene.

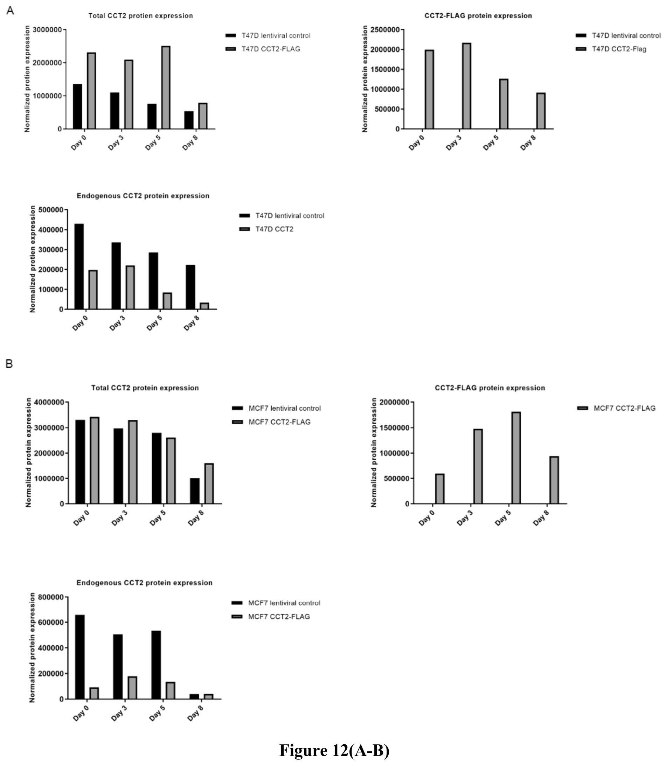

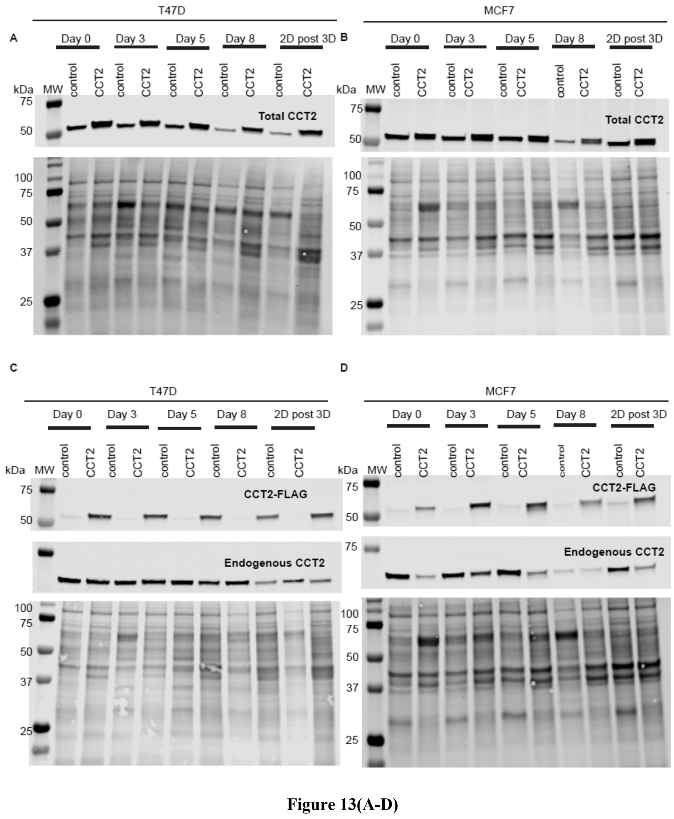

25. Figure 12(A-B). CCT2 protein levels in spheroid cultures of breast cancer cells. Graphs show total CCT2 protein, CCT2-FLAG protein (anti-FLAG) and endogenous CCT2 protein normalized to total protein in T47D (Figure 12A) and MCF7 (Figure 12B), CCT2-FLAG overexpressing and lentiviral control, cells. Data was determined from immunoblots shown in in Figure 13.

26. Figure 13(A-D). Immunoblots for CCT2 protein. Representative immunoblots for CCT2 total protein expression in T47D (Figure 13A) and MCF7 (Figure 13B), CCT2-FLAG (anti-FLAG) and endogenous CCT2 in T47D (Figure 13C) and MCF 7(Figure 13D) at day 0 (2D cultures, pre-spheroid), spheroid cultures on days 3, 5, and 8, and 2D post 3D cultures. Total protein stain is showed below each panel.

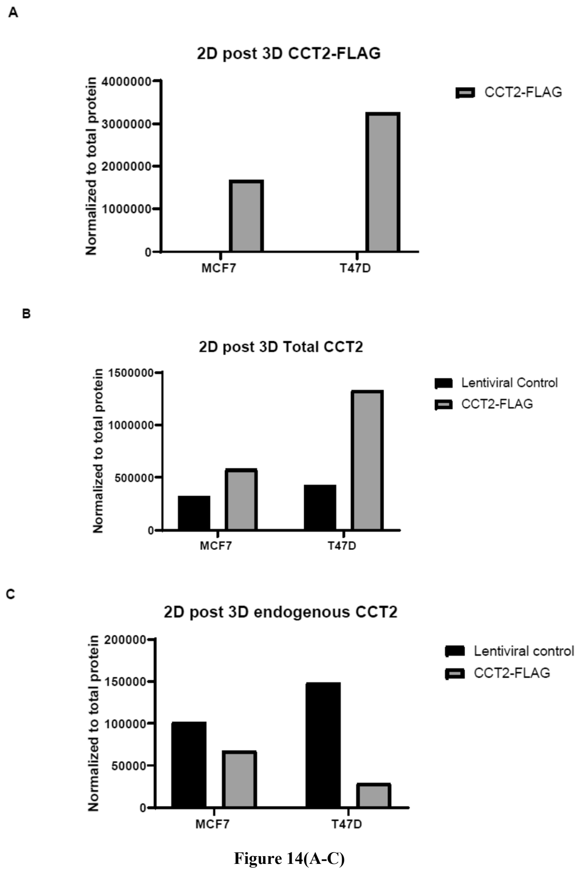

27. Figure 14(A-C). CCT2 protein levels in spheroid reversal cultures (2D post 3D) of breast cancer cells. Graphs show (Figure 14A) CCT2-FLAG (anti-FLAG), (Figure 14B) total CCT2 protein, and (Figure 14C) endogenous CCT2 protein expression normalized to total protein in T47D and MCF7, CCT2- FLAG overexpressing and lentiviral control, cells. Cells were grown in 3D spheroid cultures and then transferred to standard tissue culture plates for 2D growth (2D post 3D). Data was determined from immunoblots shown in in Figure 13.

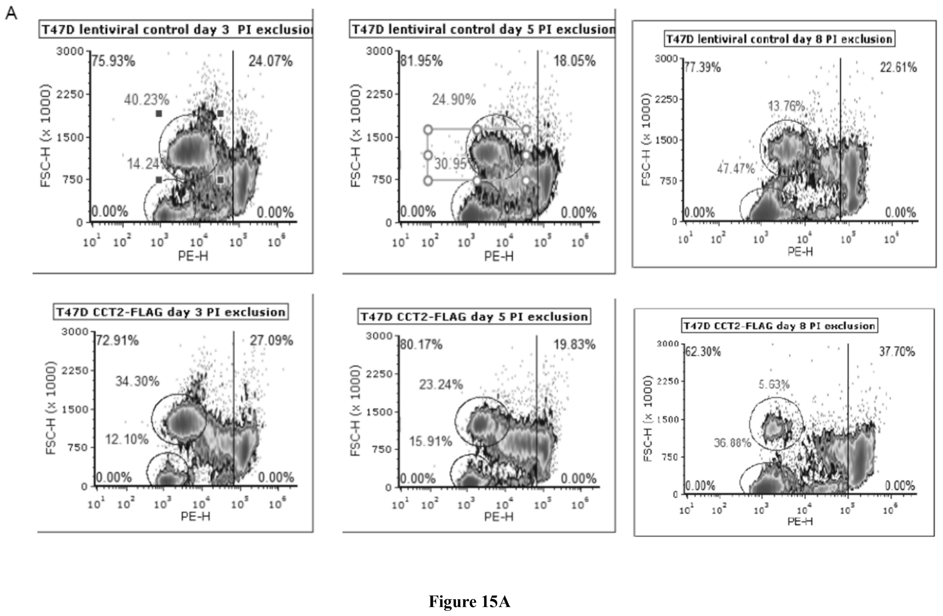

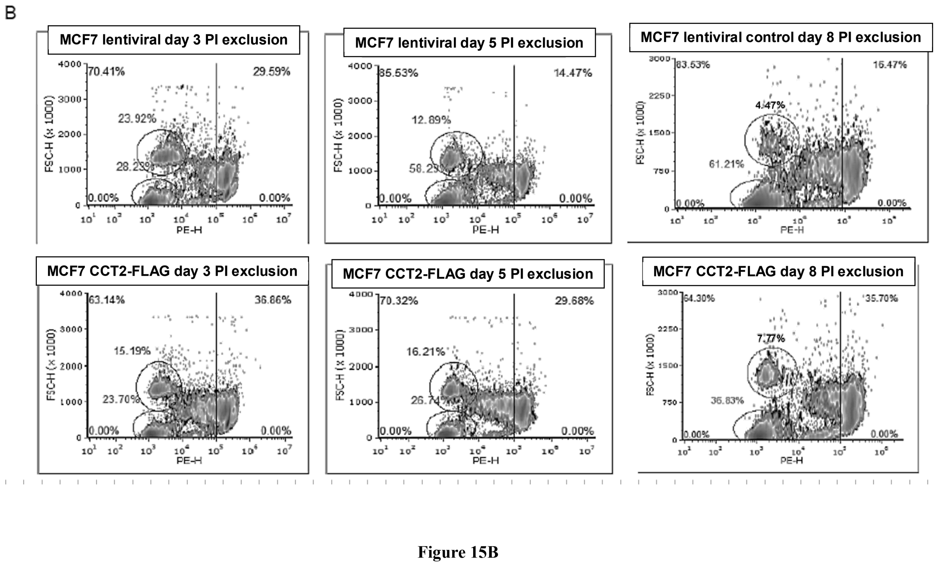

28. Figure 15(A-B). Viability gating of breast cancer cells in spheroid culture. T47D and MCF7, CCT2- FLAG overexpressing and lentiviral control, cells were cultured on ULA plates and cells recovered for PI exclusion staining. Live cell gating on PI negative cells on days 3, 5, 8 of spheroid growth is shown for T47D (Figure 15A) and MCF7 (Figure 15B) cells.

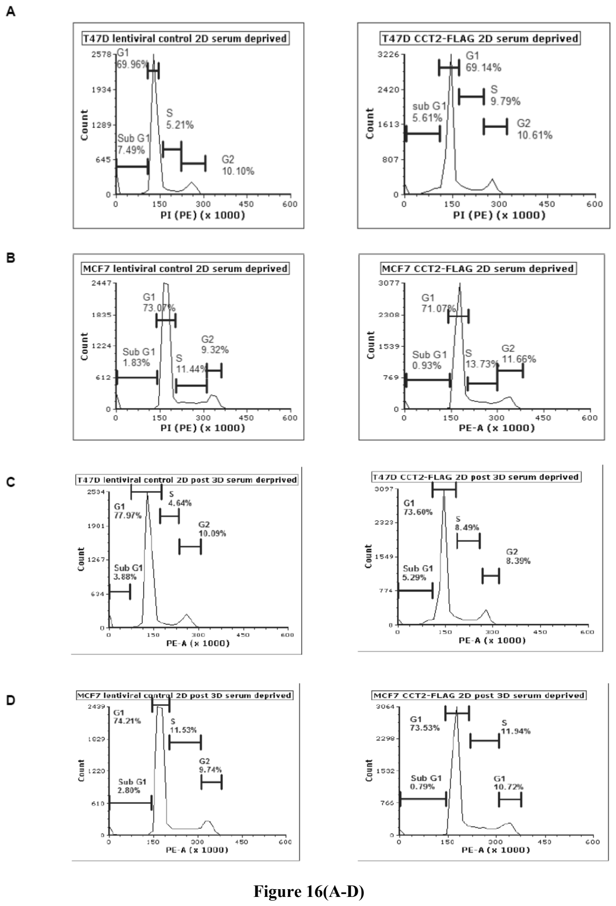

29. Figure 16(A-D). Serum deprivation causes cell cycle arrest of breast cancer cells. T47D and MCF7, CCT2-FLAG overexpressing and lentiviral control, cells were deprived of sera for 24 hours, and cells were collected for analysis of cell cycle distribution using PI staining. (Figure 16A-16D) Cell cycle distribution of T47D and MCF7 cells in standard 2D culture (Figure 16A, 16B) and of cells transitioning from 3D to 2D culture (2D post 3D culture) (Figure 16C, 16D).

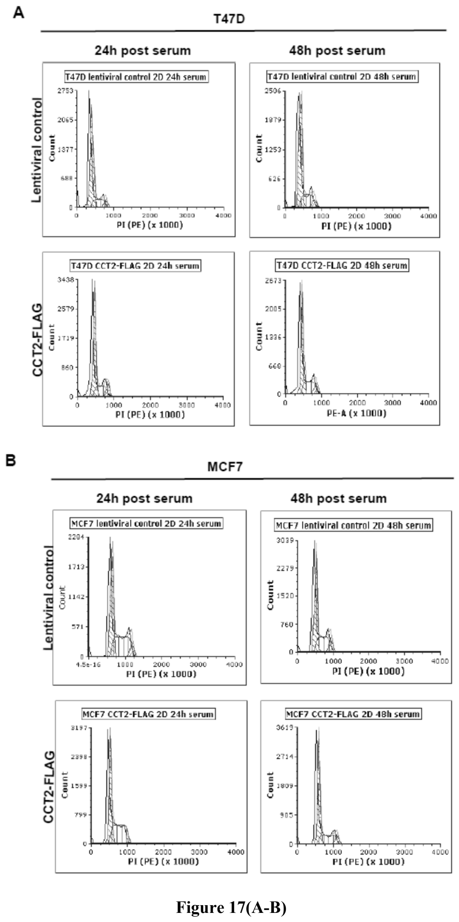

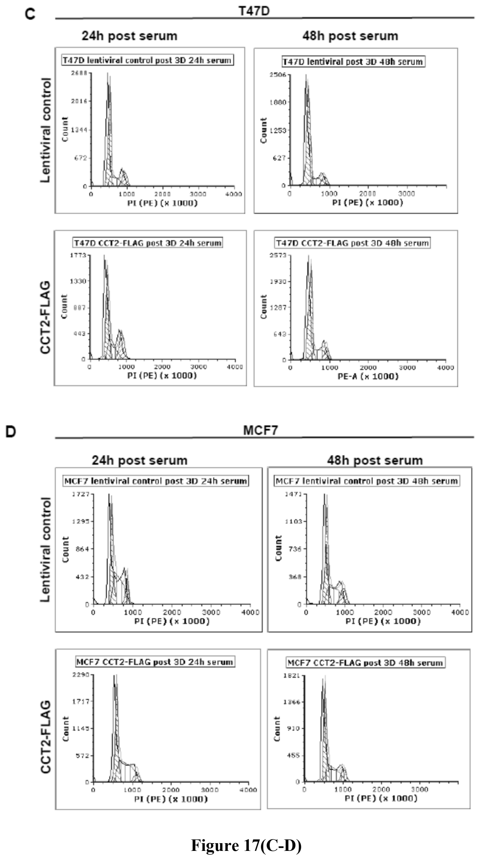

30. Figure 17(A-D). Cell Cycle distribution of growth synchronized cultures of breast cancer cells. Growth arrested T47D and MCF7, CCT2-FLAG overexpressing and lentiviral control, cells were cultured in sera-containing media for 24 to 48 hours, and cells were collected for analysis of cell cycle distribution using PI staining. (Figure 17A-17D) Cell cycle distribution of T47D and MCF7 cells in standard 2D culture (Figure 17A, 17B) and of cells transitioning from 3D to 2D culture (2D post 3D culture) (Figure 17C, 17D).

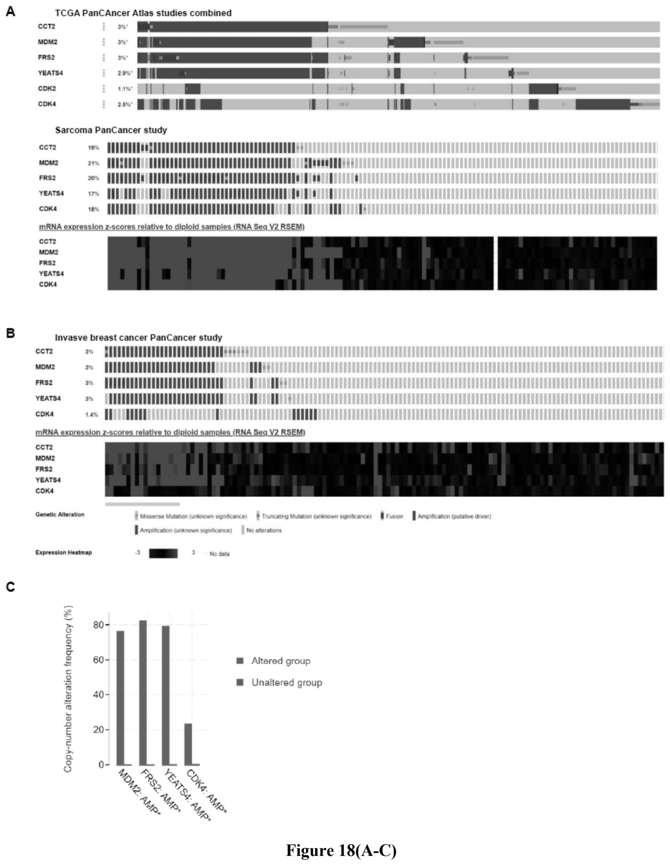

31. Figure 18(A-C). CCT2 is located in an amplicon associated with cancer progression. CCT2 is found in 12q13-15 chromosomal region that is subject to recurrent amplification in cancer. (Figure 18A-18C) CCT2, MDM2, FRS2, YEATS4, CDK4 genetic alteration and heatmap for mRNA expression the TCGA PanCancer Atlas studies combined, Sarcoma (Figure 18A) and invasive breast carcinoma TCGA PanCancer studies (Figure 18B). Co-amplification of genes in the CCT2 altered group compared to unaltered group in invasive breast carcinoma (TCGA PanCancer studies) (Figure 18C).

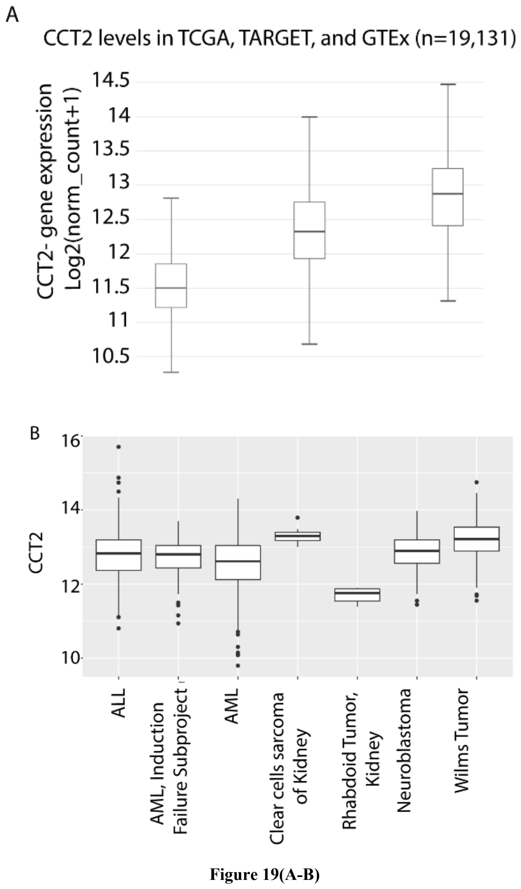

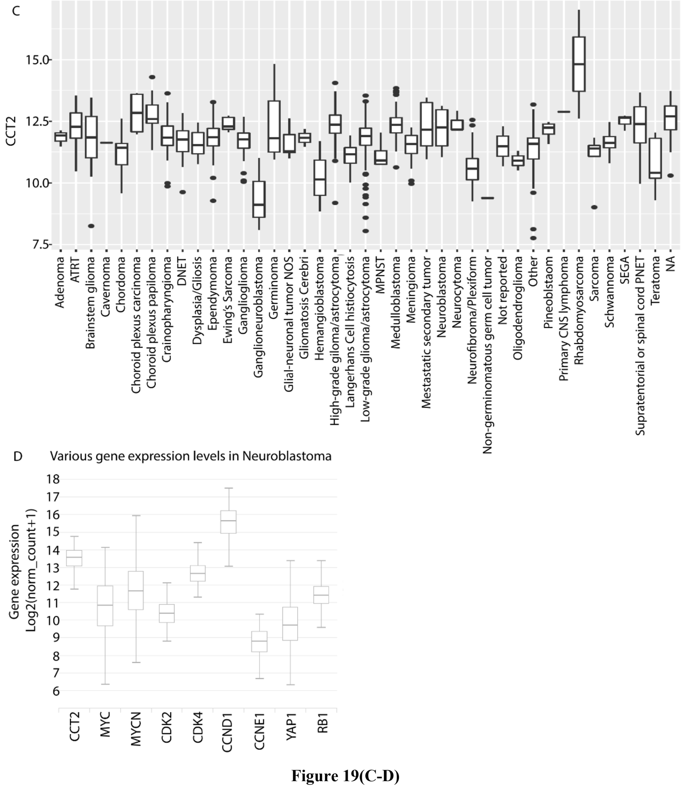

32. Figure 19(A-D): CCT2 levels are present in pediatric cancers like Neuroblastoma. Figure 19A. UCSC Xena data set TCGA (blue), TARGET (red) and GTEx (purple) cohort (n=19,131) comparing *CCT2* gene expression levels. Figure 19B. UCSC Xena dataset TARGET comparing *CCT2* gene expression in specific pediatric cancers. ALL: Acute Lymphoblastic Leukemia, AML: Acute Myeloid Leukemia. Figure 19C. KidsFirst dataset comparing *CCT2* gene expression in specific pediatric cancers. ATRT: Atypical Teratoid Rhabdoid Tumor, DNET: Dysembryoplastic neuroepithelial tumor, MPNST: Malignant peripheral nerve sheath tumor, SEGA: Subependymal giant cell astrocytoma. Glioma/astrocytoma: High-grade (WHO grade III/IV) vs low-grade (WHO grade I/II). Figure 19D. UCSC Xena TARGET dataset focused on neuroblastoma cases (n=167) comparing expression levels of several genes: *CCT2, MYC, MYCN, CDK2, CDK4, CCND1*, *CCNE1*, *YAP1*, and *RB1.*

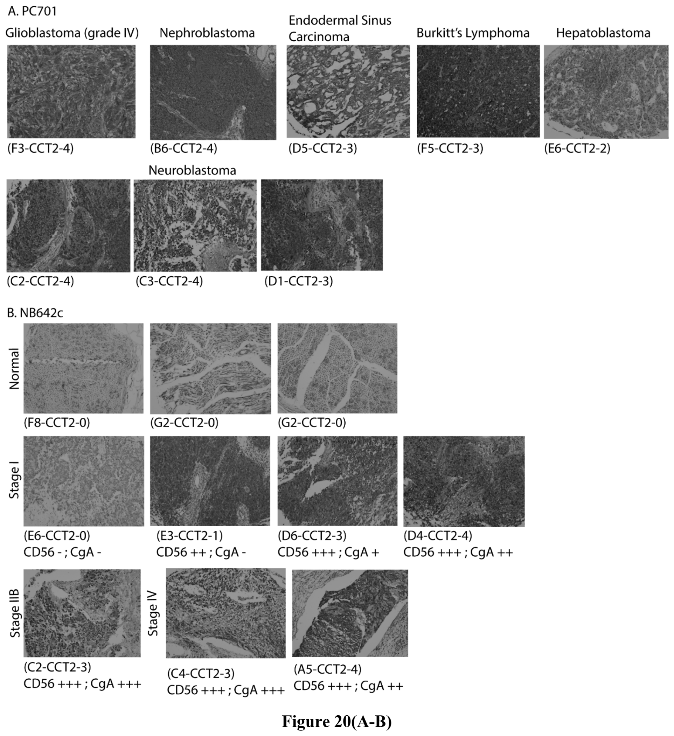

33. Figure 20(A-B): Staining for CCT2 in pediatric Tissue microarrays. Figure 20A. Example images from CCT2 staining of PC701 pediatric TMA. CCT2 stain score is listed below each image. Figure 20B. Example images from CCT2 staining of NB642c neuroblastoma TMA. CCT2 stain score is listed below each image as well as CD56 and CgA score given with TMA data. All images were taken at 20x.

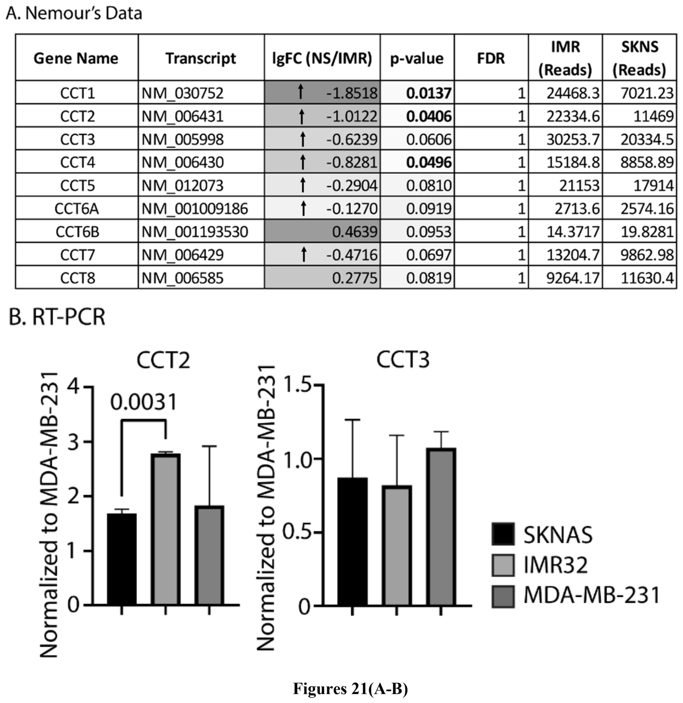

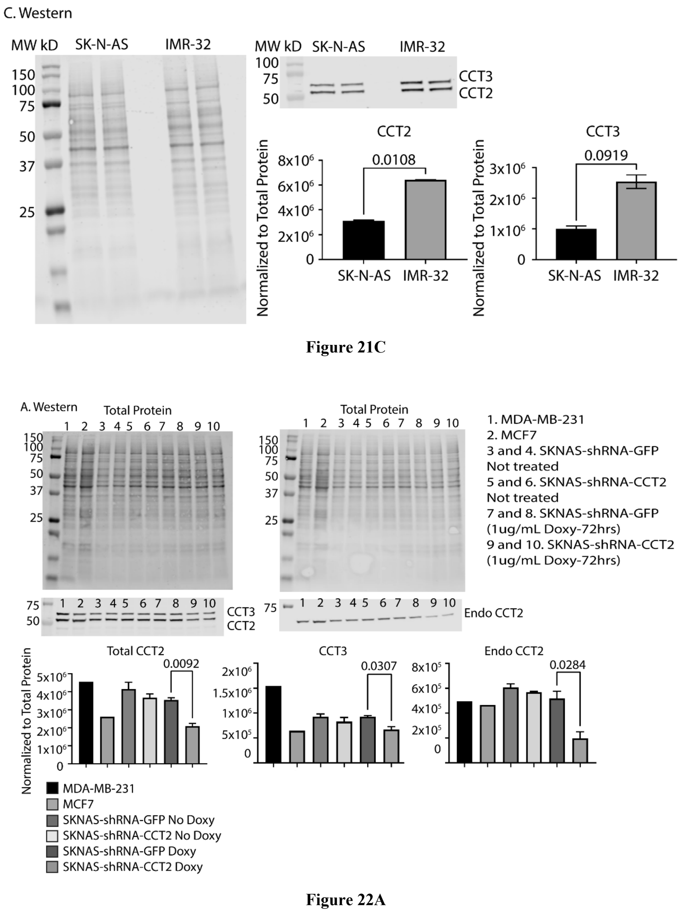

34. Figure 21(A-C): Levels of CCT2 in neuroblastoma cell lines. Figure 21A. Nemours database looking at mRNA expression for the 8 subunits of CCT in IMR32 vs SKNAS. Colors indicate changes in gene expression between cell lines. Red indicates that gene expression decreased in SK-N-AS cells compared to IMR-32 and green that gene expression increased. Expression of CCT subunits, except for CCT6B), was very robust (varying from ~10,000-30,000 reads). Typical expressed products vary from ~300-1000 reads. Reads less than 50 are insignificant. Figure 21B. RT-PCR analysis of IMR32 and SKNAS for CCT2 and CCT3 gene expression. MDA-MB-231 breast cancer cell line was used as reference. Figure 21C. Western analysis of IMR32 and SKNAS for CCT2 and CCT3. MDA-MB-231 breast cancer cell line was used as reference.

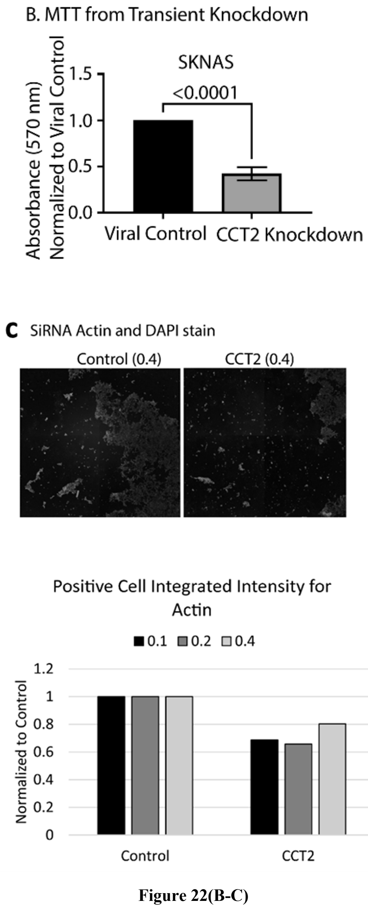

35. Figure 22(A-C): Knockdown of CCT2 in SKNAS decreases viability and IMR32 siRNA treated cells had less actin. Figure 22A. Western analysis of SKNAS cells transfected with shRNA-GFP or shRNA-CCT2 before and after activation with doxy treatment. Figure 22B. MTT viability assay after transient knockdown of CCT2 in SKNAS with shRNA plasmid. Figure 22C. Representative images of Actin and DAPI staining in IMR32 cells before and after CCT2 siRNA treatment.

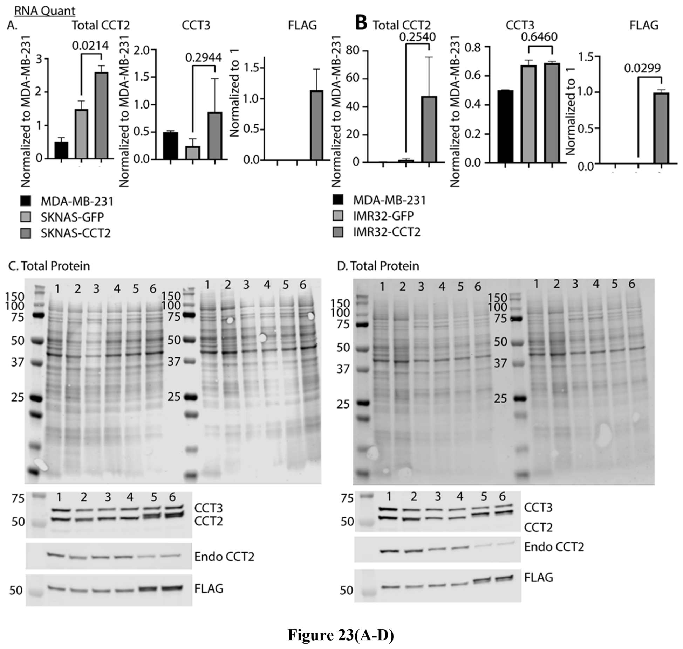

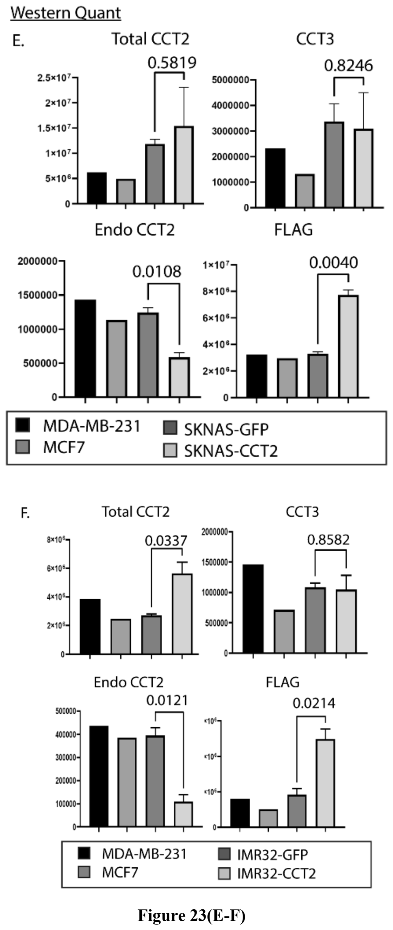

36. Figure 23(A-F): Overexpression of CCT2 in SKNAS and IMR32 cells leads to increases in RNA and protein. A-B. RT-PCR analysis for Total CCT2, CCT3 and FLAG in SKNAS (Figure 23A) and IMR32 (Figure 23B) after lentiviral transduction compared to MDA-MB-231 and MCF7 breast cancer cell lines. Figure 23(C-D). Total protein and western blots for CCT2, CCT3, Endo CCT2, and FLAG in SKNAS (Figure 23C) and IMR32 (Figure 23D) after lentiviral transduction. Figure 23C) Lanes are: 1. MDA-MB-231, 2. MCF7, 3. And 4. SKNAS-GFP, 5. And 6. SKNAS-CCT2. Figure 23D) Lanes are: 1. MDA-MB-231, 2. MCF7, 3. And 4. IMR32-GFP, 5. And 6. IMR32-CCT2.MDA-MB-231 and MCF7 cell lines are used as references. Figure 23(E-F). Quantification of bands from western blots in SKNAS (Figure 23) and IMR32 (Figure 23F).

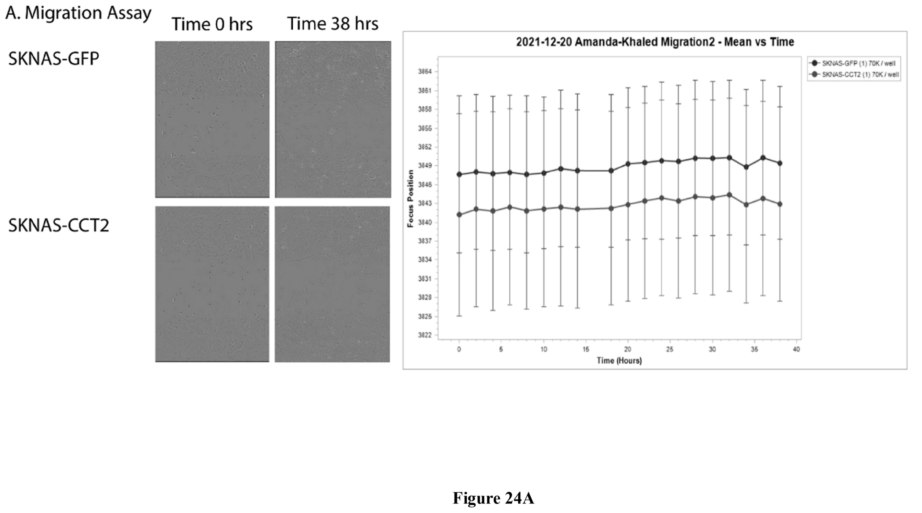

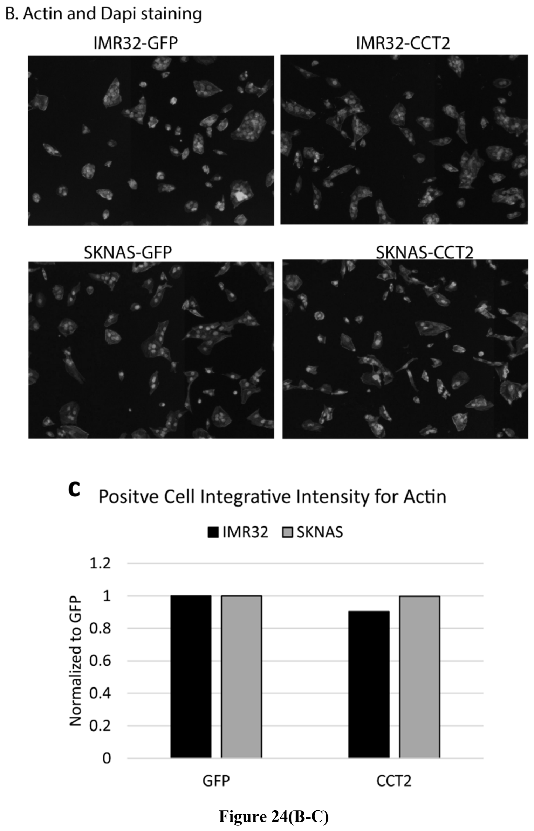

37. Figure 24(A-C): Functional assays of neuroblastoma cell lines show minimal changes due to CCT2 overexpression. Figure 24A. Migration assay comparing SKNAS-GFP and SKNAS-CCT2. Figure 24B. Actin and DAPI stainig in IMR32-GFP vs IMR32-CCT2 and SKNAS-GFP vs SKNAS-CCT2. Figure 24C. Quantification of Actin stain.

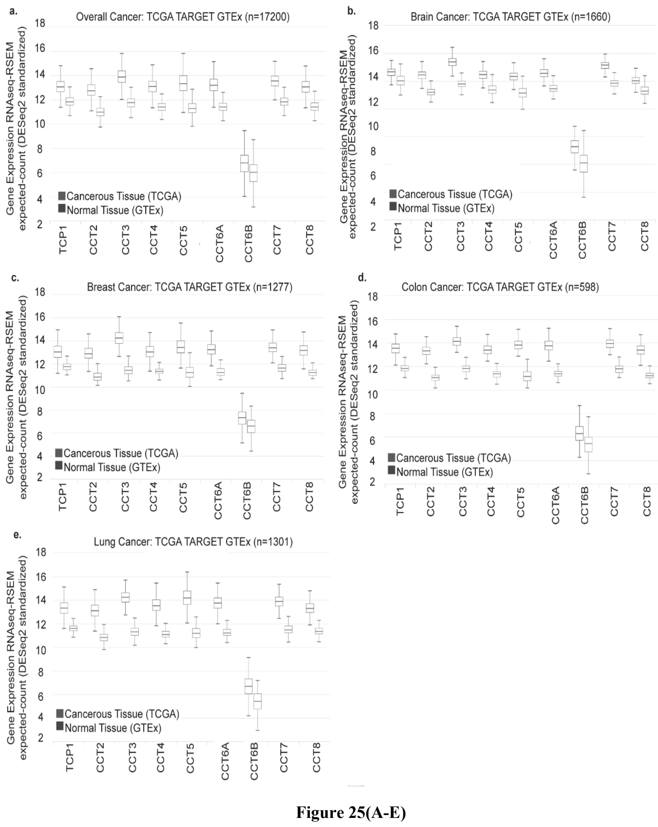

38. Figure 25(A-E) CCT gene expression is increased in cancerous tissues compared to normal tissue. UCSC Xena database comparing all eight CCT subunits in normal (GTEx) vs. cancerous (TCGA) tissue. (Figure 25a) Overall cancer (n= 17,200): the most significant differences were observed in the CCT2 and CCT3 genes, and the least difference was in the CCT6B gene. (Figure 25b) Brain cancer (n= 1,277): the most significant difference was seen in the CCT2 gene, and the least difference was in the CCT6B gene. (Figure 25c) Breast cancer (n= 1,660): the most significant differences were seen in the CCT3 and CCT8 genes and the least difference was in the CCT6B gene. (Figure 25d) Colon cancer (n= 598): the most significant difference was seen in the CCT2 gene, and the least difference was in the CCT6B gene. (Figure 25e) Lung cancer (n= 1,301): the most significant differences were seen in the CCT2 and CCT3 genes, and the least difference was in the CCT6B gene. p<0.0001 for all samples.

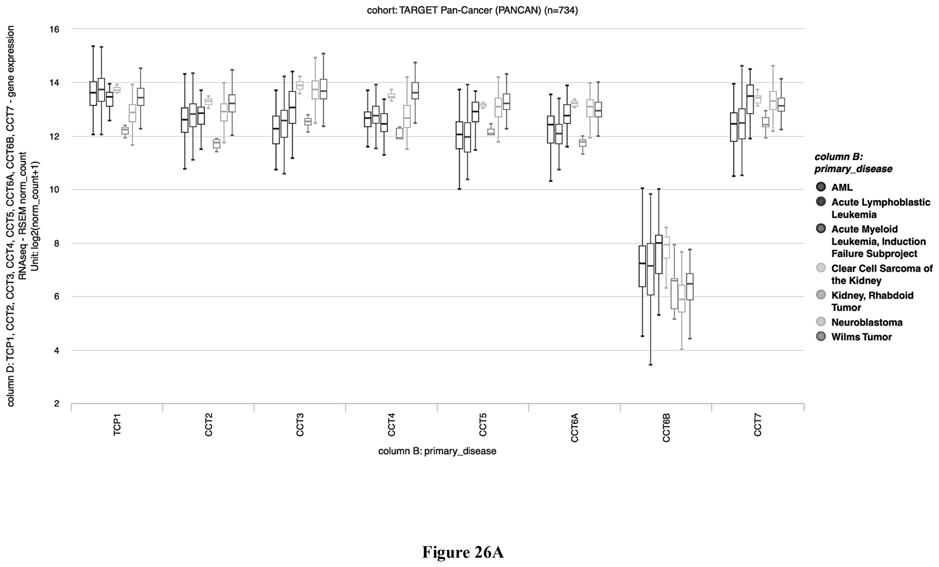

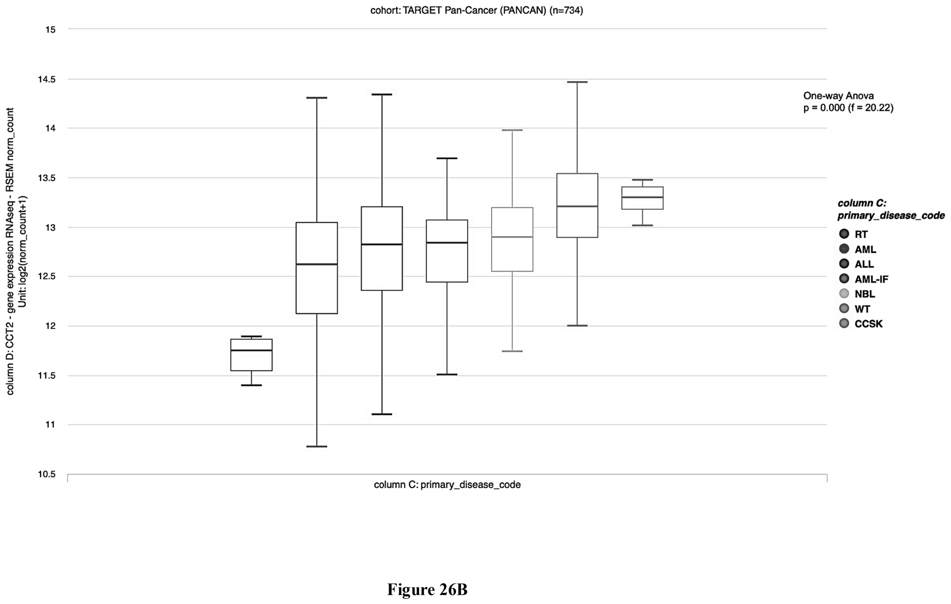

39. Figure 26(A-B). Pediatric Cancers with Altered Expression of CCT subunits. A pediatric Pan-Cancer analysis for expression of CCT subunits was performed using the TARGET database. (Figure 26A) Analysis was performed by primary disease for all CCT subunits. CCT6B which is only expressed in testis is included as a negative control. (Figure 26B) Analysis was performed by primary disease for the CCT2 subunit. RT, Rhabdoid tumor; AML, acute myeloid leukemia; ALL, acute lymphoblastic leukemia; AML-IF, Induction Failure AML; NBL, Neuroblastoma; WT, Wilms tumor ; CCSK, Clear cell sarcoma of the kidney. Arrows indicate results for neuroblastoma.

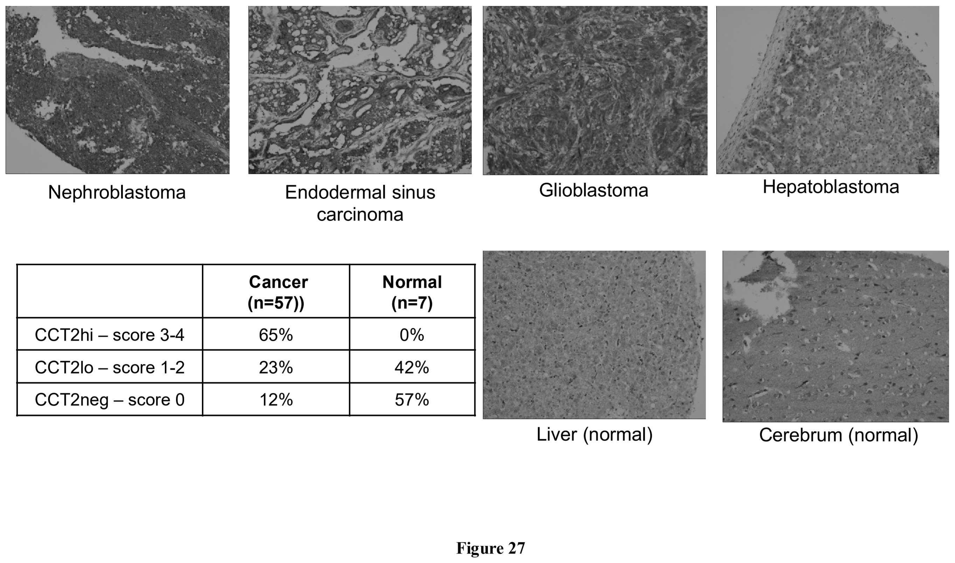

40. Figure 27 shows that CCT2 expression is increased in pediatric tumor tissues. CCT2 protein levels were examined by immunohistochemistry using a pediatric malignant tumor tissue microarray (TMA) (PC701, US Biomax) with normal tissue, containing 21 cases of nephroblastoma, 12 neuroblastoma plus, 7 endodermal sinus carcinoma, 4 retinoblastoma, 3 hepatoblastoma, 2 medulloblastoma, 4 lymphoma, 1 each of choroid plexus papilloma, glioblastoma, adrenocortical carcinoma, embryonal rhabdomyosarcoma, ependymoma, neuroblastoma, primitive neuroectodermal tumor, alveolus rhabdomyosarcoma, immaturity teratoma, leiomyosarcoma, plus 7 normal tissue, single cores per case. Each specimen was read by an independent pathologist and the CCT staining score assigned based on the published data (Bassiouni et al, CCR, 2016). Representative images are shown. Table summarizes results of CCT2 staining score in TMA of pediatric cancer and normal tissues.

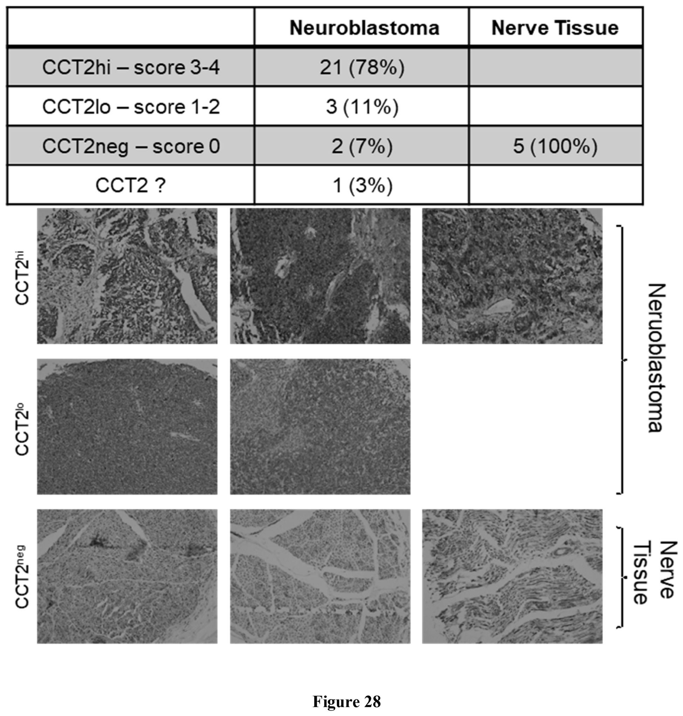

41. Figure 28 shows that CCT2 expression is increased in neuroblastoma tissues. CCT2 protein levels were examined by immunohistochemistry using a neuroblastoma tissue microarray (TMA) (NB642c, US Biomax), containing 27 cases of neuroblastoma and 5 peripheral nerve tissue, duplicate cores per case. Each specimen was read by an independent pathologist and the CCT staining score assigned based on our published data (Bassiouni et al, CCR, 2016). Representative images for CCT2neg, CCT2lo and CCT2hi staining are shown. Table summarizes results of CCT2 staining score in TMA of neuroblastoma and normal tissues.

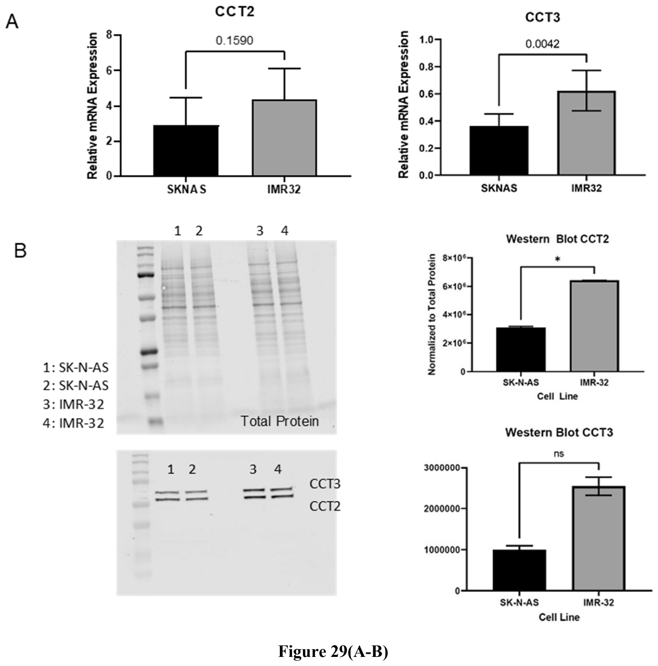

42. Figure 29(A-B) show that CCT2 (and CCT3) is highly expressed in neuroblastoma cells. (Figure 29A) Relative mRNA expression for total CCT2 and CCT3 was determined by RT-qPCR (n=5) in IMR-32 and SK-N-As neuroblastoma cell lines. GAPDH was used as a reference gene. Calculations were based on using the equation 2-ΔΔ Ct equation. Values are mean ± SD. p-values were not significant. (Figure 29B) Data from Western blots for total CCT2 (anti-CCT2 antibody), and total CCT2 (anti-CCT3 antibody) proteins in IMR-32 and SK-N-AS cell lines are shown. Data were normalized to total protein (Revert stain). Data replicates (n=2) summarized in the graphs. *p-value <0.05 or ns, non-significant.

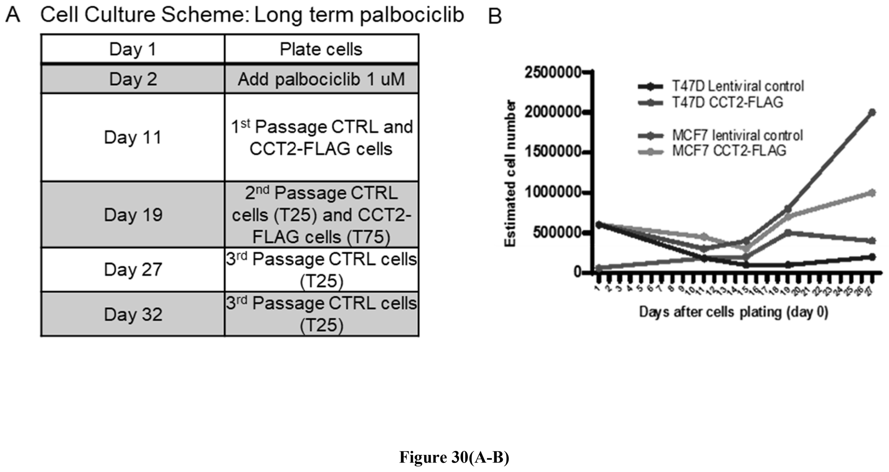

43. Figure 30(A-B). Cells that overexpress CCT2 are more resistant to 1uM palbociclib than control cells. (Figure 30A) Cell culture scheme for investigating long term drug resistance to 1 µM Palbociclib treatment is shown. (Figure 30B) Estimated cell growth curve based on cell confluency in culture plates.

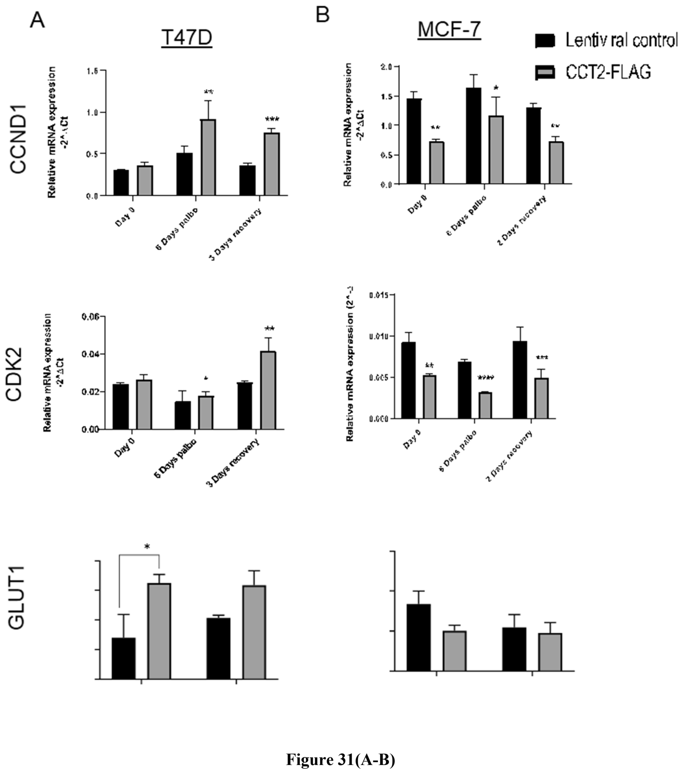

44. Figure 31(A-B). CCT2 expression correlates with increased cell cycle and glucose uptake genes during treatment with palbociclib in T47D cells. (Figure 31A, 31B) Gene expression of cell cycle regulators and GLUT1 was examined using RT-qPCR. GAPDH was used as a reference gene. Day 0 represents cells cultured overnight, day 6 for 6 days of Palbociclib treatment, 2- or 3-days recovery for post-treatment in drug-free medium, for T47D (Figure 31A) and MCF7 (Figure 31B). Experiments were performed in triplicates. * p-value <0.5, **p-value <0.005, ***p-value <0.0005, ****p-value <.00005.

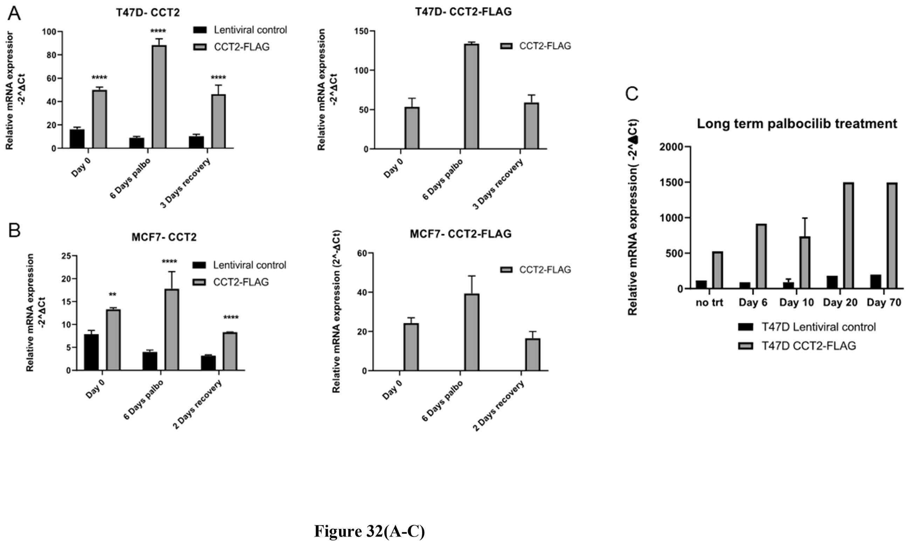

45. Figure 32(A-C). CCT2 expression increases during palbociclib treatment. Figure 32(A-B) Total CCT2 and CCT2-FLAG mRNA expression were assayed using RT-qPCR relative to GAPDH. Experiments were performed in triplicates on days 0 and 6 of 1 µM Palbociclib treatment and on day 3 post-recovery (see Figure 29A). **p-value <0.005, ****p-value <0.00005. (Figure 32C) Total CCT2 mRNA was assessed at points indicated in figure axis as above from long term palbociclib treatment (see Figure 30A).

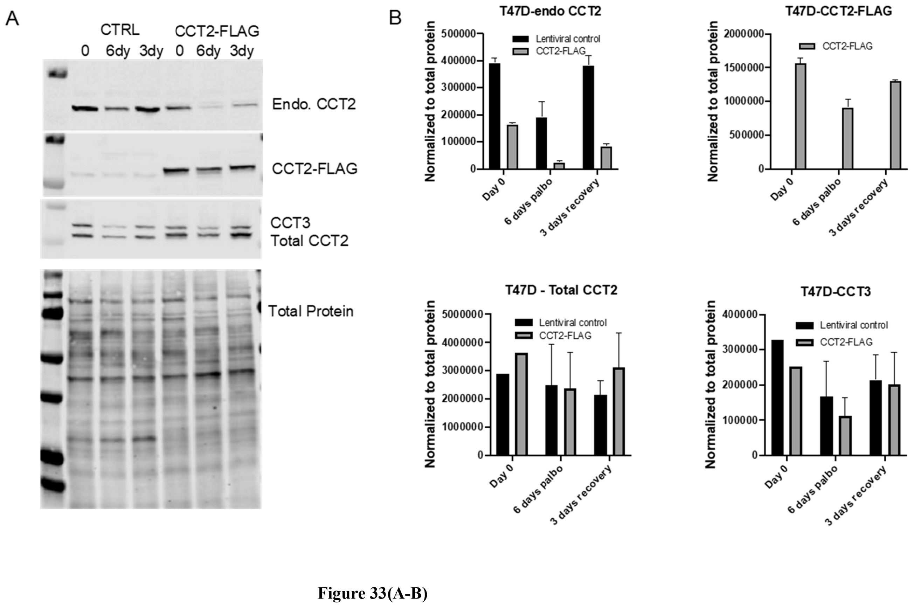

46. Figure 33(A-B). CCT2 protein levels decrease during palbociclib treatment. (Figure 33A) Relative protein levels of endogenous (endo) CCT2 (anti-CCT2-C-terminal), CCT2-FLAG (anti-FLAG), total CCT2 (anti-CCT2-N-terminal) and endogenous CCT3 were determined by immunoblot analysis in T47D (shown) and MCF-7 cells (not shown) stably expressing CCT2-FLAG or lentiviral control. Cells were treated with 1 µM palbociclib for 6 days and then recovered over 3 days (see Figure 29A). (Figure 33B) Relative protein levels between CCT2-FLAG and lentiviral control of CCT2 and CCT3 were normalized to total protein. Error bars represent the mean with s.e.m of technical replicates.

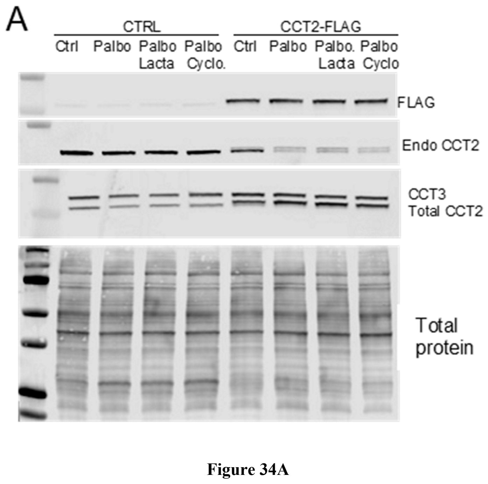

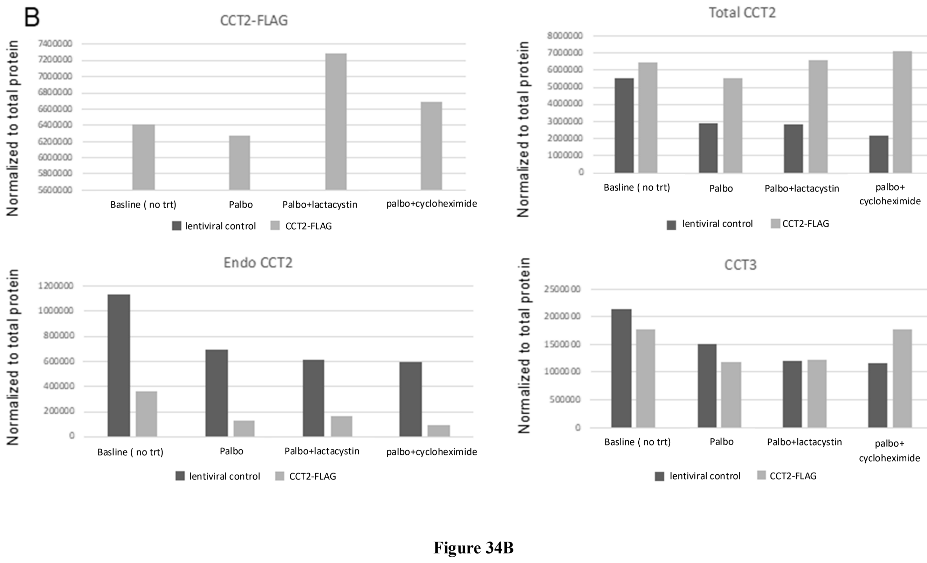

47. Figure 34(A-B). CCT2 protein is targeted for degradation through the proteosome. (Figure 34A) Relative protein levels of endogenous CCT2 (anti-CCT2-C-terminal), CCT2-FLAG (anti-FLAG), total CCT2 (anti-CCT2-N-terminal) and endogenous CCT3 were determined by immunoblot analysis in T47D (shown) and MCF-7 (not shown) cells stably expressing CCT2-FLAG or lentiviral control. Cells were treated with palbociclib, 1 µM, for 6 days (see Figure 29A) and treated with either 10 µM lactacystin (lacta) (proteosome inhibitor) or 10 µg/ml cycloheximide (cyclo) (protein synthesis inhibitor) for 5 hours. (Figure 34B) Graphs display relative CCT2 and CCT3 protein levels between CCT2-FLAG and lentiviral control cells. Results were normalized to total protein. Error bars represent the mean with s.e.m of technical replicates. A representative experiment of two performed is shown.

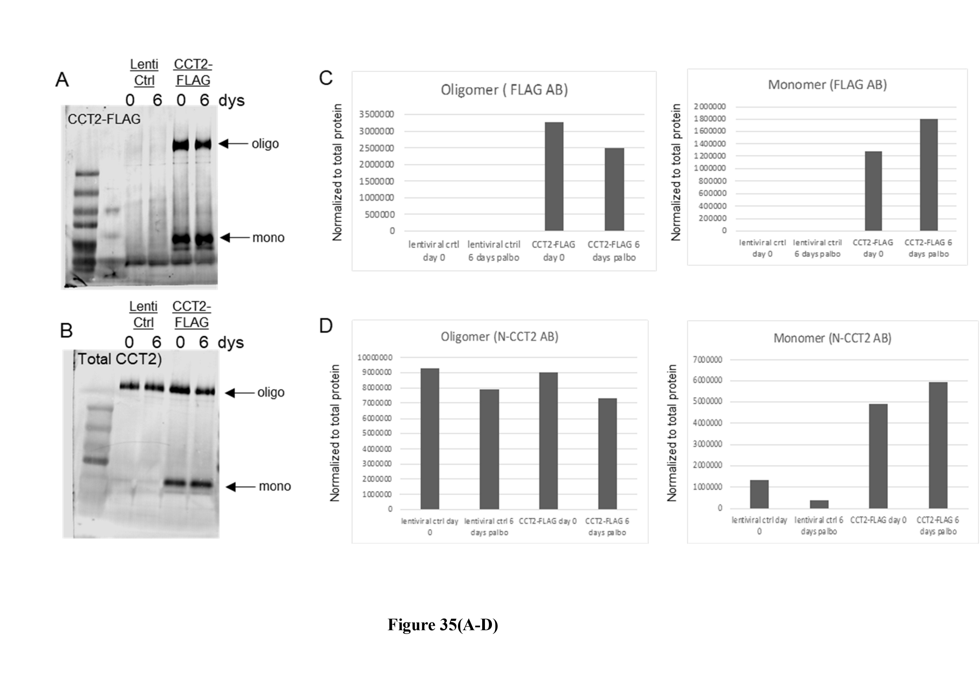

48. Figure 35(A-D). Monomeric CCT2 protein increases after palbociclib treatment. Figure 35(A-B) Protein lysates from T47D (shown) and MCF-7 (not shown) cells stably expressing CCT2-FLAG or lentiviral control and treated with palbociclib, 1 µM, for 0-6 days (see Figure 14A) were run on a native non-denaturing gel. Membranes were probed for CCT2-FLAG with anti-FLAG antibody (Figure 35A, 35C) or total CCT2 with anti-N-terminal CCT2 antibody (Figure 35B, 35D). CCT2 in the oligomeric complex (> 900 kDa) and as a monomer (~ 60kDa) are indicated by arrows.) Relative protein level of CCT2-containing oligomer and CCT2 monomer in CCT2-FLAG and lentiviral control cells were normalized to total protein. Error bars represent the mean with s.e.m of technical replicates. A representative experiment of two performed is shown.

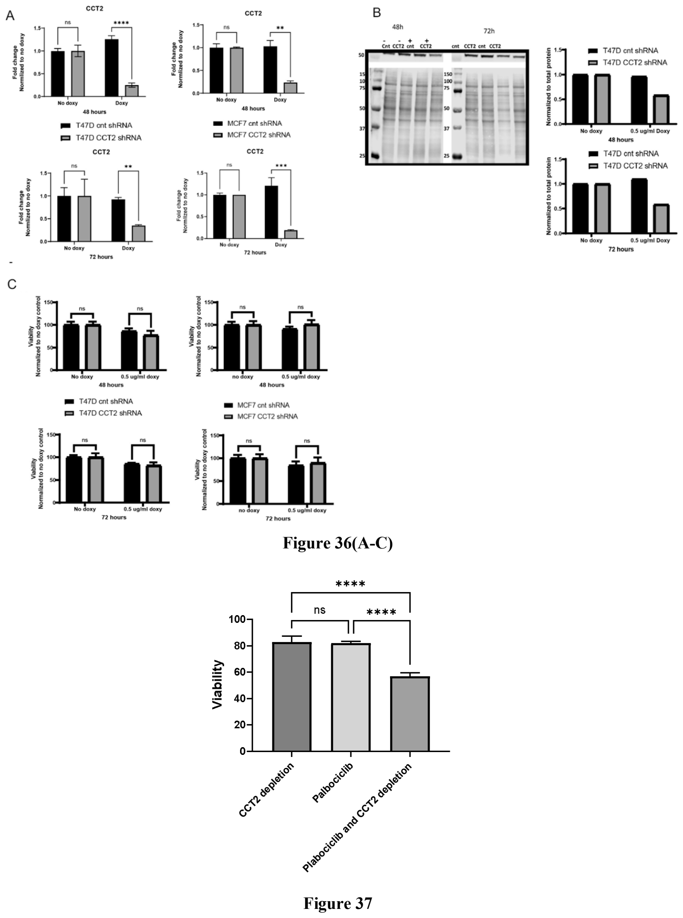

49. Figure 36(A-C). Depletion of CCT2 is achieved in luminal A breast cancer cells. (Figure 36A) CCT2 depletion in T47D and MCF7 was achieved using a doxycycline-inducible shRNA lentiviral system. CCT2 mRNA expression in T47D (shown) and MCF7 (not shown) after 48 and 72 hours 0.5 mg doxycycline addition for induction of shRNA. For RT-qPCR; GAPDH was used as a reference gene. (Figure 36B) CCT2 protein was assessed by western blot for cells from (A). Total protein staining was used for normalization. Data for T47D cells is shown. (Figure 36C) Viability of CCT2 depleted and control T47D and MCF-7 cells was assessed after 48 and 72 hours of shRNA induction by MTT assay. ns, not significant.

50. Figure 37. Combination treatment of CCT2 inhibition with palbociclib is more effective than treatments alone in luminal A breast cancer cells. T47D cells stably expressing doxycycline inducible CCT2 shRNA were treated with either 0.5 ug/ml doxycycline to induce ~50% depletion of CCT2 protein (see Fig. 24A) or 200 nM palbociclib or both for 4 days. Viability was assessed by MTT assay. **** p< 0.00005.

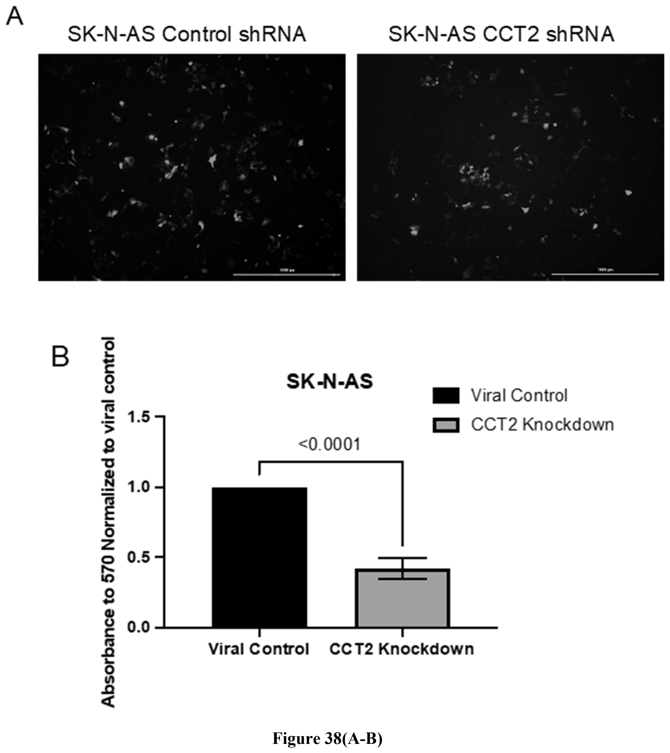

51. Figure 38(A-B). CCT2 depletion in neuroblastoma cells decreases viability. SK-N-AS cells stably expressing doxycycline inducible CCT2 shRNA or control shRNA (viral control) were treated with 0.5 ug/ml doxycycline to induce depletion of CCT2 protein. (Figure 38A) Image of lenti-viral transduced cells. (Figure 38B) Viability was assessed by MTT assay.

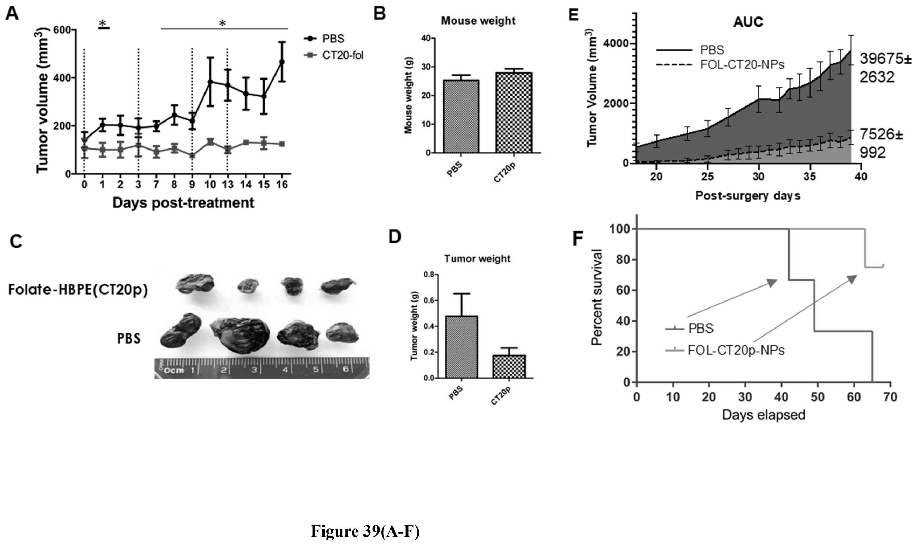

52. Figure 39(A-F). CT20p-nanoparticles reduce tumor growth and extend survival in mice. Figure 39(A-D) Growth curves of LNCaP prostate tumors implanted in male nude mice (n=4) treated intravenously (IV) 4 times (hatched lines) with 100 µl of CT20p-nanoparticles (1 mg/kg/dose) or PBS. (Figure 39B) Average weight of the PBS-treated and CT20p-treated treated mice at the end of the experiment. (Figure 39C) Tumor size comparison after necropsy. (Figure 39D) Average weight of the PBS-treated and CT20p-treated tumors at the end of the experiment. Figure 39(E-F) MDA-MB-231 triple negative breast cancer cells were orthotopically implanted in mammary pad of female nude mice (n=4). Mice were treated three times IV with 100 µl of CT20p-nanoparticles (1 mg/kg/dose) (orange) or PBS (pink). Tumor growth (Figure 39E) was reduced, and survival (Figure 39F) was extended. AUC, area under the curve.

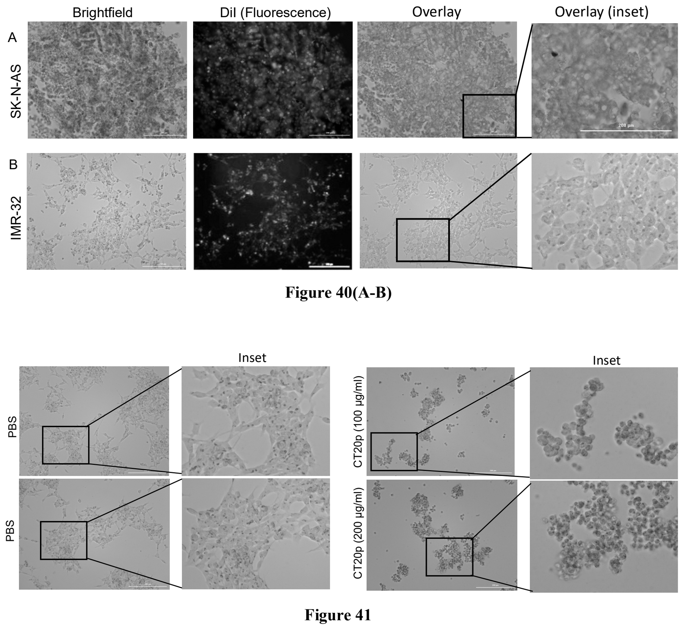

53. Figure 40(A-B). Neuroblastoma cells take up dye-loaded polymeric nanoparticles. SK-N-AS (Figure 40A) and IMR-32 (Figure 40B) cells were treated with 3 µg of DiI dye-loaded nanoparticles for 24 hours and uptake assessed using the Cytation 5 multi-model plate reader. Inset shows digitally magnified view of combined brightfield and fluorescence overlay.

54. Figure 41. CT20p-nanoparticles kill neuroblastoma cells. IMR-32 cells were treated with PBS (vehicle control), and CT20p-nanoparticles at 100 µg/ml and 200 µg/ml doses for 24 hours and cells imaged using the Cytation 5 multi-model imaging reader. Inset was digitally magnified.

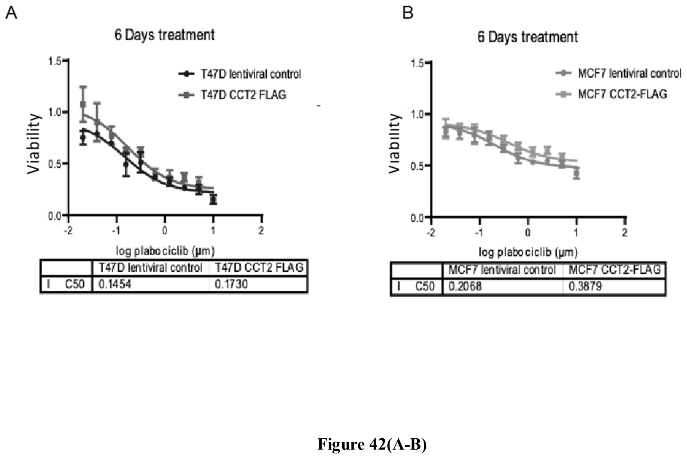

55. Figure 42(A-B). CCT2 overexpression reduces luminal A breast cancer cell sensitivity to CDK4/6 inhibitor, Palbociclib. Viability was determined using standard MTT assay for cells exposed to increasing concentration of Palbociclib for 6 days. CCT2-FLAG expressing cells had higher IC50 in T47D (Figure 42A) and MCF7 (Figure 42B) compared to lentiviral control. Model Fitting: [Inhibitor] vs. response (three parameters) was used to plot dose-response curve using GraphPad 9. Experiments were performed in quadruplicate.

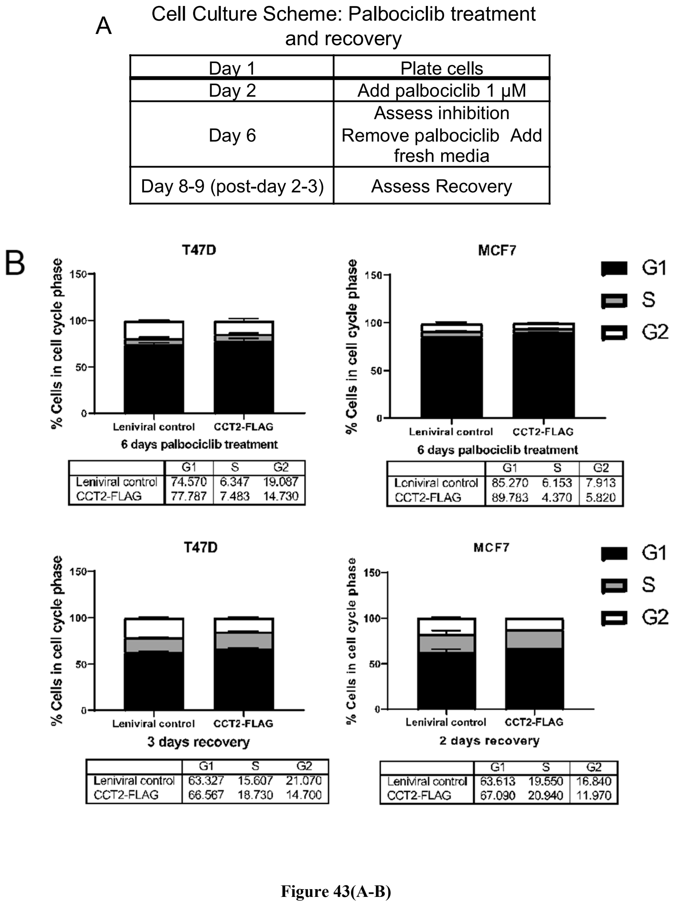

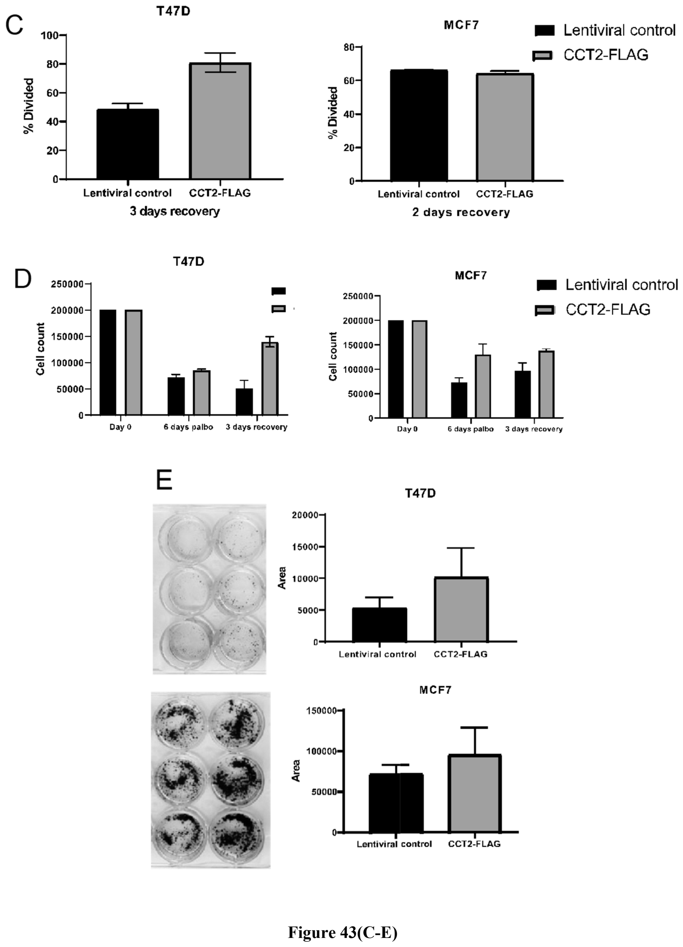

56. Figure 43(A-E) CCT2 promotes cell cycle progression and proliferation in the recovery phase post-Palbociclib treatment. (Figure 43A) Timeline for palbociclib treatment and recovery phase is shown. (Figure 43B) Cell cycle analysis was performed by intracellular PI staining after 6 days of Palbociclib treatment and 2-3 days of recovery post-Palbociclib treatment. CCT2-FLAG expressing and lentiviral control T47D and MCF7 cells were used. (Figure 43C) Proliferation assay using ViaFluor 405 dilution dye to track cell division over time in cells (from above) in recovery phase post-Palbociclib treatment. (Figure 43D) Cell count for cells (from above) in recovery phase post-Palbociclib treatment. Counts acquired by flow cytometry (Cytoflex S). (Figure 43E) Colony-forming assay was performed for 10 days during the recovery phase post-Palbociclib treatment. Cells were stained with crystal violet. Experiments were performed in triplicates.

### DETAILED DESCRIPTION

57. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### Definitions

58. Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

59. As used in the specification and claims, the singular form "a," "an," and "the" include plural references unless the context clearly dictates otherwise. For example, the term "a cell" includes a plurality of cells, including mixtures thereof.

60. The term "about" as used herein when referring to a measurable value such as an amount, a percentage, and the like, is meant to encompass variations of +20%, +10%, ±5%, or +1% from the measurable value.

61. "Activate", "activating", and "activation" mean to increase an activity, response, condition, or other biological parameter. This may also include, for example, a 10% increase in the activity, response, "or condition, as compared to the native or control level. Thus, the increase can be a 10, 20, 30, 40, 50, 60, 70, 80, 90, 100%, or any amount of reduction in between as compared to native or control levels.

62. "Administration" to a subject or "administering" includes any route of introducing or delivering to a subject an agent. Administration can be carried out by any suitable route, including intravenous, intraperitoneal, and the like. Administration can be carried out by any suitable route, including oral, topical, intravenous, subcutaneous, transcutaneous, transdermal, intramuscular, intra-joint, parenteral, intra-arteriole, intradermal, intraventricular, intracranial, intraperitoneal, intralesional, intranasal, rectal, vaginal, by inhalation, via an implanted reservoir, or via a transdermal patch, and the like. Administration includes self-administration and the administration by another.

63. The term "antibodies" is used herein in a broad sense and includes both polyclonal 5 and monoclonal antibodies. As used herein, the term "antibody" encompasses, but is not limited to, whole immunoglobulin (i.e., an intact antibody) of any class. In addition to intact immunoglobulin molecules, also included in the term "antibodies" are fragments or polymers of those immunoglobulin molecules, and human or humanized versions of immunoglobulin molecules or fragments thereof. It should be understood that the "antibody" can be monoclonal antibodies, polyclonal antibodies, chimeric antibodies, bi-specific antibodies (diabody), or tri-specific antibody (triabody).

64. The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity.

65. The disclosed monoclonal antibodies can be made using any procedure which produces mono clonal antibodies. For example, disclosed monoclonal antibodies can be prepared using hybridoma methods, such as those described by Kohler and Milstein, Nature, 256:495 (1975). In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro.*

66. As used herein, the term "antibody or fragments thereof" encompasses chimeric antibodies and hybrid antibodies, with dual or multiple antigen or epitope specificities, and fragments, such as F(ab')2, Fab', Fab, Fv, scFv, and the like, including hybrid fragments. Thus, fragments of the antibodies that retain the ability to bind their specific antigens are provided. For example, fragments of antibodies which maintain Annexin A2 binding activity are included within the meaning of the term "antibody or fragment thereof." Such antibodies and fragments can be made by techniques known in the art and can be screened for specificity and activity according to the methods set forth in the Examples and in general methods for producing antibodies and screening antibodies for specificity and activity (See Harlow and Lane. Antibodies, A Laboratory Manual. Cold Spring Harbor Publications, New York, (1988)).

67. The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr. Opin. Biotechnol. 3:348-354, 1992).

68. As used here, the terms "beneficial agent" and "active agent" are used interchangeably herein to refer to a chemical compound or composition that has a beneficial biological effect. Beneficial biological effects include both therapeutic effects, i.e., treatment of a disorder or other undesirable physiological condition, and prophylactic effects, i.e., prevention of a disorder or other undesirable physiological condition. The terms also encompass pharmaceutically acceptable, pharmacologically active derivatives of beneficial agents specifically mentioned herein, including, but not limited to, salts, esters, amides, prodrugs, active metabolites, isomers, fragments, analogs, and the like. When the terms "beneficial agent" or "active agent" are used, then, or when a particular agent is specifically identified, it is to be understood that the term includes the agent per se as well as pharmaceutically acceptable, pharmacologically active salts, esters, amides, prodrugs, conjugates, active metabolites, isomers, fragments, analogs, etc.

69. The term "biocompatible" generally refers to a material and any metabolites or degradation products thereof that are generally non-toxic to the recipient and do not cause significant adverse effects to the subject.

70. The term "biological sample" as used herein means a sample of biological tissue or fluid. Such samples include, but are not limited to, tissue isolated from animals. Biological samples can also include sections of tissues such as biopsy and autopsy samples, frozen sections taken for histologic purposes, blood, plasma, serum, sputum, stool, tears, mucus, hair, and skin. Biological samples also include explants and primary and/or transformed cell cultures derived from patient tissues. A biological sample can be provided by removing a sample of cells from an animal, but can also be accomplished by using previously isolated cells (e.g., isolated by another person, at another time, and/or for another purpose), or by performing the methods as disclosed herein in vivo. Archival tissues, such as those having treatment or outcome history can also be used.

71. As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this invention.

72. "Composition" refers to any agent that has a beneficial biological effect. Beneficial biological effects include both therapeutic effects, e.g., treatment of a disorder or other undesirable physiological condition, and prophylactic effects, e.g., prevention of a disorder or other undesirable physiological condition. The terms also encompass pharmaceutically acceptable, pharmacologically active derivatives of beneficial agents specifically mentioned herein, including, but not limited to, a vector, polynucleotide, cells, salts, esters, amides, proagents, active metabolites, isomers, fragments, analogs, and the like. When the term "composition" is used, then, or when a particular composition is specifically identified, it is to be understood that the term includes the composition per se as well as pharmaceutically acceptable, pharmacologically active vector, polynucleotide, salts, esters, amides, proagents, conjugates, active metabolites, isomers, fragments, analogs, etc.

73. A "control" is an alternative subject or sample used in an experiment for comparison purposes. A control can be "positive" or "negative." The term "reference control" refers to a level in detected in a subject in general or a study population (e.g., healthy control).

74. By the term "effective amount" of a therapeutic agent is meant a nontoxic but sufficient amount of a beneficial agent to provide the desired effect (for example, reduction of tumor size, elimination of tumor, prevention or mitigation of metastasis, reversal of drug resistance, or sensitize a subject to an anti-cancer agent). The amount of beneficial agent that is "effective" will vary from subject to subject, depending on the age and general condition of the subject, the particular beneficial agent or agents, and the like. Thus, it is not always possible to specify an exact "effective amount." However, an appropriate "effective" amount in any subject case may be determined by one of ordinary skill in the art using routine experimentation. Also, as used herein, and unless specifically stated otherwise, an "effective amount" of a beneficial can also refer to an amount covering both therapeutically effective amounts and prophylactically effective amounts. An "effective amount" of a drug necessary to achieve a therapeutic effect may vary according to factors such as the age, sex, and weight of the subject. Dosage regimens can be adjusted to provide the optimum therapeutic response. For example, several divided doses may be administered daily or the dose may be proportionally reduced as indicated by the exigencies of the therapeutic situation.

75. A "decrease" can refer to any change that results in a smaller amount of a symptom, disease, composition, condition, or activity. A substance is also understood to decrease the genetic output of a gene when the genetic output of the gene product with the substance is less relative to the output of the gene product without the substance. Also for example, a decrease can be a change in the symptoms of a disorder such that the symptoms are less than previously observed. A decrease can be any individual, median, or average decrease in a condition, symptom, activity, composition in a statistically significant amount. Thus, the decrease can be a 1, 2, 3, 4, 5, 6, 7, 8, 9, 10, 15, 20, 25, 30, 35, 40, 45, 50, 55, 60, 65, 70, 75, 80, 85, 90, 95, or 100% decrease so long as the decrease is statistically significant.

76. "Inhibit", "inhibiting," and "inhibition" mean to decrease an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

77. "Inhibitors," of expression or of activity are used to refer to inhibitory molecules, respectively, identified using in vitro and in vivo assays for expression or activity of a described target protein, e.g., ligands, antagonists, and their homologs and mimetics. Inhibitors are agents that, e.g., inhibit expression or bind to, partially or totally block stimulation or protease activity, decrease, prevent, delay activation, inactivate, desensitize, or down regulate the activity of the described target protein, e.g., antagonists. Samples or assays comprising described target protein that are treated with a potential inhibitor are compared to control samples without the inhibitor to examine the extent of effect. Control samples are assigned a relative activity value of 100%. Inhibition of a described target protein is achieved when the activity value relative to the control is about 80%, optionally 50% or 25, 10%, 5% or 1%. It should be understood that the CCP inhibitor described herein can be an inhibitor for one or more other factors (e.g., one or more genes, proteins, mRNA) involved in the CCP pathway.

78. The term "nucleic acid" as used herein means a polymer composed of nucleotides, e.g. deoxyribonucleotides (DNA) or ribonucleotides (RNA). The terms "ribonucleic acid" and "RNA" as used herein mean a polymer composed of ribonucleotides. The terms "deoxyribonucleic acid" and "DNA" as used herein mean a polymer composed of deoxyribonucleotides. (Used together with "polynucleotide" and "polypeptide".) 79. "Pharmaceutically acceptable" component can refer to a component that is not biologically or otherwise undesirable, i.e., the component may be incorporated into a pharmaceutical formulation of the invention and administered to a subject as described herein without causing significant undesirable biological effects or interacting in a deleterious manner with any of the other components of the formulation in which it is contained. When used in reference to administration to a human, the term generally implies the component has met the required standards of toxicological and manufacturing testing or that it is included on the Inactive Ingredient Guide prepared by the U.S. Food and Drug Administration.

80. "Pharmaceutically acceptable carrier" (sometimes referred to as a "carrier") means a carrier or excipient that is useful in preparing a pharmaceutical or therapeutic composition that is generally safe and non-toxic, and includes a carrier that is acceptable for veterinary and/or human pharmaceutical or therapeutic use. The terms "carrier" or "pharmaceutically acceptable carrier" can include, but are not limited to, phosphate buffered saline solution, water, emulsions (such as an oil/water or water/oil emulsion) and/or various types of wetting agents.

81. As used herein, the term "carrier" encompasses any excipient, diluent, filler, salt, buffer, stabilizer, solubilizer, lipid, stabilizer, or other material well known in the art for use in pharmaceutical formulations. The choice of a carrier for use in a composition will depend upon the intended route of administration for the composition. The preparation of pharmaceutically acceptable carriers and formulations containing these materials is described in, e.g., Remington's Pharmaceutical Sciences, 21st Edition, ed. University of the Sciences in Philadelphia, Lippincott, Williams & Wilkins, Philadelphia, PA, 2005. Examples of physiologically acceptable carriers include saline, glycerol, DMSO, buffers such as phosphate buffers, citrate buffer, and buffers with other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEENTM (ICI, Inc.; Bridgewater, New Jersey), polyethylene glycol (PEG), and PLURONICSTM (BASF; Florham Park, NJ). To provide for the administration of such dosages for the desired therapeutic treatment, compositions disclosed herein can advantageously comprise between about 0.1% and 99% by weight of the total of one or more of the subject compounds based on the weight of the total composition including carrier or diluent.

82. The term "polynucleotide" refers to a single or double stranded polymer composed of nucleotide monomers.

83. The term "polypeptide" refers to a compound made up of a single chain of D- or L-amino acids or a mixture of D- and L-amino acids joined by peptide bonds.

84. The terms "peptide," "protein," and "polypeptide" are used interchangeably to refer to a natural or synthetic molecule comprising two or more amino acids linked by the carboxyl group of one amino acid to the alpha amino group of another.

85. The term "increased" or "increase" as used herein generally means an increase by a statically significant amount; for the avoidance of any doubt, "increased" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

86. The term "reduced", "reduce", or "reduction as used herein generally means a lowering or decrease of an event or characteristic (e.g., tumor growth) by a statistically significant amount. However, for avoidance of doubt, "reduced" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (i.e. absent level as compared to a reference sample), or any decrease between 10-100% as compared to a reference level.

87. The term "subject" is defined herein to include animals such as mammals, including, but not limited to, primates (e.g., humans), cows, sheep, goats, horses, dogs, cats, rabbits, rats, mice and the like. In some embodiments, the subject is a human. The term "patient" refers to a subject under the treatment of a clinician, e.g., physician.

88. As used herein, the terms "treating" or "treatment" of a subject includes the administration of a drug to a subject with the purpose of curing, healing, alleviating, relieving, altering, remedying, ameliorating, improving, stabilizing or affecting a disease or disorder, or a symptom of a disease or disorder. The terms "treating" and "treatment" can also refer to reduction in severity and/or frequency of symptoms, elimination of symptoms and/or underlying cause, and improvement or remediation of damage. Treatments according to the invention may be applied preventively, prophylactically, pallatively or remedially. Prophylactic treatments are administered to a subject prior to onset (e.g., before obvious signs of cancer), during early onset (e.g., upon initial signs and symptoms of cancer), or after an established development of cancer.

89. By "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed.

90. "Therapeutically effective amount" or "therapeutically effective dose" of a composition (e.g. a composition comprising an agent) refers to an amount that is effective to achieve a desired therapeutic result. In some embodiments, a desired therapeutic result is the control of a cancer. In some embodiments, a desired therapeutic result is the control of metastasis, or a symptom of a cancer. Therapeutically effective amounts of a given therapeutic agent will typically vary with respect to factors such as the type and severity of the disorder or disease being treated and the age, gender, and weight of the subject. The term can also refer to an amount of a therapeutic agent, or a rate of delivery of a therapeutic agent (e.g., amount over time), effective to facilitate a desired therapeutic effect, such as elimination of a cancer or prevention of relapse. The precise desired therapeutic effect will vary according to the condition to be treated, the tolerance of the subject, the agent and/or agent formulation to be administered (e.g., the potency of the therapeutic agent, the concentration of agent in the formulation, and the like), and a variety of other factors that are appreciated by those of ordinary skill in the art. In some instances, a desired biological or medical response is achieved following administration of multiple dosages of the composition to the subject over a period of days, weeks, or years.

91. The term "cancer" as used herein is defined as disease characterized by the rapid and uncontrolled growth of aberrant cells. Cancer cells can spread locally or through the bloodstream and lymphatic system to other parts of the body. Cancer may include different histological types, cell types, and different stages of cancer, such as, for example, primary tumor or metastatic growth. Cancer may include, for example, breast cancer, cholangiocellular carcinoma, colorectal cancer, endometriosis, esophageal cancer, gastric cancer, diffused type gastric cancer, pancreatic cancer, renal carcinoma, soft tissue tumor, testicular cancer, cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma, non-small cell lung cancer (NSCLC), small cell lung cancer (SCLC); Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinorna, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma [pinealoma], glioblastoma, glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian cancer, ovarian carcinoma [serous cystadenocarcinoma, mucinous cystadenocarcinoma, unclassified carcinoma], granulosa-thecal cell tumors, SertoliLeydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma], CML; Skin: melanoma, malignant melanoma, basal cell carcinoma, squamous cell carcinoma, Karposi's sarcoma, moles, dysplastic nevi, lipoma, angioma, dermatofibroma, keloids, psoriasis; and Adrenal glands: neuroblastoma. In some embodiments, the cancer comprises non-small cell lung cancer (NSCLC). In some embodiments, the cancer is resistant to a therapy. In some embodiments, the cancer is not resistant to a therapy.

92. The term "cancer cells" and "tumor cells" are used interchangeably to refer to cells derived from a cancer or a tumor, or from a tumor cell line or a tumor cell culture.

93. The term "primary tumor" refers to a tumor growing at the site of the cancer origin.

94. The term "metastatic tumor" refers to a secondary tumor growing at the site different from the site of the cancer origin.

### Compositions and Methods of Treating Cancers

95. Inhibitors of the cell cycle are effective in the treatment of cancer but their use in many patients is hindered by the development of resistance. How to overcome the limitations of single agent inhibitors is the unmet medical need underlying our research. Chaperonin-Containing TCP1 (CCT), a multi-subunit protein folding complex, is highly expressed in many cancers and interacts with oncoproteins and mutated tumor suppressors. To date, the complex multi-subunit nature of CCT has challenged the development of targeted inhibitors. It is shown herein that overexpressing a single CCT subunit, CCT2, is sufficient to enhance cell cycling in breast cancer cells (e.g., luminal A breast cancer) and that these cells produce larger spheroids in culture and acquire invasive and metastatic-like characteristics. In these cells, expression of CCT2 correlated with MYC and CCND1, indicating that the chaperonin could be a node of intersection for these proliferative signals. Moreover, CCT2 is frequently genomically amplified in cancerous cells and is found in an amplicon associated with other oncogenes. These findings collectively support that the CCT2 subunit could be a potential oncogene whose therapeutic targeting would inhibit deregulated cell cycling factors like CCND1 and MYC and have application for patients that acquire resistance to front line treatments. In one aspect, disclosed herein are methods of treating, inhibiting, decreasing, reducing, ameliorating, and/or preventing a cancer and/or metastasis (such as, for example, sarcoma, glioma, melanoma, lymphoma, or a breast cancer) in a subject in need thereof, comprising administering to the subject a therapeutically effective amount of a chaperonin-containing TCP1 (CCT) inhibitor.

96. The CCT inhibitor described herein can be a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor. In some embodiments, the CCT inhibitor is a CCT2 inhibitor.

97. In some embodiments, the CCT inhibitor comprises a small molecule, an antibody, a peptide, a polypeptide, a small interfering RNA (siRNA), or a short hairpin RNA. In some embodiments, the CCT inhibitor is a gene editing system, including, for example, CRISPR-Cas9.

98. In some embodiments, the CCT inhibitor is a peptide, including, for example, a CT20p peptide. The CT20p peptide is known in the art. See, e.g., U.S. Patent Application Publication No. 20170165318A1, incorporated by reference herein in its entirety. The CT20 peptide may include SEQ ID NO: 1, SEQ ID NO: 2, SEQ ID NO: 3, SEQ ID NO: 4, SEQ ID NO: 5, SEQ ID NO: 6, any variant of SEQ ID NO:1-6 having at least 60% (e.g., at least 70%, 80%, 90%, 95%, or 99%) sequence identity to SEQ ID NO: 1, SEQ ID NO: 2, SEQ ID NO: 3, SEQ ID NO: 4, SEQ ID NO: 5, or SEQ ID NO: 6, or a combination of two or more of the above.

99. In some embodiments, the CT20 peptide of the therapeutic regimen may be delivered via nanoparticles suitable for delivery of the CT20 peptide into a cancer cell. The nanoparticles may also include at least one type of targeting moiety, for example, a ligand for a receptor expressed by cancer cells. In some embodiments, the receptor expressed by cancer cells is an EGF, HER2, or folate receptor. In some embodiments, the CT20 peptide is linked to an internalization domain suitable for delivery of the CT20 peptide into a cancer cell.

100. As used herein, "nanoparticle" may refer to any nanostructure capable of delivering pharmaceutical compounds, nucleic acids, peptides, or proteins. Nanoparticles may be naturally or synthetically derived. In some aspects, "nanoparticles" may include plasma vesicle particles, liposomes, exosomes, protein-based particles, albumin particles, nucleic acid-based particles, natural polymers, synthetic polymers, hydrogels, dendrimers, silicon-based materials, metal-based materials, carbon-based materials, calcium-based materials, or a combination of any of the above. In some embodiments, the nanoparticle described herein are those disclosed in U.S. Patent No. 11,129,868, incorporated by reference herein in its entirety.

101. In an aspect, the nanoparticles are hyperbranched polyester polymeric nanoparticles. In an aspect, the nanoparticles are polymeric nanoparticles. In an aspect, the nanoparticles can comprise a targeting moiety. In an aspect, the targeting moiety can comprise a targeting ligand. In an aspect, the targeting ligand can be for a receptor expressed by cancer cells. In an aspect, the receptor expressed by cancer cells can be an EGF, HER2, or folate receptor. In an aspect, the receptor expressed by cancer cells can be any receptor known to the skilled person to be expressed by cancer cells. In some embodiments, the receptor may be know to be expressed by colon cancer cells, prostate cancer cells, lung cancer cells, liver cancer cells, and/or breast cancer cells.

102. In an aspect, the targeting ligand is a folate compound. In an aspect, the targeting ligand is a glutamate compound. In an aspect, the targeting ligand is a polyglutamated folate compound. In an aspect, the targeting ligand is glutamate azido urea. In an aspect, the targeting ligand is folate azido urea. In an aspect, the targeting ligand is glutamate azido urea. In an aspect, the targeting ligand is a bifunctional glutamate-folate hybridized compound. In an aspect, the targeting ligand is at high density. In an aspect, the targeting ligand is at low density. In an aspect, the targeting ligand is at high valency. In an aspect, the targeting ligand is at low valency. In an aspect, the targeting ligand is a substrate for a solid tumor-specific cell protein.

103. In some examples, the method disclosed herein further comprises administering to the subject a therapeutically effective amount of a cell cycle inhibitor. In some embodiments, the cell cycle inhibitor comprises a CCND1 inhibitor, a CDK2 inhibitor, or a CDK4 inhibitor. In some embodiments, the cell cycle inhibitor is a CDK4 inhibitor. The CDK4 inhibitor includes, for example, palbociclib, ribociclib, or abemaciclib. In some embodiments, the cell cycle inhibitor is selected from the group consisting of lavopiridol, indisulam, AZD5438, SNS-032, bryostatin-1, seliciclib, PD 0332991, and SCH 727965. In some embodiments, the cell cycle inhibitor used herein is selected from the group consisting of flavopiridol, SNS-032, AT7519, dinaciclib, palbociclib, and P276-00. In some embodiments, the cell cycle inhibitor is vincristine, paclitaxel, or CYT997.

104. In some embodiments, the method described herein is effective on treating a breast cancer, including, for examples, luminal A breast cancer, luminal B breast cancer, estrogen receptor(ER)- progesterone receptor(PR)- HER2+ breast cancer, or triple negative breast cancer. 105. In some embodiments, the method described herein is effective to treat a pediatric cancer. In some embodiments, the pediatric cancer is neuroblastoma, clear cell sarcoma of the kidney (CCSK), Wilms tumor, Rhabdoid tumor of the kidney (RTK), rhabdomyosarcoma, or Choroid plexus carcinoma.

106. In some embodiments, cancer cells obtained from the subject have an increased level of one or more tumor biomarkers selected from the group consisting of MYC, MYCN, CDK2, CDK4, CCNE1, CCND1, YAP1, and RB1 relative to a reference control. In some embodiments, the cancer cells obtained from the subject have an increased level of one or more tumor biomarkers selected from the group consisting of MYC (UniProtKB/Swiss-Prot: P01106), MYCN (UniProtKB/Swiss-Prot: P04198), CDK2 (UniProtKB/Swiss-Prot: P24941), CDK4 (UniProtKB/Swiss-Prot: P11802), CCNE1 (UniProtKB/Swiss-Prot: P24864), CCND1 (UniProtKB/Swiss-Prot: P24385), YAP1 (UniProtKB/Swiss-Prot: P46937), and RB1 (UniProtKB/Swiss-Prot: P06400) relative to a reference control. In some embodiments, the reference control is a non-cancerous cell or a cell obtained from a healthy control.

107. Also disclosed herein are methods of treating, inhibiting, decreasing, reducing, ameliorating, and/or preventing a drug-resistant cancer and/or metastasis (such as, for example, sarcoma, glioma, melanoma, lymphoma, a breast cancer, or a pediatric cancer disclosed herein) in a subject in need thereof, comprising administering to the subject a therapeutically effective amount of a chaperonin-containing TCP1 (CCT) inhibitor.

108. In one example, the drug-resistant cancer is resistant to a cell cycle inhibitor. In one example, the drug-resistant cancer is resistant to a CDK4 inhibitor. In one example, the drug-resistance cancer and/or metastasis is resistance to chemotherapy.

109. In some embodiments, the administration of the CCT inhibitor sensitizes the cancer to a cell cycle inhibitor and/or chemotherapy. "Insensitivity" or "resistance" to a drug means, but not limited to, that a drug does not cause growth inhibition and/or death of a cell (e.g., a cancer cell).

110. Accordingly, in some aspects, disclosed herein is a method of sensitizing a subject to a cycle inhibitor and/or chemotherapy, said method comprising administering to the subject a therapeutically effective amount of a CCT inhibitor, wherein the subject is nonresponsive to a cycle inhibitor and/or chemotherapy. For example, if the traditionally recommended dosage of a cell cycle inhibitor or chemotherapy agent can reduce tumor size or tumor cell number in X amount, then combination of a CCT inhibitor and a cell cycle inhibitor or chemotherapy agent can reduce tumor size or tumor cell number in an amount of about 10%, 20%, 50%, 80%, 100%, 2-fold, 4-fold, 10-fold, 50-fold, 100-fold, or 1000-fold or more.

111. The CCT inhibitor described herein can be a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor. In some embodiments, the CCT inhibitor is a CCT2 inhibitor.

112. In some embodiments, the CCT inhibitor comprises a small molecule, an antibody, a peptide, a polypeptide, a small interfering RNA (siRNA), or a short hairpin RNA. In some embodiments, the CCT inhibitor is a gene editing system, including, for example, CRISPR-Cas9.

113. In some embodiments, the CCT inhibitor is a peptide, including, for example, a CT20p peptide. The CT20p peptide is known in the art. See, e.g., U.S. Patent Application Publication No. 20170165318A1, incorporated by reference herein in its entirety.

114. In some examples, the method disclosed herein further comprises administering to the subject a therapeutically effective amount of a cell cycle inhibitor. In some embodiments, the cell cycle inhibitor comprises a CCND1 inhibitor, a CDK2 inhibitor, or a CDK4 inhibitor. In some embodiments, the cell cycle inhibitor is a CDK4 inhibitor. The CDK4 inhibitor includes, for example, palbociclib, ribociclib, or abemaciclib.

115. As the timing of onset of a cancer can often not be predicted, it should be understood the disclosed methods of treating, preventing, reducing, and/or inhibiting a cancer can be performed any time prior to the onset of the cancer or after the onset of the cancer. In one aspect, the disclosed methods can be employed 30, 29, 28, 27, 26, 25, 24, 23, 22, 21, 20, 19, 18, 17, 16, 15, 14, 13, 12, 11, 10, 9, 8, 7, 6, 5, 4, 3, 2 years, 22, 21, 20, 19, 18, 17, 16, 15, 14, 13, 12, 11, 10, 9, 8, 7, 6, 5, 4, 3, 2 months, 30, 29, 28, 27, 26, 25, 24, 23, 22, 21, 20, 19, 18, 17, 16, 15, 14, 13, 12, 11, 10, 9, 8, 7, 6, 5, 4, 3 days, 60, 48, 36, 30, 24, 18, 15, 12, 10, 9, 8, 7, 6, 5, 4, 3, 2 hours, 60, 45, 30, 15, 10, 9, 8, 7, 6, 5, 4, 3, 2, or 1 minute prior to the onset of the cancer; or 1, 2, 3, 4, 5, 6, 7, 8, 9, 10, 15, 20, 25, 30, 35, 40, 45, 50, 55, 60, 75, 90, 105, 120 minutes, 3, 4, 5, 6, 7, 8, 9, 10, 11, 12, 15, 18, 24, 30, 36, 48, 60 hours, 3, 4, 5, 6, 7, 8, 9, 10, 11, 12, 13, 14, 15, 16, 17, 18, 19, 20, 21, 22, 23, 24, 25, 26, 27, 28, 29, 30, 45, 60, 90 or more days, 4, 5, 6, 7, 8, 9, 10, 11, 12 months, 2, 3, 4, 5, 6, 7, 8, 9, 10, 15, 20, 25, 30 or more years after the onset of the cancer.

116. In one aspect, it is understood and herein contemplated that successful treatment of a cancer in a subject is important and doing so may include the administration of additional treatments. Thus, the disclosed treatments using CCT inhibitors disclosed herein can further include any anti-cancer therapy known in the art including, but not limited to Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar , (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride , EPOCH, Erbitux (Cetuximab), Eribulin Mesylate, Erivedge (Vismodegib), Erlotinib Hydrochloride, Erwinaze (Asparaginase Erwinia chrysanthemi) , Ethyol (Amifostine), Etopophos (Etoposide Phosphate), Etoposide, Etoposide Phosphate, Evacet (Doxorubicin Hydrochloride Liposome), Everolimus, Evista , (Raloxifene Hydrochloride), Evomela (Melphalan Hydrochloride), Exemestane, 5-FU (Fluorouracil Injection), 5-FU (Fluorouracil-Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride) , Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride, Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate). Where an EGFR splice variant isoform is not detected, the treatment methods can include or further include checkpoint inhibitors include, but are not limited to antibodies that block PD-1 (Nivolumab (BMS-936558 or MDX1106), CT-011, MK-3475), PD-L1 (MDX-1105 (BMS-936559), MPDL3280A, or MSB0010718C), PD-L2 (rHIgM12B7), CTLA-4 (Ipilimumab (MDX-010), Tremelimumab (CP-675,206)), IDO, B7-H3 (MGA271), B7-H4, TIM3, LAG-3 (BMS-986016).

117. As noted throughout this application, it is understood and herein contemplated that the methods and CCT inhibitors disclosed herein alone or in combination with adoptive immunotherapies (such as, for example, CAR T cell, CAR NK cell, TIL, and MIL immunotherapies), a cell cycle inhibitors (such as, for example, a CCND1 inhibitor, a CDK2 inhibitor, and/or a CDK4 inhibitor), or any other anti-cancer therapy disclosed herein can be used to treat, inhibit, reduce, ameliorate, and/or prevent any disease where uncontrolled cellular proliferation occurs such as cancers (including, but not limited to such as, for example, sarcoma, glioma, melanoma, lymphoma, or a breast cancer). A representative but non-limiting list of cancers that the disclosed compositions can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, lung cancers such as small cell lung cancer and non-small cell lung cancer, neuroblastoma/glioblastoma, ovarian cancer, skin cancer, liver cancer, melanoma, squamous cell carcinomas of the mouth, throat, larynx, and lung, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon cancer, rectal cancer, prostatic cancer, or pancreatic cancer. In some embodiments, the method described herein is effective on treating a breast cancer, including, for examples, luminal A breast cancer, luminal B breast cancer, estrogen receptor(ER)- progesterone receptor(PR)- HER2+ breast cancer, or triple negative breast cancer.

### Diagnosis of Cancers

118. Expression of CCT (e.g., CCT2) causes these cancers to acquire invasive and metastatic like behaviors and begin to express other cancer genes that are associated with cancer progression like MYC. The outcomes are using CCT2 as a biomarker for cancer to monitor patient outcomes to treatments and predict development of drug resistance.

119. Accordingly, also disclosed herein is a method of diagnosing a subject as having a cancer, comprising a) quantifying a level of a chaperonin-containing TCP1 (CCT) relative to a reference control; b) determining the subject as having the cancer when the level of CCT is higher (for example, at least about 1%, 5%, 10%, 15%, 20 %, 25%, 30%, 35%, 40%, 45%, 50%, 55%, 60%, 65%, 70%, 75%, 80%, 85%, 90%, 95%, or 100% higher) than the reference control; and c) determining the subject as not having the cancer when the level of CCT is lower (1%, 5%, 10%, 15%, 20 %, 25%, 30%, 35%, 40%, 45%, 50%, 55%, 60%, 65%, 70%, 75%, 80%, 85%, 90%, 95%, or 100% lower) than the reference control.

120. In some embodiments, the method further comprises administering to the subject a therapeutically effective amount of a CCT inhibitor. In some embodiments, the administration of the CCT inhibitor sensitizes the cancer to a cell cycle inhibitor and/or chemotherapy.

121. Accordingly, in some aspects, disclosed herein is a method of sensitizing a subject's responsive to a cycle inhibitor and/or chemotherapy, said method comprising administering to the subject a therapeutically effective amount of a CCT inhibitor, wherein the subject is nonresponsive to a cycle inhibitor and/or chemotherapy.

122. The CCT inhibitor described herein can be a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor. In some embodiments, the CCT inhibitor is a CCT2 inhibitor.

123. In some embodiments, the CCT inhibitor comprises a small molecule, an antibody, a peptide, a polypeptide, a small interfering RNA (siRNA), or a short hairpin RNA. In some embodiments, the CCT inhibitor is a gene editing system, including, for example, CRISPR-Cas9.

124. In some embodiments, the CCT inhibitor is a peptide, including, for example, a CT20p peptide. The CT20p peptide is known in the art. See, e.g., U.S. Patent Application Publication No. 20170165318A1, incorporated by reference herein in its entirety.

125. The CT20 peptide may include SEQ ID NO: 1, SEQ ID NO: 2, SEQ ID NO: 3, SEQ ID NO: 4, SEQ ID NO: 5, SEQ ID NO: 6, any variant of SEQ ID NO:1-6 having at least 60% (e.g., at least 70%, 80%, 90%, 95%, or 99%) sequence identity to SEQ ID NO: 1, SEQ ID NO: 2, SEQ ID NO: 3, SEQ ID NO: 4, SEQ ID NO: 5, or SEQ ID NO: 6, or a combination of two or more of the above.

126. In some embodiments, the CT20 peptide of the therapeutic regimen may be delivered via nanoparticles suitable for delivery of the CT20 peptide into a cancer cell. The nanoparticles may also include at least one type of targeting moiety, for example, a ligand for a receptor expressed by cancer cells. In some embodiments, the receptor expressed by cancer cells is an EGF, HER2, or folate receptor. In some embodiments, the CT20 peptide is linked to an internalization domain suitable for delivery of the CT20 peptide into a cancer cell.

127. As used herein, "nanoparticle" may refer to any nanostructure capable of delivering pharmaceutical compounds, nucleic acids, peptides, or proteins. Nanoparticles may be naturally or synthetically derived. In some aspects, "nanoparticles" may include plasma vesicle particles, liposomes, exosomes, protein-based particles, albumin particles, nucleic acid-based particles, natural polymers, synthetic polymers, hydrogels, dendrimers, silicon-based materials, metal-based materials, carbon-based materials, calcium-based materials, or a combination of any of the above. In some embodiments, the nanoparticle described herein are those disclosed in U.S. Patent No. 11,129,868, incorporated by reference herein in its entirety.

128. In an aspect, the nanoparticles are hyperbranched polyester polymeric nanoparticles. In an aspect, the nanoparticles are polymeric nanoparticles. In an aspect, the nanoparticles can comprise a targeting moiety. In an aspect, the targeting moiety can comprise a targeting ligand. In an aspect, the targeting ligand can be for a receptor expressed by cancer cells. In an aspect, the receptor expressed by cancer cells can be an EGF, HER2, or folate receptor. In an aspect, the receptor expressed by cancer cells can be any receptor known to the skilled person to be expressed by cancer cells. In some embodiments, the receptor may be know to be expressed by colon cancer cells, prostate cancer cells, lung cancer cells, liver cancer cells, and/or breast cancer cells.

129. In an aspect, the targeting ligand is a folate compound. In an aspect, the targeting ligand is a glutamate compound. In an aspect, the targeting ligand is a polyglutamated folate compound. In an aspect, the targeting ligand is glutamate azido urea. In an aspect, the targeting ligand is folate azido urea. In an aspect, the targeting ligand is glutamate azido urea. In an aspect, the targeting ligand is a bifunctional glutamate-folate hybridized compound. In an aspect, the targeting ligand is at high density. In an aspect, the targeting ligand is at low density. In an aspect, the targeting ligand is at high valency. In an aspect, the targeting ligand is at low valency. In an aspect, the targeting ligand is a substrate for a solid tumor-specific cell protein.

130. In some examples, the method disclosed herein further comprises administering to the subject a therapeutically effective amount of a cell cycle inhibitor. In some embodiments, the cell cycle inhibitor comprises a CCND1 inhibitor, a CDK2 inhibitor, or a CDK4 inhibitor. In some embodiments, the cell cycle inhibitor is a CDK4 inhibitor. The CDK4 inhibitor includes, for example, palbociclib, ribociclib, or abemaciclib.

131. In some embodiments, the subject is an adult. In some embodiments, the subject is a child.

132. In some embodiments, the cancer is a metastatic cancer. In some embodiments, the cancer is sarcoma, glioma, melanoma, lymphoma, or a breast cancer.

133. In some embodiments, the cancer is a pediatric cancer. In some embodiments, the pediatric cancer is neuroblastoma, clear cell sarcoma of the kidney (CCSK), Wilms tumor, Rhabdoid tumor of the kidney (RTK), rhabdomyosarcoma, or Choroid plexus carcinoma. In some embodiments, cancer cells obtained from the subject have an increased level of one or more tumor biomarkers selected from the group consisting of MYC, MYCN, CDK2, CDK4, CCNE1, CCND1, YAP1, and RB1 relative to a reference control.

134. In some embodiments, the method described herein is effective on treating a breast cancer, including, for examples, luminal A breast cancer, luminal B breast cancer, estrogen receptor(ER)- progesterone receptor(PR)- HER2+ breast cancer, or triple negative breast cancer.

Particular embodiments of the invention include:
(i). A method of treating a cancer in a subject in need thereof, comprising administering to the subject a therapeutically effective amount of a chaperonin-containing TCP1 (CCT) inhibitor.
(ii). The method of embodiment (i), wherein the CCT inhibitor is a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor.
(iii). The method of embodiment (i) or (ii), wherein the CCT inhibitor is a CCT2 inhibitor.
(iv). The method of embodiment (iii), wherein the CCT inhibitor comprises a small molecule, an antibody, a peptide, a polypeptide, a small interfering RNA (siRNA), or a short hairpin RNA.
(v). The method of embodiment (iv), wherein the peptide is a CT20p peptide.
(vi). The method of embodiment (v), wherein the CT20 peptide comprises an amino acid sequence according to any one of SEQ ID NOs: 1 to 6.
(vii). The method of any one of embodiments (i)-(vi), further comprising administering to the subject a therapeutically effective amount of a cell cycle inhibitor.
(viii). The method of embodiment (vii), wherein the cell cycle inhibitor comprises a CCND1 inhibitor, a CDK2 inhibitor, or a CDK4 inhibitor.
(ix). The method of embodiment (viii), wherein the CDK4 inhibitor comprises palbociclib, ribociclib, or abemaciclib.
(x). The method of any one of embodiments (i)-(ix), wherein the cancer is a metastatic cancer.
(xi). The method of any one of embodiments (i)-(x), wherein the cancer is sarcoma, glioma, melanoma, lymphoma, or a breast cancer.
(xii). The method of embodiment (xi), wherein the breast cancer is luminal A breast cancer, luminal B breast cancer, estrogen receptor(ER)- progesterone receptor(PR)- HER2+ breast cancer, or triple negative breast cancer.
(xiii). The method of embodiments (i)-(xii), wherein the cancer is a pediatric cancer.
(xiv). The method of embodiment (xiii), wherein the pediatric cancer is neuroblastoma, clear cell sarcoma of the kidney (CCSK), Wilms tumor, Rhabdoid tumor of the kidney (RTK), rhabdomyosarcoma, or Choroid plexus carcinoma.
(xv). The method of any one of embodiments (i)-(xiv), wherein the cancer cells obtained from the subject has an increased level of one or more tumor biomarkers selected from the group consisting of MYC, MYCN, CDK2, CDK4, CCNE1, CCND1, YAP1, and RB1 relative to a reference control.
(xvi). A method of treating a drug-resistant cancer in a subject in need thereof, comprising administering to the subject a therapeutically effective amount of a chaperonin-containing TCP1 (CCT) inhibitor.
(xvii). The method of embodiment (xvi), wherein the CCT inhibitor is a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor.
(xviii). The method of embodiment (xvi) or (xvii), wherein the CCT inhibitor is a CCT2 inhibitor.
(xix). The method of embodiment (xviii), wherein the CCT inhibitor comprises a small molecule, an antibody, a peptide, a polypeptide, a small interfering RNA (siRNA), or a short hairpin RNA.
(xx). The method of embodiment (xix), wherein the peptide is a CT20p peptide.
(xxi). The method of embodiment (xx), wherein the CT20 peptide comprises an amino acid sequence according to any one of SEQ ID NOs: 1 to 6.
(xxii). The method of any one of embodiments (xvi)-(xxi), wherein the drug-resistant cancer is resistant to a cell cycle inhibitor.
(xxiii). The method of any one of embodiments (xvi)-(xxii), further comprising administering to the subject a therapeutically effective amount of a cell cycle inhibitor.
(xxiv). The method of embodiment (xxii) or (xxiii), wherein the cell cycle inhibitor comprises a CCND1 inhibitor, a CDK2 inhibitor, or a CDK4 inhibitor.
(xxv). The method of embodiment (xxiv), wherein the CDK4 inhibitor comprises palbociclib, ribociclib, or abemaciclib.
(xxvi). The method of any one of embodiments (xvi)-(xxv), wherein the cancer is a metastatic cancer.
(xxvii). The method of any one of embodiments (xvi)-(xxvi), wherein the cancer is sarcoma, glioma, melanoma, lymphoma, or a breast cancer.
(xxviii). The method of embodiment (xxvii), wherein the breast cancer is luminal A breast cancer, luminal B breast cancer, estrogen receptor(ER)- progesterone receptor(PR)- HER2+ breast cancer, or triple negative breast cancer.
(xxix). The method of embodiments (xvi)-(xxviii), wherein the cancer is a pediatric cancer.
(xxx). The method of embodiment (xxix), wherein the pediatric cancer is neuroblastoma, clear cell sarcoma of the kidney (CCSK), Wilms tumor, Rhabdoid tumor of the kidney (RTK), rhabdomyosarcoma, or Choroid plexus carcinoma.
(xxxi). The method of any one of embodiments (xvi)-(xxx), wherein cancer cells obtained from the subject have an increased level of one or more tumor biomarkers selected from the group consisting of MYC, MYCN, CDK2, CDK4, CCNE1, CCND1, YAP1, and RB1 relative to a reference control.
(xxxii). A method of diagnosing a subject as having a cancer, comprising
   a) quantifying a level of a chaperonin-containing TCP1 (CCT) relative to a reference control;
   b) determining the subject as having the cancer when the level of CCT is higher than the reference control; and
   c) determining the subject as not having the cancer when the level of CCT is lower than the reference control.
(xxxiii). The method of embodiment (xxxii), further comprising administering to the subject a therapeutically effective amount of a CCT inhibitor.
(xxxiv). The method of embodiment (xxxiii), wherein the CCT inhibitor is a CCT1 inhibitor, a CCT2 inhibitor, a CCT3 inhibitor, a CCT4 inhibitor, a CCT5 inhibitor, a CCT6 inhibitor, a CCT7 inhibitor, or a CCT8 inhibitor.
(xxxv). The method of embodiment (xxxiii) or (xxxiv), wherein the CCT inhibitor is a CCT2 inhibitor.
(xxxvi). The method of embodiment (xxxv), wherein the CCT inhibitor comprises a small molecule, an antibody, a peptide, a polypeptide, a small interfering RNA (siRNA), or a short hairpin RNA.
(xxxvii). The method of embodiment (xxxvi), wherein the peptide is a CT20p peptide.
(xxxviii). The method of any one of embodiments (xxxii)-(xxxvii), further comprising administering to the subject a therapeutically effective amount of a cell cycle inhibitor.
(xxxix). The method of embodiment (xxxviii), wherein the cell cycle inhibitor comprises a CCND1 inhibitor, a CDK2 inhibitor, or a CDK4 inhibitor.
(xl). The method of embodiment (xxxix), wherein the CDK4 inhibitor comprises palbociclib, ribociclib, or abemaciclib.
(xli). The method of any one of embodiments (xxxii)-(xl), wherein the cancer is a metastatic cancer.
(xlii). The method of any one of embodiments (xxxii)-(xli), wherein the cancer is sarcoma, glioma, melanoma, lymphoma, or a breast cancer.
(xliii). The method of embodiment (xlii), wherein the breast cancer is luminal A breast cancer, luminal B breast cancer, estrogen receptor(ER)- progesterone receptor(PR)- HER2+ breast cancer, or triple negative breast cancer.
(xliv). The method of embodiments (xvi)-(xxviii), wherein the cancer is a pediatric cancer.
(xlv). The method of embodiment (xxix), wherein the pediatric cancer is neuroblastoma, clear cell sarcoma of the kidney (CCSK), Wilms tumor, Rhabdoid tumor of the kidney (RTK), rhabdomyosarcoma, or Choroid plexus carcinoma.
(xlvi). The method of any one of embodiments (xvi)-(xxx), wherein cancer cells obtained from the subject have an increased level of one or more tumor biomarkers selected from the group consisting of MYC, MYCN, CDK2, CDK4, CCNE1, CCND1, YAP1, and RB1 relative to a reference control.

### EXAMPLES

135. Unless defined otherwise, all technical and scientific terms used herein have the same meanings as commonly understood by one of skill in the art to which the disclosed invention belongs. Publications cited herein and the materials for which they are cited are specifically incorporated by reference.

136. Those skilled in the art will recognize, or be able to ascertain using no more than routine experimentation, many equivalents to the specific embodiments of the invention described herein. While the invention has been described with reference to particular embodiments and implementations, it will be understood that various changes and additional variations may be made and equivalents may be substituted for elements thereof without departing from the scope of the invention or the inventive concept thereof. In addition, many modifications may be made to adapt a particular situation or device to the teachings of the invention without departing from the essential scope thereof. Such equivalents are intended to be encompassed by the following claims. It is intended that the invention not be limited to the particular implementations disclosed herein, but that the invention will include all implementations falling within the scope of the appended claims.

### Example 1: Introduction.

137. Genetic alterations support malignant transformation that results from uncontrolled proliferation, especially those that deregulate cell cycle phases - specifically the Gap 1 (G1) phase to Synthesis (S) phase transition. Therapeutically targeting cancer proliferative pathways is therefore of significant interest. The cell cycle is tightly regulated by cyclin dependent kinases (CDK), cyclins, and inhibitors such as p21 or p27 as well as proteolytic pathways to drive cell progression from the G1 phase through S phase, Gap 2 (G2) phase, then mitosis and cytokinesis. The G1 to S transition serves as a key checkpoint through regulation of the activities of retinoblastoma protein (Rb) and histone deacetylases, which control transcription through E2 factor (E2F). In breast cancer, as with other cancers, deregulation of the G1/S transition results in uncontrolled entry into S, bypassing the checkpoint. Normally, mitotic signals drive the expression of cyclin D, which associates with the G1 kinases, CDK4/6, leading to the phosphorylation of Rb, activation of the transcriptional activity of E2F, and the subsequent generation of the S phase kinase, CDK2/cyclin E. In breast cancer, ER+ in particular, multiple signaling pathways converge to target cyclin D. MYC is also involved in cell proliferation and differentiation, transcriptionally activating cell cycle regulators and repressing cell cycle inhibitors, and is implicated in the development of cancer drug resistance. With that said, treatment options for breast cancer patients depend on the molecular classification of their tumors and the availability of targeted therapeutics. Recent successes with CDK inhibitors, such as those that target CDK4, are encouraging, since these inhibitors block the proliferation of cancer cells. However, there are no drugs in clinical use that target CCND1/cyclin D1 or MYC. Endocrine therapies remain the best option for ER+ patients (luminal cancers), but the development of drug resistance is problematic. Patients can also develop resistance to CDK4 inhibitors, three of which are approved for clinical use. Identifying new targetable oncogenes can advance knowledge on the evolution and progression of cancer and reveal novel treatment approaches that improve the prognosis and long-term survival of cancer patients, especially those that develop resistance to front-line therapies.

138. To advance the discovery of targetable factors for cancer therapy, CCT was focused on. CCT is a type II eukaryotic chaperonin that is composed of two stacked rings consisting of eight distinct subunits (CCT1-8) that form the protein folding chamber. Each CCT subunit assembles at a specific location in relation to other subunits in cis (same) and trans (opposite) positions in the ring. A CCT subunit contains three domains: an equatorial domain that forms the base of the chamber, an intermediate domain that has the ATP binding pocket, a hinge that attaches to the apical domain, and the apical domain itself. The apical domain has multiple hydrophobic areas that bind different substrates; hence substrate binding is not based on the amino acid sequence of a protein but rather its structural features. CCT subunits have different binding affinities to ATP, which has a regulatory role, and as well as different binding affinities for substrates. While the scope and breadth of the CCT interactome are not fully understood, reports suggest that CCT can interact with about 1-15% of the proteome to support cellular processes such as those involved in proliferation, cell cycle progression, and invasion. Cytoskeleton proteins, actin and tubulin, are obligate substrates for CCT. In addition, direct interactions with CCT and cell cycle proteins, transcription factors, and tumor suppressors like PLK1, cdc20, CDH1, Cyclin E, p53, STAT3 and others are reported. Hence, cancer cells can become highly dependent on CCT to provide the functional, folded forms of many oncoproteins and essential factors required for survival and growth.

139. CCT subunits were highly expressed in breast cancer as compared to normal tissue and that their expression increased with patients' tumor stage and metastasis. Of the CCT subunits, it was found that CCT2 expression inversely correlated with the overall survival of breast cancer patients. CCT2 can thus be a novel oncogene and serve as a prognostic biomarker, which supports deeper investigation into the role of the chaperonin complex and, CCT2 in particular, in the process of carcinogenesis. Most of the understanding of the role of CCT2 in cancer is inferred from the activity of the whole CCT folding complex and identified protein-protein interactions. To augment this data, the role of CCT2 in cell cycle progression was specifically investigated using 2D and 3D cultures to investigate cellular and molecular changes directly associated with overexpressing the CCT2 subunit in luminal A cells, the most common subtype of breast cancer. It was found that CCT2 expression drove the proliferation of cancer cells in spheroid cultures as well as in 2D monolayers, endowing cancer cells with growth adaptivity irrespective of anchorage. CCT2 expression also correlated with increased expression of key proliferative factors, such as MYC, especially in spheroid cultures. These findings present CCT2 as a cell cycle regulator and proto-oncogene with prognostic and therapeutic value in breast and other cancers.

### Example 2: Materials and Methods.

140. *Cell lines and generation of CCT2 overexpressing or depleted cells:* Cell lines used were MCF7 (ATCC HTB-22) human ER+ breast cancer cells, T47D (ATCC HTB-133) human ER+ breast cancer cells, and E0771 (CH3 Biosystems) murine TNBC cells. T47D cells were cultured in RPMI-1640 (Corning) supplemented with 10% fetal bovine sera (FBS) (Gemini), 1% penicillin-streptomycin (P/S) (Corning), and 0.2 units/mL human recombinant insulin (Santa Cruz). MCF7 cells were cultured in Eagle's Minimum Essential Medium (EMEM) (ATCC) supplemented with 10% FBS (Gemini), 1% P/S (Corning), and 0.01 mg/mL human recombinant insulin (Santa Cruz). MCF7 and T47D cells were transduced with plasmids for the lentiviral control or CCT2-FLAG as previously described. For selection, cells were maintained with 0.5 µg/mL puromycin dihydrochloride (ThermoFisher) and microscopically observed for GFP expression. E0771 cells were cultured in RPMI-1640 (Corning) supplemented with 10% FBS (Gemini) and 1% P/S (Corning). E0771 were transduced with lentiviral-based inducible small hairpin RNA (shRNA) targeting CCT2 as previously reported. To induce shRNA expression, 0.5 µg/mL doxycycline was added to the media for 24-72 hours.

141. Cells were grown in ultra-low attachment plate (ULA) (Corning) and supplemented with complete growth media as appropriate for each cell line. To observe the formation of multiple spheroids, 24-well flat bottom ULA plates were seeded with 30,000 cells/well. To observe the formation of individual spheroids, 96-well rounded bottom ULA plates were seeded with 10,000 cells/well. Culture day 0 refers to the start of spheroid plating. Spheroids were grown for 8 days on ULA plates and assayed at different time points: days 3, 5, and 8. Brightfield images and overlay GFP images were captured using the Cytation 5 Cell Imaging Multi-Mode Reader (BioTek).

142. *2D post 3D cultures (spheroid growth reversal):* To assess spheroid growth reversal, day 8 spheroids grown in 24-well ULA plates were collected and disintegrated either physically by repeat pipetting or chemically using Accumax (Innovative Cell Technology), washed once in PBS, resuspended in complete media, and then plated in standard tissue culture T-25 flask with media appropriate to each cell line. Intact day 8 spheroids from 96-well ULA plate were transferred to 48-well tissue culture plates with media appropriate to each cell line. Fluorescent images were captured using the Cytation 5 (BioTek). Bright field microscopy images were captured using the AXIO Observer with the 20X objective (Carl Zeiss AG).

143. *Proliferation and viability assays:* The ViaFluor^{®} 405 SE Cell Proliferation Kit (Biotium) was used to assess proliferation by tracking the number of cell divisions. Cells from day 3 spheroid cultures were stained following manufacturer's protocol, incubated for 48 hours, then collected on day 5 for analysis. As a reference population, a subset of cells from day 3 spheroids were stained and immediately collected for analysis. Samples were analyzed by flow cytometry using the Cytoflex S flow cytometer (Beckman Coulter). The viability of cells was assessed using propidium iodide (PI) (Invitrogen) staining in an exclusion assay. Five µl of (1mg/ml) of PI were added to 200 µl cell suspension of 10⁶ cell/ml concentration. Samples were analyzed by flow cytometry (Cytoflex S). Data analysis was performed using FCS Express 6 software (DeNovo).

144. *Cell cycle analysis:* To assess cell cycle progression, 200,000 cells/well were added to 6-well tissue culture plates and supplemented with complete growth media. Cells were cultured overnight. To induce growth arrest, cells were washed with and then supplemented with serum-free media and cultured for 24 hours. Initiation of growth was synchronized by the addition of complete growth media with 10% FBS and cells assessed after 24- and 48-hours. Samples were collected for PI intracellular staining as follows. Briefly, equal volumes of detergent buffer and PI (Invitrogen) solution were added to cells at 10⁶ cells/ml followed by the addition of 15 µl RNAase solution (Thermo Scientific) per 1ml total volume. Cells were incubated for 3 hours at room temperature. Samples were analyzed by flow cytometry (Cytoflex S). Data analysis was performed using FCS Express 6 software (DeNovo).
Detergent buffer recipe: 8 gm sodium chloride, 0.4 gm potassium chloride, 0.06 gm KH₂PO₄, 0.09 gm Na₂HPO₄, 0.14 gm CaCl₂, 0.10 gm MgCl₂, 0.10 gm MgSO₄, 5.6 gm HEPES, 2 gm bovine serum albumin (BSA), 4 gm Nonidet P-40 in 1000 ml distilled water.
PI staining solution recipe: 25 gm PI in 500 ml detergent buffer.
RNAase solution recipe: 0.006 g RNAse in 1 ml distilled water

145. *Adhesion assay.* Spheroids were grown on 96 well ULA plates as described above for 8 days. On day 8, the spheroids were transferred to 96-well standard tissue culture plates (Eppendorf) and allowed to attach for 3 hours. After 3 hours, spheroids were washed to remove remaining floating cells and imaged using the Cytation 5 multi-model plate reader (BioTek). The following days, the spheroids were imaged again with the Cytation 5 and then lifted from the plate. Spheroids were then dissociated using ice-cold Accumax (Innovative Cell Technologies) with shaking for 15 min. Once dissociated, the cells were counted by imaging using Cytation 5 reader and Gen 5 software based on green fluorescent protein (GFP) levels.

*146. Immunofluorescence staining for confocal microscopy:* Spheroids grown on 24-well ULA plate were collected and chemically dissociated using Accumax (Innovative Cell Technologies). Dissociated spheroid cells were plated on poly-L-lysine coated 25 mm coverslips (Fisher Scientific), and cells were left for 1 hour to adhere before fixation. Cells were fixed with 4% PFA for 10 min, washed with PBS for 10 min, permeabilized in 0.5% TritonX-100 in PBS for 5 min, blocked with 1% BSA and 0.05% Tween in PBS for 1 hour, stained with and ActinRed^{™} 555 ReadyProbes^{™} Reagent (Rhodamine phalloidin) (Thermofisher) for 1 hour, and mounted with anti-fade DAPI (Invitrogen). Images were acquired with a Zeiss LSM 710 confocal microscope (Carl Zeiss AG) with the 20X objective.

147. *Western blot:* Cell lysis, sodium dodecyl sulfate polyacrylamide gel electrophoresis (SDS-PAGE), total protein staining, gel visualization, and gel band quantification were performed as previously described. Lysates were collected from cells grown in 2D culture flask or 24-well ULA plates. Total protein concentration was determined using te Pierce BCA Protein Assay Kit (Thermo Scientific) following the manufacturer's protocol. Anti-CCT2 (ab109184) and anti-FLAG (ab1162) antibodies were obtained from Abcam, anti-CCT3 (MA5-27872) antibodies were from from Invitrogen, and anti-CCT-beta (MAB10050) antibodies were from Millipore. Note that anti-CCT2 (Abcam) and anti-CCT-beta (Millipore) antibodies target the N-terminal amino acids 1-100 and C-terminal amino acids of human CCT-beta, respectively. Secondary antibodies used were IRDye 800CW and IRDye 680CW (LI-COR).

148. *Quantitative Real Time Polymerase Chain Reaction (RT-qPCR):* Total RNA was extracted from cells using Trizol (Ambion) following the manufacturer's instructions. RNA concentration was determined using the NanoDrop instrument (Nanodrop 8000, ThermoFisher). Approximately 1 µg of RNA was reverse transcribed to cDNA using iScript reverse transcription Supermix for RT-qPCR ( Bio-Rad) according to the manufacturer's instruction. cDNA was diluted to 10 ng/µl. For RT-qPCR, a 20 µl PCR reaction was performed using 5 µl of Fast Syber Green Mastermix (Applied Biosystem), 0.2 forward primer, 0.2 reverse primer, and 2 µl cDNA (10 µg/ml) and RNase free water. PCR reactions were performed in duplicate. GAPDH was used as a reference gene. PCR reactions were performed using the Applied Biosystems QuantStudio 7 Flex Real-Time PCR system of 40 cycles of 95°C for 3 seconds and 62°C for 30 seconds. The melting curve was evaluated for each reaction to verify a single amplification product. Relative mRNA expression was calculated using 2^{-ΔCt} and fold change using 2^{-ΔΔCt} equations. Primers used are shown in Table 1.

149. *Bioinformatics analysis:* Interrogation of The Cancer Genome Atlas (TCGA) database was accomplished using the websource cBioPortal for Cancer Genomics [cbioportal.org] to visualize copy number alteration, mutual exclusivity, and co-expression of genes in different studies. TCGA data were analyzed and graphics were downloaded using the webtool. CCT subunits alterations were analyzed using the Catalog of Somatic Mutations in Cancer (COSMIC). mRNA expression and copy number data from The Cancer Cell Line Encyclopedia (CCLE) databases were downloaded using the Xena browser.

*150. Statistical analysis:* For statistical analysis of protein levels, imaging data, spheroid growth, adhesion, viability and proliferation data, Prism 8 (GraphPad) was used to determine statistical significance. Different groups were tested using Student's t-test or ANOVA as relevant. P-values of less than 0.05 were considered statistically significant. For analysis of gene expression data, we first checked the raw Ct data for batch effect. If it existed, the batch effect was then removed using R package (Limma) prior to downstream gene expression analysis. After batch effect was corrected, the 2^{-ΔΔCt} method was used to calculate and normalize gene expression for each target gene, in which -ΔΔCt was calculated using the formula: - ΔΔCt=average(ΔCt control sample)-ΔCt treated sample, where ΔCt=Ct target gene - Ct housekeeping gene. In all subsequent analysis, the fold change was log2-transformed (2^{-ΔΔCt}), i.e., used -ΔΔCt, to evaluate the effect of different treatments on gene expression of each target gene. Multiple mixed effect linear regression model was used to evaluate expression of each gene in response to the effects of cell line, treatment and time. Time was set as a continuous variable with value of 0 for day 0, 1 for day 3, 2 for day 5 and 3 for day 8. Multi-factor ANOVA analysis was performed to evaluate the effect of individual factors, such as cell line, overexpression of CCT2 and culture type, depending on expression of each target gene. Spearman correlation coefficient was applied to test the gene interaction among MYC, CCND1 and total CCT2. These statistical analyses were performed using Stata MP 15 (StataCorp LLC, 2019) and R packages. All tests were two-tailed with a significance level of α (type I error) <0.05. The p-values were adjusted based on Holm adjustment when multiple tests were conducted.

### Example 3. Co-occurrence of CCT2 genetic alterations with cell cycle gene alterations is suggestive of functional relationships.

151. The co-occurrence of genetic mutations that are functionally related contributes to the process of carcinogenesis. Identifying such patterns can reveal novel cancer initiating pathways and treatment targets. Genetic alterations in cell cycle regulators are common in breast cancer, supporting the uncontrolled proliferation of cancer cells. However, the heterogeneity of tumor types and subtypes, suggests that there is not a single mechanism that exerts a biological function like proliferation but rather different signaling pathways converge that are responsible for the complex dynamics of cancer growth. Identifying possible points of convergence for future therapeutic targeting underlies the present studies. To this end, pan-cancer databases like TCGA were mined for the expression of CCT subunits in all cancers and it was showed that the most common type of copy number alteration of the CCT2 subunit gene was amplification, and that this gene was rarely deleted (Fig. 1A). As comparison, data for CCT3 is included (Fig. 1A). The importance of CCT2 in cancer progression is further emphasized by the fact that cancer patients with genetic alterations in CCT2 had reduced overall and progression free survival (Fig. 1B). In support, it was previously reported that breast cancer patients with CCT2 genetic alterations died up 70 months sooner than patients without alterations. These findings support investigating the relationship between CCT2 and cell cycle gene expression to reveal new pathways for therapeutic intervention. Since the previous studies showed that the CCT2 subunit was essential for breast cancer growth and tumor formation, whether alterations of the CCT2 gene associated with cell cycle gene alterations was first examined. As shown in Table 2, genetic alterations in CCT2 co-occurred with CDK4, CDK2, CCND1, and MYC.

152. As the platform to investigate a role for CCT2 in cell cycle regulation, the T47D and MCF-7 cell lines were chosen to use. Both cell lines are luminal A epithelial breast cancer cells, ER+, and are derived from a metastatic site of pleural effusion. It was previously reported that T47D and MCF7 cells had lower levels of cytosolic CCT2 protein as compared to basal cell lines like MDA-MB-231. Luminal A subtypes are among the most common breast cancer subtypes but also display molecular heterogeneity. Clinically this is reflected by differential treatment outcomes and the development of acquired endocrine therapy resistance, such as due to the overexpression/amplification of CCND1 and CDK4. The Cancer Cell Line Encyclopedia (CCLE) database for global and selected genes alterations indicates that MCF7 and T47D cell lines have variable copy number alteration profiles and mutation rates (Fig.1C). Focusing on the copy number and expression level of cell cycle genes, MCF7 cells have a relatively higher copy number of CCT2 and MYC but lower gene expression relative to T47D cells (Fig. 1D). CCT2 gene expression is thus higher in T47D cells. MCF7 cells also had the same copy number but relatively higher mRNA expression for CCND1 and CDKN1A compared to T47D cells (Fig. 1D). Further, whether these cell lines harbored any genetic alterations in CCT subunits was examined. Using the COSMIC web-tool, it was found that MCF7 and T47D cells had mutations in CCT6B and CCT8 subunits, respectively. In addition, CCT3 was overexpressed in MCF7 cells, but not in T47D cells (Table 3). These data indicate that, while T47D and MCF7 cell lines are representative of the most common forms of breast cancer, these display genetic heterogeneity that is observed in cancerous cells and thus can help reveal the role that CCT2 plays in the regulation of cancer cell growth, especially as cancers spread from local to disseminated disease.

### Example 4. CCT2 overexpression in breast cancer cells promotes the growth of spheroids.

153. To achieve the exogenous expression of CCT2 in T47D and MCF7 cells, a lentiviral system was used to transduce cells with a FLAG-tagged CCT2 construct. As shown in Figure 2A, the transduction of both cell lines was equivalent as indicated by comparable levels of GFP expression from the lentiviral plasmid. CCT2-FLAG overexpression was examined by western blot using antibodies specific for the exogenous CCT2-FLAG protein (anti-FLAG), total CCT2 protein (anti-CCT2 N-terminal specific), and endogenous CCT2 protein (anti-CCT2 C-terminal specific). While both cell lines expressed the CCT2-FLAG protein, T47D cells had increased total CCT2 protein as compared to MCF7 cells (Fig. 2B). This is in part due to the downregulation of endogenous CCT2 in CCT2-FLAG expressing cells (Fig. 2B). In T47D cells, CCT2-FLAG expression increased total CCT2 mRNA (Fig. 2C), which correlated with CCT2 protein expression. In MCF7 cells, CCT2-FLAG mRNA was increased as well; however, this did not correlate with increased total CCT2 protein as compared to lentiviral control, due to differences in post-transcriptional mechanisms. This difference in total CCT2 expression between the cell lines is important as the effects of CCT2-FLAG overexpression upon cell growth, proliferation, and correlation with cell cycle regulators were considered.

154. Since it was observed that overexpressing CCT2-FLAG caused a decrease in endogenous CCT2, whether other endogenous CCT subunits were also decreased was determined. In MCF7 cells expressing CCT2-FLAG, endogenous CCT3 protein and mRNA expression were slightly increased, not decreased, and no statistically significant changes were noted in T47D cells expressing CCT2-FLAG (Fig. 11). It was reported that the endogenous levels of the other CCT subunits (e.g., CCT4, CCT5) were the same or increased in cells expressing CCT2-FLAG. Hence, the decrease in endogenous CCT2 upon expression of CCT2-FLAG was not accompanied by the decrease of other CCT subunits. Moreover, it was found that a major portion of the CCT2-FLAG protein physically associated with the other CCT subunits to form a complex. While this shows that the biological activity of CCT2-FLAG in T47D and MCF7 cells is mediated as part of the CCT protein-folding complex, it does not rule a biological function for the monomeric CCT2 mRNA or protein.

155. 3D culture models better mimic tumor growth dynamics and are a valuable tool to investigate tumor growth mechanisms. Cells grown in 3D culture tend to aggregate and form spheroids. The core of these spheroids is usually hypoxic and necrotic, having less access to growth factors, the middle layers contain quiescent cells, and the outer layers are typically composed of actively proliferating cells in contact with the surrounding media. Utilizing 3D culture conditions, enabled through the use of ULA plates, the impact that CCT2-FLAG overexpression can have on spheroid formation by breast cancer cells was investigated. In flat bottom 24 well ULA plates, T47D and MCF7 cells expressing CCT2-FLAG formed multiple large spheroids as compared to cells expressing a lentiviral control plasmid (Fig. 3A). Morphologically, the spheroids formed by T47D cells expressing CCT2-FLAG attained a sheet-like pattern rather than growing in multiple but individual spheroids as seen with MCF7 cells expressing CCT2-FLAG or the T47D and MCF7 lentiviral controls (Fig. 3A). Spheroid number per well was next evaluated at different days of spheroid formation (days 3, 5, and 8). A statistically significant increase in the number of spheroids formed was seen with T47D cells that overexpressed CCT2-FLAG, and a similar, though not statistically significant trend, was observed with MCF7 CCT2-FLAG expressing cells (Fig. 3B). To measure the size of individual spheroids (by imaging single spheroids), T47D and MCF7 CCT2-FLAG overexpressing and lentiviral control cells were grew in 96-well rounded bottom ULA plates. For T47D and MCF7 CCT2 overexpressing cells, larger spheroids formed that increased with each time point - 3, 5 and 8 days (Fig. 3C). It was confirmed that CCT2-FLAG protein levels were maintained in each cell line through day 5 of spheroid growth, while in lentiviral control cells, endogenous CCT2 protein was downregulated (Figs. 12-13). T47D CCT2-FLAG over-expressing cells maintained higher total CCT2 mRNA that peaked at day 3 of the 3D culture (Fig. 3D). These results demonstrate that increased expression of CCT2 can drive spheroid growth, even when normal cellular processes are decreasing, and that T47D cells, which maintain a higher expression level of total CCT2 than MCF7 cells, display corresponding increased growth of multiple and larger spheroids under 3D culture conditions.

156. To verify that CCT2 is essential for spheroid formation and growth, a CCT2 depletion experiment was performed using a doxycycline (doxy) inducible shRNA. Depletion of CCT2 in breast cancer cells was lethal and can inhibit tumor growth in a syngeneic mouse model of TNBC. TNBC cells were used because, unlike luminal A breast cancer cells, these cells tend to express increased amounts of endogenous CCT2 and are amenable to depletion. In the previous study, using E0771 TNBC cells achieved ~50% depletion of CCT2 after 72 hours of treatment with doxy. Using this same system to deplete CCT2, the E0771 control or CCT2 shRNA cells were plated on 96-well ULA round bottom plates and assessed the growth of individual spheroids as follows. As shown in Figure 4A, E0771 cells (control and CCT2 shRNA) were plated at day 0, and depletion of CCT2 was induced with doxy after day 3 of spheroid growth. Cells were imaged at 24-, 48-, and 72-hours post-doxy treatment. CCT2 depletion interfered with the formation of spheroids as these cells lost their tight interactions and loosely aggregated at the bottom of the well (Fig.4A). As shown in Figure 4B, the experiment was repeated but instead CCT2 was depleted at the start of spheroid culture. When CCT2 depletion was induced on day 0, this prevented cells from forming tight spheroid structures and were loosely aggregated (Fig. 4B). In both experiments, the resulting aggregates formed after CCT2 depletion were easily dispersed by pipetting, indicating that the tight cell-to-cell interactions needed to form spheroids were lost. These finding helps confirm that CCT2 promotes spheroid formation and growth.

### Example 5. CCT2 overexpression supports the transition of cells from 3D spheroid to 2D monolayer cultures.

157. CCT2-FLAG overexpressing cells growing in 3D culture mimic in vivo tumors. Whether these cells can undergo a reversal of 3D growth, re-attaching and expanding in a 2D monolayer was next wanted to determine. This model can mimic metastasis by retaining the malignant characteristics (e.g., invasiveness, stemness) of spheroid cells when these transition to 2D culture. Spheroids from day 8 cultures of T47D and MCF7 cells, lentiviral control and CCT2-FLAG expressing, were collected and transferred to treated tissue culture plates and allowed to re-attach for 3 hours. Cells were washed to remove unattached cells and then imaged and quantitated. More CCT2-FLAG expressing cells were able to re-attach as compared to lentiviral controls, indicating that these cells preserved features that enabled re-establishment of growth in adherent cultures (Fig. 5A). This was most apparent with T47D CCT2-FLAG expressing cells, since these retained cell-to-cell attachments, while also anchoring to a surface. Since actin is an obligate substrate of the CCT complex and has a role in spheroid formation by supporting cell-to-cell and cell-to-substrate interactions along with other cytoskeleton components, the intracellular distribution of F-actin was microscopically examined. T47D cells were chosen for F-actin visualization, since CCT2-FLAG overexpressing cells from this cell line displayed reversal of spheroid growth and re-attachment in monolayer culture (Fig. 5A). F-actin levels were assessed by confocal microscopy using fluorescent phalloidin, which binds F-actin. CCT2-FLAG overexpression in T47D spheroid cells significantly upregulated F-actin levels as compared to lentiviral control cells (Fig. 5B). These cells also had filopodial like cellular extensions (Fig.5B). While MCF7 CCT2-FLAG overexpressing cells also underwent spheroid growth reversal (Fig. 5A), in contrast to T47D cells, these cells did not attach strongly to coated coverslips and only a few cells can be imaged for F-actin by microscopy. Collectively, these results show that CCT2 overexpression supports spheroid growth reversal and increased F-actin levels.

158. To further investigate the effect of CCT2-FLAG expression on spheroid growth reversal, spheroids from T47D cells were examined, CCT2-FLAG overexpressing and lentiviral control, under two experimental conditions for re-growth in 2D monolayers. First, day 8 spheroids were disassociated and placed in standard tissue culture plates. Under these conditions, CCT2-FLAG overexpressing cells gained anchorage-independent growth. As shown in Figure 6A, CCT2-FLAG overexpressing cells, in 2D culture conditions, exhibited superimposed growth, forming a spheroid-like structure that attached to the flask. In contrast, lentiviral control cells recovered from spheroid culture grew in only in a 2D monolayer (Fig. 6A). In the second approach, intact day 8 spheroids (not dissociated) were transferred from T47D and MCF7, CCT2-FLAG overexpressing and lentiviral control, cells to a standard tissue culture plate and imaged at different time points. CCT2-FLAG overexpression enhanced the transition of spheroids from 3D to 2D culture; by day 2 after the transfer, spheroids from CCT2-FLAG overexpressing cells were attached and growing faster than the lentiviral controls, especially the T47D cells (Fig. 6B). These cells maintained CCT2-FLAG protein levels (Figs. S3 and S4) and higher total CCT2 mRNA (especially T47D cells) (Fig.6C). Based on these observations, it is concluded that CCT2 overexpression confers to tumor cells enhanced growth potential and adaptability, even as these cell transition between suspension and adherent culture conditions, indicative of the potential for invasive and metastatic-like behavior.

### Example 6. CCT2 overexpression increases cell cycling in 3D and post-3D cultures

159. Having shown that overexpression of CCT2-FLAG resulted in larger spheroids and promoted spheroid growth reversal, the proliferation of cells in 2D and 3D culture conditions was examined using a dilution dye, ViaFluor^{®} 405 . Cells in spheroid cultures (growing on ULA plates) were treated with the dye on day 3 and collected for analysis on day 5 of spheroid culture. The percent of cells divided during this period was significantly higher for the T47D and MCF7 cells overexpressing CCT2-FLAG as compared to lentiviral control. For T47D cells, 60% of CCT2-FLAG overexpressing cells divided over time compared with 40% of lentiviral control cells (Fig. 7A). For MCF7 cells, about 80% of CCT2-FLAG overexpressing cells divided compared to about 40% of lentiviral control cells (Fig.7B). The viability of these cells was assessed on days 3, 5 and 8 using PI exclusion. Spheroids formed with CCT2-FLAG overexpressing cells had lower viability in later culture days compared to lentiviral control in both cell lines, which was most evident for MCF7 CCT2-FLAG overexpressing cells at days 5 and 8 of spheroid growth (Figs. 7C and 7D; gating shown in Fig. 15). Loss of viability in the T47D CCT2-FLAG overexpressing cells at day 8 can be explained by a bigger necrotic core in these larger spheroids (Fig. 3C). Viability loss in MCF7 CCT2-FLAG overexpressing cells can be due to increased proliferation of cells that failed to thrive in spheroid cultures. In total, these findings provide additional support for CCT2 in promoting the proliferation of cancer cells and growth of spheroids.

160. Based on the data mining showing the co-occurrence of CCT2 with cell cycle genes (Table 2) and the previous finding that overexpression of CCT2-FLAG promoted cell division in 2D monolayer cultures, whether CCT2-FLAG overexpression can enhance cell cycle entry and progression was tested. Cultures of T47D and MCF7, CCT2 overexpressing and lentiviral control, cells were synchronized for growth. After 24 hours of serum deprivation, cell cycle analysis using PI to stain the DNA confirmed that a majority of cells (~70%) were in G1 phase arrest (Fig. 16A-16D). Serum-containing media was introduced to the culture to synchronize growth, and cell cycling was assessed at 24- and 48-hours post-serum addition. Results showed that overexpression of CCT2-FLAG in T47D cells promoted the transition of cells from G1 to S and G2 phases of cell cycle. T47D cells overexpressing CCT2-FLAG had more cells in S and G2 phases at 24- and 48-hours post-serum addition compared to lentiviral control cells (Fig. 8A, Fig. 17A). MCF7 CCT2-FLAG overexpressing and lentiviral control cells had about the same cell cycle distribution post-serum addition (Fig. 8B, Fig. 17B).

161. 2D monolayer cultures were also established from the 3D cultures that underwent spheroid growth reversal - referred to as 2D post 3D. Using these cells, whether CCT2-FLAG overexpression impacted cell cycle progression was tested. The 2D post-3D cells derived from T47D and MCF7 spheroids were synchronized by serum deprivation and analyzed for the effect of CCT2-FLAG overexpression on the cell cycle. Using PI staining for DNA analysis, we confirmed that about 70% of cells were in G1 after 24-hours of serum deprivation (Fig. 16A-16D). T47D CCT2 overexpressing cells had more cells in S and G2 phases after 24- and 48-hours post-serum addition compared to lentiviral control cells (Fig. 8C, Fig. 17C). MCF7 CCT2 overexpressing and lentiviral control cells had comparable cell cycle distribution after serum addition, however these cells were inherently more proliferative (Fig. 8D, Fig. 17D). Collectively, it was found that CCT2 promotes breast cancer cell cycle progression through G1/S, in 2D, spheroid and 2D post 3D cultures; however, this effect can be cell type dependent and may involve different mechanisms depending on the cell's genetic make-up.

### Example 7. CCT2 upregulates expression of MYC and cell cycle genes under different culture conditions

162. To investigate whether CCT2 overexpression increased the expression of genes involved in cell cycling, the gene expression of MYC and key cell cycle regulators were measured by RT-qPCR. GAPDH was used as a reference gene. Gene expression is presented as -ΔΔ Ct relative to the MCF7 lentiviral control reference sample, since these had the lowest CCT2 expression. MCF7 and T47D cell lines had inherent differences in gene expression (Table 4; Fig. 9A). Collectively, T47D spheroids displayed statistically significant higher levels of MYC, CDK2, CDK4, and CCNE1 compared to MCF7 spheroids (Table 4). As spheroid cultures grew, expression for selected genes was downregulated, which is consistent with a general decrease in cellular processes observed with spheroid growth. Peaks of gene expression most commonly occurred on day 3 of spheroid culture, which correlated with peak levels of CCT2 (Fig. 9A). Importantly, CCT2-FLAG overexpression significantly upregulated the expression of the cell cycle regulators, MYC (Fig 9A) and CCND1/cyclin D1 (using T47D lentiviral cells as the reference sample), in T47D cells relative to the lentiviral control cells (Fig. 9B, Table 4).

163. Two different population of cells grown in 2D monolayer cultures were evaluated, the more heterogeneous cells that are adapted to 2D adherent growth conditions and the 2D post 3D cells, that survived the selection process of spheroid growth and transitioned back to monolayer growth (underwent spheroid growth reversal). CCT2-FLAG overexpression upregulated MYC, CCND1, CDK2, and CDKN1A gene expression in cells from 2D cultures (Table 5). Especially significant was the increased expression of MYC and CCND1 in T47D CCT2-FLAG overexpressing cells as compared to lentiviral controls (Fig. 9C, Table 5). In summary, it was found that in CCT2 overexpressing cells (especially T47D cells) gene expression of key regulators of cell proliferation, such as MYC and CCND1, was increased and contributed to the proliferative expansion detected across 2D, 3D and 2D post-3D culture conditions.

### Example 8. CCT2 as an oncogene.

164. The highest gene expression for MYC in the CCT2-FLAG overexpressing T47D cell line was observed on day 3 of spheroid culture (Fig. 9A) and is consistent with total CCT2 mRNA expression (Fig. 9A). Whether there was a statistically significant correlation between CCT2, MYC, and CCND1 were determined in the breast cancer cell lines. Gene expression of CCT2, MYC and CCND1 was assessed from the same batch of 2D and day 3 spheroid cultures of T47D and MCF7 cells, CCT2-FLAG and lentiviral control. Combining the datasets to increase statistical power, we performed a gene correlation analysis and found that total CCT2 expression significantly correlated with the increased expression of MYC and CCND1, with Spearman correlations of 0.864 and 0.824, respectively, and p< 0.05 (Fig. 10A). To determine if the correlation between CCT2, MYC, and CCND1 was clinically relevant, we analyzed TCGA data for CCT2 mRNA co-expression with these genes. Using combined pan-cancer studies, moderately positive correlations of CCT2 mRNA were found with MYC, CDK2, CDK4, CCNE1 but not CCND1 (slight negative correlation) (Fig. 10B). However, a different correlation test using the UCSC Xena database (study: TCGA BRCA) did find a weak but significant correlation between CCT2 and CCND1 (Pearson's correlation 0.095, p-value < 0.001). These findings suggest that CCT2 can be a possible node for the interaction of key cell cycle regulators in the uncontrolled growth of cancer cells. An analysis of the network resulting from compiling data of potential CCT2 interactors (evidence from both physical and genetic interactions, BioGRID) included MYC, CDK2, and cyclin D1 as part of the CCT2 interactome, supporting the idea that CCT2 can be a central point or node for regulation of cancer proliferation pathways (Fig. 10C, Table 6).

165. CCT2 is located on chromosome 12q15 along with other identified oncogenes MDM2, FRS2, YEATS4 and others. CDK4 (12q13) also spans the chromosomal region 12q13-15. Hence CCT2 is part of an amplicon that is associated with cancer development. The highest percent of samples/patients with CCT2 gene amplification or increased gene expression is observed in soft tissue cancers like sarcoma (19% of sarcoma samples/patients have gene alterations in CCT2, TCGA) (Fig. 18A). CCT2 is also genetically altered in invasive breast cancer along with other 12q15 oncogenes, the percent of which varies depending on the study and availability of samples (Fig. 18B, 18C). Along with its role in the CCT protein folding complex, we showed that CCT2 promotes the proliferation of cancer cells that is anchorage-independent, correlates with the expression of other oncogenes like MYC, and thus may fulfill the basic requirements for an oncogene as it undergoes genomic amplification during the mutational processes that drives tumorigenesis.

166. The CCT complex assists in the folding, stability, maturation, or assembly of many proteins essential for cancer cells. However, being a large complex composed of eight subunits, the therapeutic targeting of CCT is challenging. To address this, it is shown that overexpressing one subunit, CCT2, is sufficient to promote the proliferation of cancer cells and enhance the potential for metastasis. We chose two luminal A breast cancer cell lines that had different genetic backgrounds to study the function of CCT2. T47D cells, which are also PR high, manifested the greatest changes upon CCT2 overexpression, with increased spheroid size and numbers and enhanced proliferation. T47D cells also sustained higher levels of CCT2-FLAG expression, displayed spheroid growth reversal, and anchorage-independent growth. Importantly, increases in the gene expression of MYC and CCND1 correlated with CCT2 in T47D cells. Hence, T47D cells show that overexpressing CCT2 can result in the uncontrolled proliferation and the metastatic-like behavior of aggressive, invasive cancer cells and is linked to increased expression of cell cycle regulators. In contrast, MCF7 cells, that have higher CCT2 copy number compared to T47D cells, inherently do not express more CCT2 but rather have high endogenous levels of MYC and CCND1. While CCT2-FLAG overexpression was achieved in these cells, high levels of total CCT2 were not sustained. As a result, we did not observe significant increases in spheroid growth or spheroid growth reversal over that of MCF7 control cells. Taken together, the data from these cell lines support that CCT2 is a novel regulator of cell proliferation that can be anchorage independent and can be a node for the interaction of growth pathways involving MYC and CCND1.

167. MYC functions as a transcription factor and is involved in cell cycle regulation, transformation, angiogenesis and growth of cancers. As a result, MYC is a major proto-oncogene in breast cancer, especially for HER2 and BRCA-1 associated cancers that have poor prognosis. Likewise, CCND1 (cyclin D1) is amplified or overexpressed in cancer and may cooperate with MYC to promote transformation. Herein, we showed that CCT2 expression can upregulate MYC and CCND1 gene expression and promote metastatic-like behavior in luminal A breast cancer cells that are typically less aggressive than the basal-like/TNBC subtypes usually associated with MYC amplification. While CCT2 was identified as a MYC-interacting protein, the relationship between MYC and CCT2 (or any of the CCT subunits) is not well-understood. Others reported that overexpression or suppression of CCT3 in basal-like TNBC cells altered cell proliferation and changed the expression of MYC. One pathway of interest is WNT/ 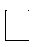 -catenin signaling that regulates cell growth and can drive proliferation in breast cancer cells through MYC and CCND1. Overexpression of CCT3 increased 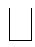 -catenin in MDA-MB-231 and T47D breast cancer cells, which can be modulated by microRNA (miRNA) 223. This miRNA was shown to bind to the 3'UTR of both CCT3 and 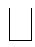 -catenin. The conclusion drawn from this study was that CCT3 mediates breast cancer growth by binding to and competitively inhibiting miRNA 223. However, a predication analysis for miRNA targets in CCT2 did not reveal similar findings (www.targetscan.org/cgi-bin/targetscan/vert_72/view_gene.cgi?rs=ENST00000299300.6&taxid=9606&members=&show cnc=0&shownc=0&showncf1=&showncf2=&subset=1). Moreover, CCT3 mRNA and protein in the current study did not vary substantially between the lentiviral control and CCT2-FLAG overexpressing breast cancer cells. While the contribution of miRNAs and other CCT subunits in the effects of CCT2 overexpression upon MYC, cyclin D1 and subsequent proliferation and spheroid growth is not definitively rule out, the mechanisms driving this interaction remain to be elucidated. The relationship between CCT2, MYC and p53 is studied. In a study of mutant TP53 in head and neck cancers, CCT2 was identified as a MYC and mutant TP53 target gene. Depletion of mutant p53 reduced the interaction of MYC with the CCT2 promoter. Since T47D cells have a missense TP53 mutation with potential gain of function (MCF7 have wild type p53), it is interesting to speculate that the mutant TP53-MYC axis can be involved in the responsiveness of this cell line to CCT2 overexpression.

168. Using 3D cultures to model breast tumor growth, increased spheroid formation upon CCT2 over expression was observed, as evidenced by multiple and larger spheroids, and loss of cell-to-cell contacts that support spheroid structure when CCT2 was depleted. In support, it was also found that actin was upregulated in CCT2 overexpressing cells, especially as cells transitioning from 3D to monolayer cultures. One group showed that cells undergoing 3D to 2D culture transitions retained signatures typical of metastatic cells, such as the expression of cancer stem-cell markers and the development of chemoresistance. These data show that CCT2 can enhance the potential of spheroids to not only to grow but to transition to 2D culture, which is indicative of a more aggressive phenotype, and such a phenotype that can be responsible for promoting drug resistance. As example, spheroids from MDA-MB-231 cells, which was reported had high endogenous levels of CCT2, developed increased resistance to treatment with carboplatin or doxorubicin. Hence, inhibition of CCT2 can be used as a treatment approach for reversing cancer drug resistance. Applications of CCT2 (or CCT) inhibition can be a combination approach with CDK4 inhibitors. The development of resistance to these inhibitors has in part been attributed to the modulation of Rb and CDK2 activity. Since we found that CCT2 overexpression increased CCND1 and CDK2, CCT2 inhibition can improve sensitivity to CDK4 inhibitors.

169. Targeting CCT (and CCT2 specifically) can be an effective therapeutic approach for breast cancer as well as other cancers in which CCT is highly expressed. The highest amplification rate of the CCT2 gene was found in soft tissue cancers. The gene for CCT2 is located in the 12q15 amplicon, which also contains the oncogenes MDM2, YEATS4, FRS2 among others. High level amplification of this amplicon can be an early event in precursor cells that give rise to sarcomas and other cancers like gliomas and melanomas. Genomic imbalances in this region are also found in other cancers such as follicular lymphomas. Given this, therapeutically targeting CCT is complicated by the fact that this is a multi-subunit complex, which challenges the development of small molecule inhibitors. Identifying a subunit, such as CCT2 as essential for the activity of the chaperonin complex, is a first step towards developing a CCT inhibitor for clinical use. Others identified a small molecule that interferes with the CCT2-β-tubulin interaction that can have application in the treatment of resistance to tubulin-binding agents. However, given the genetic heterogeneity of cancer, a CCT inhibitor that works independently of substrate identification is needed. To this end, a small amphipathic peptide was identified, called CT20p, as being cytotoxic in cancer cells that highly express CCT. Using polymeric nanoparticles to systemically deliver CT20p, regression of breast and prostate tumors was achieved in mice and it was demonstrated that CT20p directly binds to the CCT2 subunit. CT20p thus demonstrates that CCT2 and the chaperonin complex can be therapeutically targeted. Herein the study shows the value of CCT2 as a druggable target by demonstrating its role as a cell cycle regulator that intersects with key proliferative factors like MYC and CCND1. The function of CCT2 as an oncogene can be established and elucidating its relationship with MYC can undercover a novel mechanism underlying cancer growth and dissemination and reveal uses for CCT inhibitors in the treatment of drug resistant cancers and in the prevention of cancer relapse and metastasis.

### Example 8. CCT Neuroblastoma

### Methods:

170. *Bioinformatics:* Data was collected from University of California Santa Cruz (UCSC) Xena at xena.ucsc.edu using the combined cohort of "The Cancer Genome Atlas (TCGA), Therapeutically Applicable Research to Generate Effective Treatments (TARGET), and Genotype Tissue Expression (GTEx) samples" dataset (Goldman et al., 2019). Expression for CCT2 gene was compared in GTEx, TCGA, and TARGET samples. Neuroblastoma cases from the TARGET samples were then isolated and analyzed for 9 different gene expressions: CCT2, MYC, MYCN, CDK2, CDK4, CCND1, CCNE1, YAP1, and RB1. Muriel's Data.

171. *Histology:* Slides with formalin-fixed paraffin-embedded (FFPE) tissues were received from US Biomax 1) PC701 and NB642c. Slides were processed by standard immunohistochemistry (IHC) methods for CCT2 and STAT3 staining [ref]. Anti-CCTβ antibody [amino acids 277 and 473 of Human TCP1 beta] (LifeSpan Biosciences) was used. Slides were stained and scored for the CCT2 *stain* by an independent and identification-blinded pathologist, as described previously [ref]. Images were taken using the BZ-X800 Keyence.

172. *Cell Lines*: IMR-32 cells (ATCC^{®} CCL-12^{™}) were cultured in Eagle's Modified Eagle's Medium (EMEM) (Corning) and supplemented with 10% fetal bovine serum (FBS) (Gemini) and 1% Penicillin-Streptomycin (P/S) (Corning) and L-Glutamine. SK-N-AS cells (ATCC^{®} CRL-2137^{™}) were cultured in Dulbecco's Modified Eagle's Medium (DMEM) (Corning) and supplemented with 10% FBS (Gemini), 1X Non-Essential Amino Acids, 1% (P/S) (Coming), and L-Glutamine. IMR-32 and SK-N-AS cells were transfected with a lentiviral plasmid to express CCT2 with a FLAG tag (DYKDDDDK (SEQ ID NO: 7)) hereafter referred to as IMR32-CCT2 or SKNAS-CCT2. Scrambled lentiviral plasmid was used as a control and these cells are hereafter referred to as IMR32-GFP or SKNAS-GFP. 0.5 µg/mL puromycin dihydrochloride (ThermoFisher) was added to maintain plasmids in SKNAS cells and 1.0 µg/mL was used in IMR32 cells.

173. *Western.* Cell lysis, sodium dodecyl sulfate polyacrylamide gel electrophoresis (SDS-PAGE), total protein staining, gel visualization, and gel band quantification were performed as previously described (ref). Lysates collected from 6 well plates or 2D culture flasks. Total protein was determined using the Pierce BCA Protein Assay Kit (Thermo Scientific) following the manufacturer's protocol. Primary antibodies included: Total CCT2 (ab109184) abcam which targets N-terminal ammino acids 1-100; CCT3 (MA5-27872) Invitrogen; Endogenous CCT2 (MAB10050) millipore which targets C-terminal amino acids; and FLAG (ab1162) abcam. Secondary antibodies included IRDye 800CW and IRDye 680CW (LI-COR). Images were collected on LiCORE imager and processed using ImageStudio.

174. *RT-PCR.* RNA was isolated from cells using TRIzolTM (Invitrogen) following the manufacturer's standard protocol for RNA extraction. RNA was quantified using a Nanodrop. Cdna was synthesized using the iScriptTM Cdna Synthesis Kit (Bio-Rad) following the manufacturer's protocol. Cdna was diluted 1:5 and mixed with a Fast SYBRTM Green Master Mix (Applied Biosystems) according to manufacturer's recommendations and then run in the Quantstudio PCR for 40 cycles at 95 °C for 3 sec, and 62 °C for 30 sec.

175. *Migration Assay.* Cells were cultured as seed density of 50,000 cells in 96 well plate (SKNAS) and 60,000 cells (IMR32) and let sit overnight. Sartorius Scratch Wound Assay kit was used to create the scratch and cells were washed according to protocol. Images were collected every 2hrs for 48hrs using Incucyte S3 and analyzed using Incucyte 2021 A software.

176. *Actin and DAPI stain.* Cells were seeded at 10,000-30,000 cells per well in 96 well plate. The next day, cells were fixed in 10% neutral buffered formalin for 10 mins. Cells were then permeabilized in 0.5% Trition X100 in PBS for 5 mins with gentle shaking. Cells were then placed in a 1% blocking solution (1-2% BSA, 0.05% Tween20, in PBS) for 30 mins are room temperature under gentle shaking. Cells were then stained with 1uL Actin in 99 uL of 1% blocking solution in the dark for 30 mins. Cells were washed 3 times with washing solution (0.05% Tween20 in PBS) and let dry for 10 mins. Cells were then stained for DAPI using 5 uL of DAPI in 95 uL of PBS for 10 mins. Cells were then visualized under Pico and images were captured and analyzed using Pico software.

177. Colony Assay. Cells were seeded at 10,000 cells in 6 well plate and cultured for 6 cycles. This was 2 weeks for IMR32 and 3 weeks for SKNAS.

178. *Buffer Experiment.* The protocol was adapted from Lowes et al [ref] as described previously (ref). Samples were washed twice with dilution buffer post antibody staining, resuspended in dilution buffer (350 µL) and transferred into CSS magnets cartridges and run in CSS Analyzer II and analyzed.

179. *Statistics.* Statistical analysis was carried out using GraphPad Prism Software. For RT-PCR analysis, relative gene expression was calculated by dividing a gene's expression in that sample by the average expression of this gene in control samples, i.e., 2-ΔΔCt/average (2-ΔΔCt control sample) with MDA-MB-231 assigned as the control sample.

180. *Bioinformatic databases show that pediatric cancers have high levels of CCT2.* To determine the relevance of CCT2 in pediatric cancers, this study used the UCSC Xena database to compare GTEx (normal tissues), TCGA (adult cancerous tissues) and TARGET (pediatric cancerous tissues). The results show that TCGA cases have significantly higher levels of CCT2 (<0.0001) than GTEx and pediatric cases have significantly higher levels of CCT2 (<0.0001) than TCGA cases, Figure 19A. When pediatric cases were broken down by cancer type, it found that Clear cell sarcoma of the kidney, Neuroblastoma, and Wilms tumor had higher levels than Rhabdoid tumor of the kidney, Figure 1B. To confirm these findings, a different database called KidsFirst was used, where CCT2 levels were assessed across several pediatric cancers, Figure 19C. Some of the highest CCT2 levels were seen in Rhabdomyosarcoma, Choroid plexus carcinoma, and neuroblastoma. To determine where CCT2 levels compared to other tumor markers, the study looked at neuroblastoma cases in UCSC Xena database and several common biomarkers. CCT2 levels were higher than MYC, MYCN, CDK2, CDK4, CCNE1, CCND1, YAP1, and RB1. CCT2 levels were lower than CCND1, Figure 19D.

181. *CCT2 staining is present in multiple pediatric cancer tissues and is high in neuroblastoma.* To determine CCT2 staining in pediatric cancerous tissues, a tissue microarray (TMA) for pediatric cancers was stained. It was found that all cancers had high (score 3-4) staining for CCT2, Figure 20A. Meanwhile most normal tissues had low staining (score 1-2) for CCT2. Neuroblastoma had some of the highest CCT2 staining, therefore we stained a neuroblastoma TMA to get a better range of tumor stage and aggression. The study found staining was present in all tumor tissues and minimal in normal tissue, Figure 20B. While not statistically significant due to few samples, there did seem to be a trend with increased staining and increased tumor grade, CD56 staining, and//or CgA staining.

182. *CCT2 is present in neuroblastoma cell lines SKNAS and IMR32.* To determine if CCT2 was detectable in neuroblastoma cell lines, this study first analyzed RNA from Nemours' database on IMR32 and SKNAS cell lines. IMR32 is a MYCN-amplified high risk neuroblastoma, pre-treatment. SKNAS is a MYCN down-regulated high-risk neuroblastoma, post-treatment from a metastatic recurrence. CCT1, CCT2, and CCT4 show statistically significant increases in IMR32 compared to SKNAS, Figure 21A. The study then cultured IMR32 and SKNAS cell lines and measured the RNA and protein for CCT2 and CCT3 levels. RT-PCR showed high levels of CCT2 in IMR32 and SKNAS compared to MDA-MB-231, which is a Triple Negative Breast Cancer cell line and has historically high levels of CCT2 (REF). This increase in CCT2 above MDA-MB-231 was only statistically significant for IMR32 (p=0.0031), Figure 21B. CCT3 levels were comparably high across IMR32, SKNAS, and MDA-MB-231 cell lines. Western analysis saw similar results. CCT2 and CCT3 levels were statistically significantly higher in IMR32 over SKNAS (p=0.0108 and p=0.0919 respectively), Figure 21C.

183. *Knockdown of CCT2 in SKNAS cells decreases viability and decreases actin IMR32.* To determine if the roll of CCT2 in these neuroblastoma cell lines was vital, the cell lines were transfected with CCT2-shRNA. GFP-shRNA was used for control transfections. Figure 22 shows that transfection was successful in SKNAS cell lines. This inducible system is activated by doxycycline. Western analysis showed statistically significant decreases in total CCT2 (p=0.0092), CCT3 (p=0.0307), and Endo CCT2 (p=0.0284) after treatment with doxycycline, Figure 22A. To determine the knockdown's effect on cell viability, MTT assay was completed on transiently transfected cells. MTT assay showed 50% decrease in viable cells after 48hrs in CCT2-shRNA treated cells than GFP-shRNA treated cells (p<0.0001), Figure 22B. IMR32 cells did not accept the transfection as well, therefore we treated these cells with a pool of siRNA that targets CCT2. Actin and DAPI staining shows a 80-60% decrease in actin present in cells that were treated with siRNA compared to control, Figure 22C.

184. *Lentiviral transduction of CCT2 plasmid increases CCT2 RNA and protein.* To determine if CCT2 can be overexpressed in SKNAS and IMR32 cell lines, they were transfected with lentiviral plasmid to overexpress CCT2. Lentiviral GFP was used for control. RT-PCR analysis show statistically significant increases in CCT2 in SKNAS (P=0.0214) but not in IMR32 (0.2540), Figure 23A. Increases in CCT3 were not statistically significant, Figure 23A. Western analysis showed that CCT2 overexpression in SKNAS resulted in statistically significant decreases in endogenous CCT2 (p=0.0108) and statistically significant increases in FLAG (p=0.0040). Overall, the increases in Total CCT2 and CCT3 were not statistically significant. In IMR32 cells, the CCT2 overexpression resulted in statistically significant increases in Total CCT2 (p=0.0337) and FLAG (p=0.0214) with statistically significant decreases in Endogenous CCT2 (p=0.0121). CCT3 remained unchanged.

185. *Functional Assays show no change from overexpression of CCT2 in neuroblastoma cell lines.* To determine the effect overexpression of CCT2 has on the SKNAS cell lines, a migration assay was completed. The migration assay showed no significant differences between the SKNAS-GFP cell line and the SKNAS-CCT2 cell line, Figure 24A. To determine if there was an increase in chaperonin activity, we measured actin levels in SKNAS and IMR32 cell lines and compared control vs CCT2 overexpressing. The Actin staining remained unchanged between overexpressing cell lines and controls, Figure 5B.

**Table 1. Primer pairs used in RT-qPCR assay.**

| | |
|---|---|
| CDK4 | Forward: TCTGGTACCGAGCTCCCGAA (SEQ ID NO: 8) |
| | Reverse: GATTTGCCCAACTGGTCGG (SEQ ID NO: 9) |
| CDK2 | Forward: ATGGAGAACTTCCAAAAGGTGGA (SEQ ID NO: 10) |
| | Reverse: CAGGCGGATTTTCTTAAGCG (SEQ ID NO: 11) |
| Cyclin D1 | Forward: GCGTCCATGCGGAAGATC (SEQ ID NO: 12) |
| | Reverse: ATGGCCAGCGGGAAGAC (SEQ ID NO: 13) |
| Cyclin E1 | Forward: GCCAGCCTTGGGACAATAATG (SEQ ID NO: 14) |
| | Reverse: AGTTTGGGTAAACCCGGTCAT (SEQ ID NO: 15) |
| MYC | Forward: GGAGGCTATTCTGCCCATTTG (SEQ ID NO: 16) |
| | Reverse: GAGGCTGCTGGTTTTCCACTA (SEQ ID NO: 17) |
| P27 | Forward: ACCTGCAACCGACGATTCT (SEQ ID NO: 18) |
| | Reverse: CAGGCTTCTTGGGCGTCTG (SEQ ID NO: 19) |
| P21 | Forward: TGAGCCGCGACTGTGATG (SEQ ID NO: 20) |
| | Reverse: GTCTCGGTGACAAAGTCGAAGTT (SEQ ID NO: 21) |
| GAPDH | Forward: GAAGGTGAAGGTCGGAGTCAAC (SEQ ID NO: 22) |
| | Reverse: TGGAAGATGGTGATGGGATTTC (SEQ ID NO: 23) |
| CCT3 | Forward: TCAGTCGGTGGTCATCTTTGG (SEQ ID NO: 24) |
| | Reverse: CCTCCAGGTATCTTTTCCACTCT (SEQ ID NO: 25) |
| CCT2-FLAG | Forward: CAGAGGTGATTCTGCGTGTG (SEQ ID NO: 26) |
| | Reverse: TGTCGTCGTCGTCCTTGTAG (SEQ ID NO: 27) |
| CCT2 | Forward: GTTGGAGAGAAGCCACGAAG (SEQ ID NO: 28) |
| | Reverse: GTTGCCAGAGCCTTTCAGTC (SEQ ID NO: 29) |

**Table 2. Co-occurring genetic alterations of CCT2 with cell cycle genes [From combined breast cancer studies (9103 patients/9524 samples in 17 studies) The Cancer Genome Atlas (TCGA)].**

| **A** | **B** | **p-Value** | **Tendency** |
|---|---|---|---|
| **CCT2** | **CDK4** | <0.001 | Co-occurrence |
| MYC | CCNE1 | <0.001 | Co-occurrence |
| CCNE1 | CDKN1A | <0.001 | Co-occurrence |
| CCND1 | CDK4 | <0.001 | Co-occurrence |
| **CCT2** | **CCND1** | <0.001 | Co-occurrence |
| CDKN1A | CDKN1B | <0.001 | Co-occurrence |
| CDK4 | CDKN1A | <0.001 | Co-occurrence |
| CDK4 | CDKN1B | <0.001 | Co-occurrence |
| CCND1 | CDK2 | <0.001 | Co-occurrence |
| MYC | CDKN1A | <0.001 | Co-occurrence |
| CDK4 | CDK2 | <0.001 | Co-occurrence |
| CDK2 | CDKN1B | 0.001 | Co-occurrence |
| **CCT2** | **MYC** | 0.002 | Co-occurrence |
| **CCT2** | **CDK2** | 0.002 | Co-occurrence |
| MYC | CDKN1B | 0.002 | Co-occurrence |
| MYC | CDK4 | 0.004 | Co-occurrence |
| MYC | CCND1 | 0.005 | Co-occurrence |
| CCNE1 | CDKN1B | 0.005 | Co-occurrence |

**Table 3. Genetic alterations of CCT subunits in MCF7 and T47D cells (COSMIC database)**

| **Cell Line** | **CCT2 subunits alterations** | |
|---|---|---|
| | **Mutation** | **CNV and expression** |
| **MCF7** | CCT6B (Substitution - intronic) | CCT3 is overexpressed |
| **T47D** | CCT8 (Substitution - intronic) | No changes reported |

**Table 4. Summary of multiple mixed effect linear regression analysis on expression of each gene in response to cell line (T47D vs MCF-7), treatment [lentiviral control (control) vs CCT2-FLAG overexpression (over-CCT2)] and day. Day is set as a continuous variable with value of 0 for day 0, 1 for day 3, 2 for day 5 and 3 for day 8 of spheroid culture. * p <0.05, ** p<0.005, *** p<0.0005, SE: standard error.**

| **Gene** | **Factor** | **Estimated effect** | **SE** | **P-value** | **Sig.** |
|---|---|---|---|---|---|
| MYC | T47D vs. MCF7 | *2.459* | *0.386* | *0* | *** |
| | Over-CCT2 vs. Control | *0.765* | *0.386* | *0.048* | * |
| | Day | *-1.124* | *0.167* | *0* | *** |
| | Intercept | -0.623 | 0.418 | 0.136 | |
| CDK4 | T47D vs. MCF7 | 0.525 | 0.235 | 0.025 | * |
| | Over-CCT2 vs. Control | 0.047 | 0.235 | 0.842 | |
| | Day | -0.976 | 0.105 | 0 | *** |
| | Intercept | -0.062 | 0.257 | 0.81 | |
| CDK2 | T47D vs. MCF7 | 3.276 | 0.228 | 0 | *** |
| | Over-CCT2 vs. Control | -0.083 | 0.228 | 0.715 | |
| | Day | -1.13 | 0.102 | 0 | *** |
| | Intercept | 0.51 | 0.25 | 0.041 | * |
| Cyclin_D1 | T47D vs. MCF7 | -0.579 | 0.348 | 0.096 | |
| | Over-CCT2 vs. Control | 0.381 | 0.348 | 0.274 | |
| | Day | -0.981 | 0.069 | 0 | *** |
| | Intercept | -0.406 | 0.319 | 0.203 | |
| Cyclin_D1 in T47D* | Over-CCT2 vs. Control | 1.130 | 0.350 | 0.001 | *** |
| | Day | -0.930 | 0.101 | 0.000 | *** |
| | Intercept | 0.037 | 0.290 | 0.899 | |
| Cyclin_E1 | T47D vs. MCF7 | 3.698 | 0.281 | 0 | *** |
| | Over-CCT2 vs. Control | 0.122 | 0.281 | 0.665 | |
| | Day | -1.266 | 0.126 | 0 | *** |
| | Intercept | 0.696 | 0.308 | 0.024 | * |
| Total_CCT2 | T47D vs. MCF7 | 2.738 | 0.333 | 0 | *** |
| | Over-CCT2 vs. Control | 2.448 | 0.333 | 0 | *** |
| | Day | -0.845 | 0.097 | 0 | *** |
| | Intercept | -0.089 | 0.323 | 0.784 | |
| CCT2_FLAG | T47D vs. MCF7 | 3.276 | 0.565 | 0 | *** |
| | Over-CCT2 vs. Control | | | | |
| | Day | -0.657 | 0.253 | 0.009 | ** |
| | Intercept | -0.522 | 0.551 | 0.344 | |

| | | | | | |
|---|---|---|---|---|---|
| *MCF-7 lentiviral control was used as the reference samples for all analysis except for cyclin D1 in T47D cells in which the T47D lentiviral control was used as the reference sample. | | | | | |

**Table 5. Summary of multi-factor ANOVA analysis showing effect of individual factors on the expression of each gene in response to effects of cell line (T47D vs MCF-7), treatment [lentiviral control (control) vs CCT2-FLAG overexpression (over-CCT2)] and 2D (before spheroid culture) vs 2D post 3D (after spheroid growth reversal).**

| **Gene** | **Factor** | **Coef.** | **SE** | **P-value** | **Sig.** |
|---|---|---|---|---|---|
| MYC | T47D vs. MCF7 | 0.455 | 0.279 | 0.113 | |
| | Over-CCT2 vs. Control | 0.815 | 0.279 | 0.007 | ** |
| | 2D post 3D vs. 2D | -0.69 | 0.301 | 0.029 | * |
| | Intercept | -0.603 | 0.308 | 0.059 | |
| CDK4 | T47D vs. MCF7 | 0.019 | 0.163 | 0.906 | |
| | Over-CCT2 vs. Control | 0.314 | 0.163 | 0.063 | |
| | 2D post 3D vs. 2D | -0.3 | 0.173 | 0.093 | |
| | Intercept | -0.009 | 0.182 | 0.961 | |
| CDK2 | T47D vs. MCF7 | 2.784 | 0.287 | 0 | *** |
| | Over-CCT2 vs. Control | 0.667 | 0.287 | 0.027 | * |
| | 2D post 3D vs. 2D | -0.221 | 0.305 | 0.474 | |
| | Intercept | -0.253 | 0.321 | 0.436 | |
| Cyclin_D1 | T47D vs. MCF7 | -0.977 | 0.197 | 0 | *** |
| | Over-CCT2 vs. Control | 0.63 | 0.197 | 0.003 | ** |
| | 2D post 3D vs. 2D | -0.289 | 0.212 | 0.182 | |
| | Intercept | -0.259 | 0.217 | 0.242 | |
| Cyclin_E1 | T47D vs. MCF7 | 3.275 | 0.172 | 0 | *** |
| | Over-CCT2 vs. Control | 0.201 | 0.172 | 0.251 | |
| | 2D post 3D vs. 2D | 0.022 | 0.185 | 0.905 | |
| | Intercept | 0.559 | 0.19 | 0.006 | ** |
| P21 | T47D vs. MCF7 | 0.377 | 0.368 | 0.319 | |
| | Over-CCT2 vs. Control | 1.101 | 0.368 | 0.008 | ** |
| | 2D post 3D vs. 2D | -0.565 | 0.368 | 0.142 | |
| | Intercept | -0.54 | 0.359 | 0.149 | |
| P27 | T47D vs. MCF7 | -0.179 | 0.268 | 0.514 | |
| | Over-CCT2 vs. Control | 0.213 | 0.268 | 0.436 | |
| | 2D post 3D vs. 2D | -0.777 | 0.268 | 0.009 | ** |
| | Intercept | -0.388 | 0.262 | 0.155 | |

**Table 6. CCT2 interaction network based on having two or more pieces of experimental evidence for interactions (generated using BioGRid)**

| Interactor | Description |
|---|---|
| TCP1 | t-complex 1 |
| PPP2R2C | protein phosphatase 2, regulatory subunit B, gamma |
| CCT7 | chaperonin containing TCP1, subunit 7 (eta) |
| CCT5 | chaperonin containing TCP1, subunit 5 (epsilon) |
| CCT4 | chaperonin containing TCP1, subunit 4 (delta) |
| TUBG1 | tubulin, gamma 1 |
| YAP1 | Yes-associated protein 1 |
| CCT6A | chaperonin containing TCP1, subunit 6A (zeta 1) |
| DCAF7 | DDB1 and CUL4 associated factor 7 |
| CCT3 | chaperonin containing TCP1, subunit 3 (gamma) |
| CDC20 | cell division cycle 20 |
| STRN | striatin, calmodulin binding protein |
| PPP4C | protein phosphatase 4, catalytic subunit |
| MLST8 | MTOR associated protein, LST8 homolog (S. cerevisiae) |
| CCT8 | chaperonin containing TCP1, subunit 8 (theta) |
| PPP2CA | protein phosphatase 2, catalytic subunit, alpha isozyme |
| ACTR1B | ARP1 actin-related protein 1 homolog B, centractin beta (yeast) |
| PPP2R2B | protein phosphatase 2, regulatory subunit B, beta |
| CCT6B | chaperonin containing TCP1, subunit 6B (zeta 2) |
| FBXW4 | F-box and WD repeat domain containing 4 |
| VCP | valosin containing protein |
| TP53 | tumor protein p53 |
| PPP2CB | protein phosphatase 2, catalytic subunit, beta isozyme |
| HDAC1 | histone deacetylase 1 |
| PPP2R2D | protein phosphatase 2, regulatory subunit B, delta |
| STRN3 | striatin, calmodulin binding protein 3 |
| NEDD1 | neural precursor cell expressed, developmentally down-regulated 1 |
| POC1A | POC1 centriolar protein A |
| BUB3 | BUB3 mitotic checkpoint protein |
| DCAF5 | DDB1 and CUL4 associated factor 5 |
| FBXW8 | F-box and WD repeat domain containing 8 |
| ILK | integrin-linked kinase |
| RFWD2 | ring finger and WD repeat domain 2, E3 ubiquitin protein ligase |
| IGBP1 | immunoglobulin (CD79A) binding protein 1 |
| RFWD3 | ring finger and WD repeat domain 3 |
| WDR76 | WD repeat domain 76 |
| WDR83 | WD repeat domain 83 |
| FYCO1 | FYVE and coiled-coil domain containing 1 |
| OBSL1 | obscurin-like 1 |
| POC1B | POC1 centriolar protein B |
| MEPCE | methylphosphate capping enzyme |
| CASP7 | caspase 7, apoptosis-related cysteine peptidase |
| WRAP53 | WD repeat containing, antisense to TP53 |
| TUBB2A | tubulin, beta 2A class IIa |
| MAPK10 | mitogen-activated protein kinase 10 |
| SEC31B | SEC31 homolog B (S. cerevisiae) |
| TUBA1B | tubulin, alpha 1b |
| RPTOR | regulatory associated protein of MTOR, complex 1 |
| DDB2 | damage-specific DNA binding protein 2, 48kDa |
| SSSCA1 | Sjogren syndrome/scleroderma autoantigen 1 |
| PACRG | PARK2 co-regulated |
| XIAP | X-linked inhibitor of apoptosis, E3 ubiquitin protein ligase |
| TUBB2B | tubulin, beta 2B class IIb |
| CCDC8 | coiled-coil domain containing 8 |
| ESR1 | estrogen receptor 1 |
| WDR92 | WD repeat domain 92 |
| TUBB | tubulin, beta class I |
| TUBA3E | tubulin, alpha 3e |
| ACTL6A | actin-like 6A |
| MYC | v-mvc avian mvelocvtomatosis viral oncogene homolog |
| TUBAL3 | tubulin, alpha-like 3 |
| ERCC8 | excision repair cross-complementation group 8 |
| PEX7 | peroxisomal biogenesis factor 7 |
| FLCN | folliculin |
| TTLL3 | tubulin tyrosine ligase-like family, member 3 |
| CYLD | cylindromatosis (turban tumor syndrome) |
| NLE1 | notchless homolog 1 (Drosophila) |
| PSMA3 | proteasome (prosome, macropain) subunit, alpha type, 3 |
| PPP6C | protein phosphatase 6, catalytic subunit |
| ATP6V1B1 | ATPase, H+ transporting, lysosomal 56/58kDa, V1 subunit B1 |
| CDK5 | cyclin-dependent kinase 5 |
| CUL7 | cullin 7 |
| PARK2 | parkin RBR E3 ubiquitin protein ligase |
| HDAC3 | histone deacetylase 3 |

**Table 7**

| Name | Sequence |
|---|---|
| CT20 | VTIFVAGVLTASLTIWKKMG (SEQ ID NO:1) |
| CT20-V1 | ASLTIWKKMG (SEQ ID NO:2) |
| CT20-V2 | VTIFVAGVLT (SEQ ID NO:3) |
| CT20-V3 | VTIFVAG (SEQ ID NO:4) |
| CT20-V4 | IFVAG (SEQ ID NO:5) |
| CT20-V5 | IWKKMG (SEQ ID NO:6) |

### References

1. Bray F, Ferlay J, Soerjomataram I, Siegel RL, Torre LA, Jemal A. Global cancer statistics 2018: GLOBOCAN estimates of incidence and mortality worldwide for 36 cancers in 185 countries. CA: A Cancer Journal for Clinicians. 2018;68(6):394-424.
2. Cejalvo JM, Martinez de Duenas E, Galvan P, Garcia-Recio S, Burgues Gasion O, Pare L, et al. Intrinsic Subtypes and Gene Expression Profiles in Primary and Metastatic Breast Cancer. Cancer Res. 2017;77(9):2213-21.
3. Cancer Genome Atlas N. Comprehensive molecular portraits of human breast tumours. Nature. 2012;490(7418):61-70.
4. Perou CM, Sorlie T, Eisen MB, van de Rijn M, Jeffrey SS, Rees CA, et al. Molecular portraits of human breast tumours. Nature. 2000;406(6797):747-52.
5. van de Vijver MJ. Genetic alterations in breast cancer. Current Diagnostic Pathology. 2000;6(4):271-81.
6. Berns EM, Foekens JA, van Staveren IL, van Putten WL, de Koning HY, Portengen H, et al. Oncogene amplification and prognosis in breast cancer: relationship with systemic treatment. Gene. 1995;159(1):11-8.
7. Quesnel B, Preudhomme C, Fournier J, Fenaux P, Peyrat JP. MDM2 gene amplification in human breast cancer. European journal of cancer. 1994;30A(7):982-4.
8. Turner N, Pearson A, Sharpe R, Lambros M, Geyer F, Lopez-Garcia MA, et al. FGFR1 amplification drives endocrine therapy resistance and is a therapeutic target in breast cancer. Cancer Res. 2010;70(5):2085-94.
9. Lebeau J, Goubin G. Amplification of the epidermal growth factor receptor gene in the BT20 breast carcinoma cell line. Int J Cancer. 1987;40(2):189-91.
10. An HX, Beckmann MW, Reifenberger G, Bender HG, Niederacher D. Gene amplification and overexpression of CDK4 in sporadic breast carcinomas is associated with high tumor cell proliferation. The American journal of pathology. 1999;154(1):113-8.
11. Yersal O, Barutca S. Biological subtypes of breast cancer: Prognostic and therapeutic implications. World J Clin Oncol. 2014;5(3):412-24.
12. Peurala E, Koivunen P, Haapasaari KM, Bloigu R, Jukkola-Vuorinen A. The prognostic significance and value of cyclin D1, CDK4 and p16 in human breast cancer. Breast Cancer Res. 2013;15(1):R5.
13. Bergamaschi A, Kim YH, Wang P, Sorlie T, Hernandez-Boussard T, Lonning PE, et al. Distinct patterns of DNA copy number alteration are associated with different clinicopathological features and gene-expression subtypes of breast cancer. Genes Chromosomes Cancer. 2006;45(11):1033-40.
14. Nielsen NH, Loden M, Cajander J, Emdin SO, Landberg G. G1-S transition defects occur in most breast cancers and predict outcome. Breast Cancer Res Treat. 1999;56(2):105-12.
15. Otto T, Sicinski P. Cell cycle proteins as promising targets in cancer therapy. Nat Rev Cancer. 2017;17(2):93-115.
16. Caldon CE, Daly RJ, Sutherland RL, Musgrove EA. Cell cycle control in breast cancer cells. J Cell Biochem. 2006;97(2):261-74.
17. Elsheikh S, Green AR, Aleskandarany MA, Grainge M, Paish CE, Lambros MB, et al. CCND1 amplification and cyclin D1 expression in breast cancer and their relation with proteomic subgroups and patient outcome. Breast Cancer Res Treat. 2008;109(2):325-35.
18. Li Z, Cui J, Yu Q, Wu X, Pan A, Li L. Evaluation of CCND1 amplification and CyclinD1 expression: diffuse and strong staining of CyclinD1 could have same predictive roles as CCND1 amplification in ER positive breast cancers. American journal of translational research. 2016;8(1):142-53.
19. Deming SL, Nass SJ, Dickson RB, Trock BJ. C-myc amplification in breast cancer: a meta-analysis of its occurrence and prognostic relevance. Br J Cancer. 2000;83(12):1688-95.
20. Garcia-Gutierrez L, Delgado MD, Leon J. MYC Oncogene Contributions to Release of Cell Cycle Brakes. Genes (Basel). 2019;10(3).
21. Bretones G, Delgado MD, Leon J. Myc and cell cycle control. Biochim Biophys Acta. 2015;1849(5):506-16.
22. Yu Q, Sicinska E, Geng Y, Ahnstrom M, Zagozdzon A, Kong Y, et al. Requirement for CDK4 kinase function in breast cancer. Cancer Cell. 2006;9(1):23-32.
23. Shah M, Nunes MR, Steams V. CDK4/6 Inhibitors: Game Changers in the Management of Hormone Receptor-Positive Advanced Breast Cancer? Oncology (Williston Park). 2018;32(5):216-22.
24. Gao JJ, Cheng J, Bloomquist E, Sanchez J, Wedam SB, Singh H, et al. CDK4/6 inhibitor treatment for patients with hormone receptor-positive, HER2-negative, advanced or metastatic breast cancer: a US Food and Drug Administration pooled analysis. Lancet Oncol. 2020;21(2):250-60.
25. Li Z, Zou W, Zhang J, Zhang Y, Xu Q, Li S, et al. Mechanisms of CDK4/6 Inhibitor Resistance in Luminal Breast Cancer. Front Pharmacol. 2020;11:580251.
26. Du Q, Guo X, Wang M, Li Y, Sun X, Li Q. The application and prospect of CDK4/6 inhibitors in malignant solid tumors. J Hematol Oncol. 2020;13(1):41.
27. Kim S, Willison KR, Horwich AL. Cystosolic chaperonin subunits have a conserved ATPase domain but diverged polypeptide-binding domains. Trends Biochem Sci. 1994;19(12):543-8.
28. Spiess C, Miller EJ, McClellan AJ, Frydman J. Identification of the TRiC/CCT substrate binding sites uncovers the function of subunit diversity in eukaryotic chaperonins. Molecular cell. 2006;24(1):25-37.
29. Willison KR. The substrate specificity of eukaryotic cytosolic chaperonin CCT. Philos Trans R Soc Lond B Biol Sci. 2018;373(1749).
30. Narayanan A, Pullepu D, Kabir MA. The interactome of CCT complex - A computational analysis. Comput Biol Chem. 2016;64:396-402.
31. Yam AY, Xia Y, Lin HT, Burlingame A, Gerstein M, Frydman J. Defining the TRiC/CCT interactome links chaperonin function to stabilization of newly made proteins with complex topologies. Nature structural & molecular biology. 2008;15(12):1255-62.
32. Vallin J, Grantham J. The role of the molecular chaperone CCT in protein folding and mediation of cytoskeleton-associated processes: implications for cancer cell biology. Cell Stress Chaperones. 2019;24(1):17-27.
33. Liu X, Lin CY, Lei M, Yan S, Zhou T, Erikson RL. CCT chaperonin complex is required for the biogenesis of functional Plk1. Mol Cell Biol. 2005;25(12):4993-5010.
34. Wang Q, Huang WR, Chih WY, Chuang KP, Chang CD, Wu Y, et al. Cdc20 and molecular chaperone CCT2 and CCT5 are required for the Muscovy duck reovirus p10.8-induced cell cycle arrest and apoptosis. Veterinary microbiology. 2019;235:151-63.
35. Dekker C. On the role of the chaperonin CCT in the just-in-time assembly process of APC/CCdc20. FEBS Lett. 2010;584(3):477-81.
36. Camasses A, Bogdanova A, Shevchenko A, Zachariae W. The CCT chaperonin promotes activation of the anaphase-promoting complex through the generation of functional Cdc20. Mol Cell. 2003;12(1):87-100.
37. Won KA, Schumacher RJ, Farr GW, Horwich AL, Reed SI. Maturation of human cyclin E requires the function of eukaryotic chaperonin CCT. Mol Cell Biol. 1998;18(12):7584-9.
38. Trinidad AG, Muller PA, Cuellar J, Klejnot M, Nobis M, Valpuesta JM, et al. Interaction of p53 with the CCT complex promotes protein folding and wild-type p53 activity. Mol Cell. 2013;50(6):805-17.
39. Kasembeli M, Lau WC, Roh SH, Eckols TK, Frydman J, Chiu W, et al. Modulation of STAT3 folding and function by TRiC/CCT chaperonin. PLoS Biol. 2014;12(4):e1001844.
40. Showalter AE, Martini AC, Nierenberg D, Hosang K, Fahmi NA, Gopalan P, et al. Investigating Chaperonin-Containing TCP-1 subunit 2 as an essential component of the chaperonin complex for tumorigenesis. Sci Rep. 2020;10(1):798.
41. Guest ST, Kratche ZR, Bollig-Fischer A, Haddad R, Ethier SP. Two members of the TRiC chaperonin complex, CCT2 and TCP1 are essential for survival of breast cancer cells and are linked to driving oncogenes. Exp Cell Res. 2015;332(2):223-35.
42. Bassiouni R, Nemec KN, Iketani A, Flores O, Showalter A, Khaled AS, et al. Chaperonin Containing TCP-1 Protein Level in Breast Cancer Cells Predicts Therapeutic Application of a Cytotoxic Peptide. Clin Cancer Res. 2016;22(17):4366-79.
43. Carr AC, Khaled AS, Bassiouni R, Flores O, Nierenberg D, Bhatti H, et al. Targeting chaperonin containing TCP1 (CCT) as a molecular therapeutic for small cell lung cancer. Oncotarget. 2017;8(66):110273-88.
44. Gao J, Aksoy BA, Dogrusoz U, Dresdner G, Gross B, Sumer SO, et al. Integrative analysis of complex cancer genomics and clinical profiles using the cBioPortal. Sci Signal. 2013;6(269):pl1.
45. Cerami E, Gao J, Dogrusoz U, Gross BE, Sumer SO, Aksoy BA, et al. The cBio cancer genomics portal: an open platform for exploring multidimensional cancer genomics data. Cancer Discov. 2012;2(5):401-4.
46. Tate JG, Bamford S, Jubb HC, Sondka Z, Beare DM, Bindal N, et al. COSMIC: the Catalogue Of Somatic Mutations In Cancer. Nucleic acids research. 2019;47(D1):D941-D7.
47. Goldman MJ, Craft B, Hastie M, Repecka K, McDade F, Kamath A, et al. Visualizing and interpreting cancer genomics data via the Xena platform. Nat Biotechnol. 2020;38(6):675-8.
48. Zwijsen RM, Wientjens E, Klompmaker R, van der Sman J, Bernards R, Michalides RJ. CDK-independent activation of estrogen receptor by cyclin D1. Cell. 1997;88(3):405-15.
49. Guarducci C, Bonechi M, Boccalini G, Benelli M, Risi E, Di Leo A, et al. Mechanisms of Resistance to CDK4/6 Inhibitors in Breast Cancer and Potential Biomarkers of Response. Breast Care (Basel). 2017;12(5):304-8.
50. Kunjithapatham R, Karthikeyan S, Geschwind JF, Kieserman E, Lin M, Fu DX, et al. Reversal of anchorage-independent multicellular spheroid into a monolayer mimics a metastatic model. Sci Rep. 2014;4:6816.
51. Smyrek I, Mathew B, Fischer SC, Lissek SM, Becker S, Stelzer EHK. E-cadherin, actin, microtubules and FAK dominate different spheroid formation phases and important elements of tissue integrity. Biology open. 2019;8(1):bio037051.
52. Chitcholtan K, Asselin E, Parent S, Sykes PH, Evans JJ. Differences in growth properties of endometrial cancer in three dimensional (3D) culture and 2D cell monolayer. Exp Cell Res. 2013;319(1):75-87.
53. Riedl A, Schlederer M, Pudelko K, Stadler M, Walter S, Unterleuthner D, et al. Comparison of cancer cells in 2D vs 3D culture reveals differences in AKT-mTOR-S6K signaling and drug responses. J Cell Sci. 2017;130(1):203-18.
54. Schmidt M, Scholz CJ, Polednik C, Roller J. Spheroid-based 3-dimensional culture models: Gene expression and functionality in head and neck cancer. Oncology reports. 2016;35(4):2431-40.
55. Tanaka H, Watanabe T. Mechanisms Underlying Recurrent Genomic Amplification in Human Cancers. Trends Cancer. 2020;6(6):462-77.
56. Jonsson G, Staaf J, Vallon-Christersson J, Ringner M, Holm K, Hegardt C, et al. Genomic subtypes of breast cancer identified by array-comparative genomic hybridization display distinct molecular and clinical characteristics. Breast Cancer Res. 2010;12(3):R42.
57. Nardulli AM, Katzenellenbogen BS. Progesterone receptor regulation in T47D human breast cancer cells: analysis by density labeling of progesterone receptor synthesis and degradation and their modulation by progestin. Endocrinology. 1988;122(4):1532-40.
58. Xu J, Chen Y, Olopade OI. MYC and Breast Cancer. Genes Cancer. 2010;1(6):629-40.
59. Chen Y, Olopade OI. MYC in breast tumor progression. Expert Rev Anticancer Ther. 2008;8(10):1689-98.
60. Wang Y, Thakur A, Sun Y, Wu J, Biliran H, Bollig A, et al. Synergistic effect of cyclin D1 and c-Myc leads to more aggressive and invasive mammary tumors in severe combined immunodeficient mice. Cancer Res. 2007;67(8):3698-707.
61. Spiniello M, Steinbrink MI, Cesnik AJ, Miller RM, Scalf M, Shortreed MR, et al. Comprehensive in vivo identification of the c-Myc mRNA protein interactome using HyPR-MS. RNA. 2019;25(10):1337-52.
62. Xu G, Bu S, Wang X, Zhang H, Ge H. Suppression of CCT3 inhibits the proliferation and migration in breast cancer cells. Cancer Cell Int. 2020;20:218.
63. Qu H, Zhu F, Dong H, Hu X, Han M. Upregulation of CCT-3 Induces Breast Cancer Cell Proliferation Through miR-223 Competition and Wnt/β-Catenin Signaling Pathway Activation. Frontiers in oncology. 2020;10:533176-.
64. Wang SH, Li N, Wei Y, Li QR, Yu ZP. beta-catenin deacetylation is essential for WNT-induced proliferation of breast cancer cells. Molecular medicine reports. 2014;9(3):973-8.
65. Bilir B, Kucuk O, Moreno CS. Wnt signaling blockage inhibits cell proliferation and migration, and induces apoptosis in triple-negative breast cancer cells. J Transl Med. 2013;11:280.
66. Ganci F, Pulito C, Valsoni S, Sacconi A, Turco C, Vahabi M, et al. PI3K Inhibitors Curtail MYC-Dependent Mutant p53 Gain-of-Function in Head and Neck Squamous Cell Carcinoma. Clin Cancer Res. 2020;26(12):2956-71.
67. Huang Z, Yu P, Tang J. Characterization of Triple-Negative Breast Cancer MDA-MB-231 Cell Spheroid Model. OncoTargets and therapy. 2020;13:5395-405.
68. Kumarasamy V, Vail P, Nambiar R, Witkiewicz AK, Knudsen ES. Functional determinants of cell-cycle plasticity and sensitivity to CDK4/6 inhibition. Cancer Res. 2020.
69. Tyler R, Wanigasooriya K, Taniere P, Almond M, Ford S, Desai A, et al. A review of retroperitoneal liposarcoma genomics. Cancer Treat Rev. 2020;86:102013.
70. Italiano A, Cardot N, Dupre F, Monticelli I, Keslair F, Piche M, et al. Gains and complex rearrangements of the 12q13-15 chromosomal region in ordinary lipomas: the "missing link" between lipomas and liposarcomas? Int J Cancer. 2007;121(2):308-15.
71. Berglund M, Enblad G, Thunberg U, Amini RM, Sundstrom C, Roos G, et al. Genomic imbalances during transformation from follicular lymphoma to diffuse large B-cell lymphoma. Modern pathology : an official journal of the United States and Canadian Academy of Pathology, Inc. 2007;20(1):63-75.
72. Lin YF, Tsai WP, Liu HG, Liang PH. Intracellular beta-tubulin/chaperonin containing TCP1-beta complex serves as a novel chemotherapeutic target against drug-resistant tumors. Cancer Res. 2009;69(17):6879-88.
73. Lee MW, Bassiouni R, Sparrow NA, Iketani A, Boohaker RJ, Moskowitz C, et al. The CT20 peptide causes detachment and death of metastatic breast cancer cells by promoting mitochondrial aggregation and cytoskeletal disruption. Cell Death Dis. 2014;5:e1249.
74. Boohaker RJ, Zhang G, Lee MW, Nemec KN, Santra S, Perez JM, et al. Rational development of a cytotoxic peptide to trigger cell death. Molecular pharmaceutics. 2012;9(7):2080-93.
75. Flores O, Santra S, Kaittanis C, Bassiouni R, Khaled AS, Khaled AR, et al. PSMA-Targeted Theranostic Nanocarrier for Prostate Cancer. Theranostics. 2017;7(9):2477-94.

## Claims

1. A chaperonin-containing TCP1 subunit 2 (CCT2) inhibitor for use in treating a cancer or a drug-resistant cancer in a subject.

2. The CCT2 inhibitor for the use of claim 1, wherein the CCT2 inhibitor comprises a small molecule, an antibody, a peptide, a polypeptide, a small interfering RNA (siRNA), or a short hairpin RNA; preferably wherein the peptide is a CT20p peptide; most preferably wherein the CT20 peptide comprises an amino acid sequence according to any one of SEQ ID NOs: 1 to 6.

3. The CCT2 inhibitor for the use of claim 1 or 2, wherein in said treatment a therapeutically effective amount of a cell cycle inhibitor is further to be administered to said subject; preferably wherein the cell cycle inhibitor comprises a CCND1 inhibitor, a CDK2 inhibitor, or a CDK4 inhibitor; optionally wherein the CDK4 inhibitor comprises palbociclib, ribociclib, or abemaciclib.

4. The CCT2 inhibitor for the use of any one of claims 1-3, wherein the cancer is a metastatic cancer.

5. The CCT2 inhibitor for the use of any one of claims 1-4, wherein the cancer is sarcoma, glioma, melanoma, lymphoma, a breast cancer, neuroblastoma, clear cell sarcoma of the kidney (CCSK), Wilms tumor, Rhabdoid tumor of the kidney (RTK), rhabdomyosarcoma, or Choroid plexus carcinoma; preferably wherein the breast cancer is luminal A breast cancer, luminal B breast cancer, estrogen receptor(ER)- progesterone receptor(PR)- HER2+ breast cancer, or triple negative breast cancer.

6. The CCT2 inhibitor for the use of any one of claims 1-5, wherein cancer cells obtained from the subject have an increased level of one or more tumor biomarkers selected from the group consisting of MYC, MYCN, CDK2, CDK4, CCNE1, CCND1, YAP1, and RB1 relative to a reference control.

7. The CCT2 inhibitor for the use of any one of claims 1-6, wherein the drug-resistant cancer is resistant to a cell cycle inhibitor; preferably wherein the cell cycle inhibitor comprises a CCND1 inhibitor, a CDK2 inhibitor, or a CDK4 inhibitor; optionally wherein the CDK4 inhibitor comprises palbociclib, ribociclib, or abemaciclib.

8. A method of diagnosing a subject as having a cancer, comprising:
a) quantifying a level of a chaperonin-containing TCP1 (CCT) relative to a reference control;
b) determining the subject as having the cancer when the level of CCT is higher than the reference control; and
c) determining the subject as not having the cancer when the level of CCT is lower than the reference control.

9. The method of claim 8, wherein the cancer is a metastatic cancer.

10. The method of claim 8 or 9, wherein the cancer is sarcoma, glioma, melanoma, lymphoma, a breast cancer, neuroblastoma, clear cell sarcoma of the kidney (CCSK), Wilms tumor, Rhabdoid tumor of the kidney (RTK), rhabdomyosarcoma, or Choroid plexus carcinoma; preferably wherein the breast cancer is luminal A breast cancer, luminal B breast cancer, estrogen receptor(ER)- progesterone receptor(PR)- HER2+ breast cancer, or triple negative breast cancer.

11. The method of any one of claims 8-10, wherein cancer cells obtained from the subject have an increased level of one or more tumor biomarkers selected from the group consisting of MYC, MYCN, CDK2, CDK4, CCNE1, CCND1, YAP1, and RB1 relative to a reference control.

12. A chaperonin-containing TCP1 subunit 2 (CCT2) inhibitor for use in treating cancer in a subject, wherein said subject has been diagnosed with cancer by a method as claimed in any one of claims 8-11.

13. The CCT2 inhibitor for the use of claim 12, wherein the CCT2 inhibitor comprises a small molecule, an antibody, a peptide, a polypeptide, a small interfering RNA (siRNA), or a short hairpin RNA; preferably a CT20p peptide; most preferably wherein the CT20 peptide comprises an amino acid sequence according to any one of SEQ ID NOs: 1 to 6.

14. The CCT2 inhibitor for the use of claim 12 or 13, wherein in said treatment a therapeutically effective amount of a cell cycle inhibitor is further to be administered to said subject; preferably wherein the cell cycle inhibitor comprises a CCND1 inhibitor, a CDK2 inhibitor, or a CDK4 inhibitor; optionally wherein the CDK4 inhibitor comprises palbociclib, ribociclib, or abemaciclib.

15. A composition comprising a chaperonin-containing TCP1 subunit 2 (CCT2) inhibitor and a cell cycle inhibitor; optionally wherein the CCT2 inhibitor comprises a small molecule, an antibody, a peptide, a polypeptide, a small interfering RNA (siRNA), or a short hairpin RNA; preferably, wherein the peptide is a CT20p peptide; most preferably wherein the CT20 peptide comprises an amino acid sequence according to any one of SEQ ID NOs: 1 to 6; preferably wherein the cell cycle inhibitor comprises a CCND1 inhibitor, a CDK2 inhibitor, or a CDK4 inhibitor; optionally, wherein the CDK4 inhibitor comprises palbociclib, ribociclib, or abemaciclib.
